# EUROPEAN PATENT SPECIFICATION

(11) **EP 1 086 956 B1**
(45) Date of publication and mention of the grant of the patent: **15.04.2009**
(21) Application number: 99921206.1
(22) Date of filing: 20.05.1999
(51) Int. Cl.: C07K 16/28, C12N 15/13, C12N 5/10, C12P 21/08, C07K 16/46, G01N 33/53, G01N 33/577, A61K 39/395

(54) **GENE RECOMBINANT ANTIBODIES**
GEN REKOMBINANTE ANTIKÖRPER
ANTICORPS RECOMBINES DE GENE

(30) Priority: 20.05.1998 JP 13900098
(43) Date of publication of application: 28.03.2001
(73) Proprietor: KYOWA HAKKO KOGYO CO., LTD., Chiyoda-ku, Tokyo 100-8185 (JP)
(72) Inventor: SHITARA, Kenya, Fujisawa-shi, Kanagawa 251-0025 (JP); ITO, Mikito, Machida-shi, Tokyo 194-0023 (JP); KAWADA, Yoko, Abiko-shi, Chiba 270-1165 (JP); NAKAMURA, Kazuyasu, Machida-shi, Tokyo 194-0032 (JP)
(74) Representative: Kinzebach, Werner
(86) International application number: PCT/JP1999/002661
(87) International publication number: WO 1999/060025

(56) References cited:
- WO-A-95/21868
- WO-A-97/10354
- WO-A1-95/21868
- WO-A1-98/22616
- JP-A1- 5 304 989
- JP-A1- 6 205 694
- DAVIS-SMYTH TERRI ET AL: "The second immunoglobulin-like domain of the VEGF tyrosine kinase receptor Flt-1 determines ligand binding and may initiate a signal transduction cascade." EMBO (EUROPEAN MOLECULAR BIOLOGY ORGANIZATION) JOURNAL, vol. 15, no. 18, 1996, pages 4919-4927, XP000611912 ISSN: 0261-4189
- TANAKA K ET AL: "CHARACTERIZATION OF THE EXTRACELLULAR DOMAIN IN VASCULAR ENDOTHELIAL GROWTH FACTOR RECEPTOR-1 (FLT-1 TYROSINE KINASE)" JAPANESE JOURNAL OF CANCER RESEARCH, JAPANESE CANCER ASSOCIATION, TOKYO, JP, vol. 88, no. 9, September 1997 (1997-09), pages 867-876, XP001015447 ISSN: 0910-5050
- WIESMANN C ET AL: "Crystal structure at 1.7 A resolution of VEGF in complex with domain 2 of the Flt-1 receptor" CELL, CELL PRESS, CAMBRIDGE, NA, US, vol. 91, 28 November 1997 (1997-11-28), pages 695-704, XP002126906 ISSN: 0092-8674
- SCIENCE BIRD R.E. ET AL: 'Single-chain antigen-binding proteins' vol. 242, no. 4877, 1988, pages 423 - 426
- MOL. IMMUNOL. WEBBER K.O. ET AL: 'Preparation and characterization of a disulfide-stabilized Fv fragment of the anti-Tac antibody: comparison with its single-chain analog' vol. 32, no. 4, 1995, pages 249 - 258
- MITOLA S. ET AL: 'Tat-human immunodeficiency virus-1 induces human monocyte chemotaxis by activation of vascular endothelial growth factor (Receptor-1)' BLOOD vol. 90, no. 4, 1997, pages 1365 - 1372, XP002937441
- ONCOGENE SHIBUYA M. ET AL: 'Nucleotide sequence and expression of a novel human receptor-type tyrosine kinase gene(flt) closely related to the fms family' vol. 5, no. 4, 1990, pages 519 - 524

## Description

### Technical Field

This invention relates to a gene recombinant antibody which specifically binds to human VEGF receptor Flt-1. The antibody is useful for the diagnosis or treatment of diseases in which their morbid states progress by abnormal angiogenesis, such as proliferation or metastasis of solid tumors, arthritis in rheumatoid arthritis, diabetic retinopathy, retinopathy of prematurity, and psoriasis. The invention also discloses a cell line capable of producing the antibody; a method for immunologically detecting human VEGF receptor Flt-1 using the antibody, and diagnostic and therapeutic uses of the antibody.

### Background Art

Angiogenesis plays an important role in the individual development and construction of tissues in vertebrates, is directly involved in the formation of the corpus luteum during the sexual cycle, transient proliferation of the uterine endometrium and formation of the placenta in mature individuals (females). With regard to pathological states, angiogenesis is involved in the proliferation or metastasis of solid tumors and formation or acceleration of morbidity in diabetic retinopathy and rheumatoid arthritis (J. Biol. Chem., 267: 10931 (1992)]. Angiogenesis occurs by the secretion of an angiogenesis factor and involves the process of a tube formation and producing a new blood vessel. During this process, the basement membrane and interstitum are destroyed by a protease secreted from endothelial cells of an existing blood vessel around the secreted angiogenesis factor, followed by subsequent migration and proliferation of vascular endothelial cells [J. Biol. Chem., 267: 10931 (1992)]. Factors which induce angiogenesis include vascular permeability factor (hereinafter referred to as "VPF") and vascular endothelial growth factor (hereinafter referred to as "VEGF") (hereinafter referred to as "VPF/VEGF"). These factors are considered the most important factors in pathological and non-pathological angiogenesis [Advances in Cancer Research, 67: 281 (1995)]. VPF/VEGF is a protein having a molecular weight of about 40,000 constituted by homodimers, which had been reported to be independent molecules as vascular permeability factor (VPF) in 1983 [Science, 219: 983 (1983))] and as vascular endothelial growth factor (VEGF) in 1989 [Biochem. Biophys. Res. Comm., 161: 851 (1989)], but it has been revealed as the results of cDNA cloning that they are the same substance [Science, 246: 1306 (1989) ; Science, 246: 1309 (1989)] (hereinafter, the term "VPF/VEGF" is referred to as "VEGF") . Beyond the activity of VEGF upon vascular endothelial cells described above, VEGF has also been shown to have a growth enhancing activity [Biochem. Biophys. Res. Comm., 161: 851 (1989)], a migration enhancing activity [J. Immunology, 152: 4149 (1994)], a metalloprotease secretion enhancing activity [J. Cell Physiol., 153: 557 (1992)], and a urokinase and tPA secretion enhancing activity [Biochem. Biophys. Res. Comm., 181: 902 (1991)]. Furthermore, VEGF has been shown to have an angiogenesis enhancing activity [Circulation, 92 suppl II : 365 (1995)], and a vascular permeability enhancing activity [Science, 219: 983 (1983)] as its *in vivo* activities. It has been reported that VEGF is a growth factor having extremely high specificity for vascular endothelial cells [Biochem. Biophys. Res. Comm., 161: 851 (1989)] and that four proteins having different molecular weight are present due to alternative splicing of mRNA [J. Biol. Chem., 267: 26031 (1991)].

Among diseases accompanied by angiogenesis, it has been reported that VEGF plays an important role in the proliferation or metastasis of solid tumors and formation of morbid states of diabetic retinopathy and chronic rheumatoid arthritis. With regard to solid tumors, production of VEGF in a number of human tumor tissues has been reported, such as in renal carcinoma [Cancer Research, 54: 4233 (1994)], breast cancer (Human Pathology, 26: 86 (1995)], brain tumor [J. Clinical Investigation, 91: 153 (1993)], gastrointestinal cancer [Cancer Research, 53: 4727 (1993)], ovarian cancer [Cancer Research, 54: 276 (1994)], and the like. Also, results of a study on the correlation between VEGF expression quantity in tumors and survival ratio of patients in patients with breast cancer have revealed that tumor angiogenesis is more active in tumors expressing high levels of VEGF than low VEGF expression tumors and that the survival ratio is lower in breast cancer patients having high VEGF expression tumors than breast cancer patients having low VEGF expression tumors [Japanese J, Cancer Research, 85: 1045 (1994)]. It has been reported also that an anti-VEGF monoclonal antibody inhibited tumor growth in a xenograft model test system in which a human tumor was transferred into nude mice by subcutaneous transplantation [Nature, 362: 841 (1993)]. Also, it has been reported that, in a metastatic cancer model of a human tumor in nude mice, an anti-VEGF monoclonal antibody inhibited metastasis of the tumor [Cancer Research, 56: 921 (1996)]. Additionally, since a high concentration of VEGF was detected in human carcinomatous pleural perfusions and ascites, the possibility that VEGF is a major factor involved in the retention of pleural perfusions and ascites has been suggested [Biochimica et Biophysica Acta, 1221: 211 (1994)].

In diabetic retinopathy, abnormal angiogenesis causes retinal detachment and hemorrhage of the vitreous body, resulting in blindness, and it has been reported that angiogenesis in diabetic retinopathy and the expression level of VEGF in the patient's eye balls are positively correlative [New England J. Medicine, 331: 1480 (1994)]. Also, it has been reported that angiogenesis in a monkey retinopathy model is inhibited when the VEGF activity is inhibited by the intraocular administration of an anti-VEGF neutralizing monoclonal antibody [Arch. Ophthalmol., 114: 66 (1996)].

Progress in the morbid states of rheumatoid arthritis (destruction of bone and cartilage) is accompanied by angiogenesis, and it has been reported that a high concentration of VEGF is contained in the synovial fluid of patients with rheumatoid arthritis and that macrophages in joints of patients with rheumatoid arthritis produce VEGF rheumatoid [Journal of Immunology, 152: 4149 (1994); J. Experimental Medicine, 180: 341 (1994)].

VEGF receptors have been reported. These include fms-like tyrosine kinase (hereinafter referred to as "Flt-1") [Oncogene, 5: 519 (1990); Science, 255: 989 (1992)] and kinase insert domain-containing receptor (hereinafter referred to as "KDR") [WO 92/14748; Proc. Natl. Acad. Sci. USA, 88: 9026 (1991); Biochem. Biophys. Res. Comm., 187: 1579 (1992); WO 94/11499], which belong to the receptor type tyrosine kinase family. Each of Flt-1 and KDR is a membrane protein of 180 to 200 kilodalton in molecular weight which has an extracellular domain consisting of 7 immunoglobulin-like regions and an intracellular domain consisting of a tyrosine kinase region. It has been reported that VEGF specifically binds to Flt-1 and KDR at Kd values of 20 pM and 75 pM and that Flt-1 and CDR are expressed in vascular endothelial cells in a specific manner [Proc. Natl. Acad. Sci. USA, 90: 7533 (1993) : Proc. Natl. Acad. Sci. USA, 90: 8915 (1993)]. With regard to Flt-1 in various diseases, it has been reported that, in comparison with vascular endothelial cells in normal tissues, expression of Flt-1 mRNA increases in tumor vascular endothelial cells of human glioblastoma tissues [Nature, 359: 845 (1992)] and tumor vascular endothelial cells of human digestive organ cancer tissues [Cancer Research, 53: 4727 (1993)]. Additionally, it has been reported that expression of Flt-1 mRNA is observed by in situ hybridization in vascular endothelial cells of joints of patients with rheumatoid arthritis [J. Experimental Medicine, 180: 341 (1994)]. These results strongly suggest that a VEGF/VEGF receptor Flt-1 system plays an important role in tumor angiogenesis. Although it has been reported that VEGF binds to Flt-1 and the intracellular domain is auto-phosphorylated [Science, 255: 989 (1992)], the detailed function of the receptor mechanism is still unclear. However, it has been discovered that knock out mice in which the Flt-1 gene was destroyed die after a fetal age of 8.5 to 9.5 days due to abnormal blood vessel construction caused by abnormal morphology of vascular endothelial cells during blood island formation in the early stage of development and subsequent angiogenesis. This had led to an assumption that Flt-1 has a function essential for the tube formation of vascular andothelial cells in angiogenesis [Nature, 376: 66 (1995)].

In view of the above, it is expected that an antibody which can inhibit activity which neutralizes VEGF through its binding to VEGF receptor Flt-1 will be useful for the diagnosis or treatment of diseases in which their morbid states progress by abnormal angiogenesis, such as proliferation or metastasis of solid tumors, arthritis in rheumatoid arthritis, diabetic retinopathy, retinopathy of prematurity, psoriasis, and the like.

Generally, when a monoclonal antibody derived from non-human animal is administered to human, the monoclonal antibody is recognized as a foreign material so that an antibody against the monoclonal antibody derived from non-human animal is produced in the human body. As a result, the antibody is allowed to react with the monoclonal antibody derived from non-human animal, and it is known that side effects are caused [J. Clin. Oncol., 2: 881 (1984); Blood, 65: 1349 (1985) ; J. Natl. Cancer Inst., 80: 932 (1988) ; Proc. Natl. Acad. Sci. USA, 82: 1242 (1985)], the monoclonal antibody is shortly cleared [J. Nucl. Med. , 26: 1011 (1985) ; Blood, 65: 1349 (1985) ; J. Natl. Cancer Inst., 80: 937 (1988)], and that the treating effect of the antibody is decreased [J. Immunol., 135: 1530 (1985); Cancer. Res., 46: 6489 (1986)].

In order to solve the above problems, it has been attempted that a monoclonal antibody derived from non-human animal is modified to a humanized antibody, such as a human chimeric antibody or a human CDR (complementarity-determining region; hereinafter often referred to as "CDR") -antibody (reconstructed human antibody), using gene engineering technique. The human chimeric antibody is an antibody in which an antibody variable region (hereinafter referred to as "V region") is derived from a non-human animal antibody and an antibody constant region (hereinafter referred to as "C region") is derived from a human antibody [Proc. Natl. Acad. Sci. USA, 81: 6851 (1984)]. It is reported that when the human chimeric antibody is administered to human, antibodies against the monoclonal antibody derived from non-human animal are not almost induced and the half-life in blood is prolonged six times [Proc. Natl. Acad. Sci. USA, 86: 4220 (1989)]. The human CDR-grafted antibody is an antibody in which the complementarity-determining region (CDR) of a human antibody is replaced with the CDR of an antibody derived from non-human animal [Nature, 321: 522 (1986)]. It is reported that in the experiment using a monkey, immunogenicity is lowered by the human CDR-grafted antibody as compared with a mouse antibody, and the half-life in blood is prolonged four to five times [J. Immunol., 147: 1352 (1991)].

Accordingly, when the chimeric antibody and the humanized antibody which specifically react with human VEGF receptor Flt-1, side effects are decreased and the half-life in blood is prolonged because no antibody against a monoclonal antibody derived from non-human animal is produced. Therefore, it is expected that these antibodies can treat diseases in which their morbid states progress by abnormal angiogenesis, such as proliferation or metastasis of solid tumors, arthritis in rheumatoid arthritis, diabetic retinopathy, retinopathy of prematurity, psoriasis, and the like.

In addition, with the recent progress of protein engineering and gene engineering, the production of smaller antibody molecules, such as a single chain antibody [Science, 242: 423 (1988)] and a disulfide stabilized antibody [Molecular Immunology, 32: 249 (1995)], have been tried. Since the single chain antibody and the disulfide stabilized antibody have a molecular weight lower than a monoclonal antibody or a humanized antibody, they are excellent in tissue transition property and clearance from blood and are applied to imaging and the like, a composite thereof with a toxin was produced, and therefore, the treatment effect can be expected [Cancer Research, 55: 318 (1995)]. Therefore, it is expected that these antibodies can treat diseases in which their morbid states progress by abnormal angiogenesis, such as proliferation or metastasis of solid tumors, arthritis in rheumatoid arthritis, diabetic retinopathy, retinopathy of prematurity, psoriasis, and the like.

WO-A 95/21868 describes a monoclonal antibody produced by hybridoma cell line DC101 (IgG1k) deposited as ATCC accession no. ATCC HB 11534. Said antibody is said to specifically react with murine flk-1. Flk-1 is a further VEGF receptor that is distinct from Flt-1.

Davis-Smyth and co-workers report that the second immunoglobulin-like domain of Flt-1 determines ligand binding (the EMBO Journal 15, 4919-4927 (1996)). Tanaka and co-workers describe that a region carrying the N-terminal first to third immunoglobulin-like domains of Flt-1 binds VEGF and placenta growth factor with high affinity (Jpn. J. Cancer Res. 88, 1997, 867-876). Wiesmann and co-workers describe that the second and third domains of Flt-1 are necessary and sufficient for binding VEGF (Cell, 91, 1997, 695-704).

### Disclosure of the Invention

Concert has been directed toward the development of a method which is useful for the diagnosis or treatment of diseases in which their morbid states progress by abnormal angiogenesis, such as proliferation or metastasis of solid tumors, arthritis in rheumatoid arthritis, diabetic retinopathy, retinopathy of prematurity, and psoriasis.

It is considered that detection of the regions of angiogenesis and inhibition of angiogenesis by the use of an anti-human VEGF receptor Flt-1 monoclonal antibody will be useful for the diagnosis and treatment of the above diseases in which their morbid states progress by abnormal angiogenesis.

The present invention relates to a gene recombinant antibody as defined in any one of claims 1 to 12, 17 to 22 and 27 to 42.

The gene recombinant antibody of the present invention is obtained by modifying the above monoclonal antibody of the present invention using the genetic recombination technique. The gene recombinant antibody includes antibodies produced by gene recombination, for example, a humanized antibody, an antibody fragment, such as a single chain antibody, and a disulfide stabilized antibody. The gene recombinant antibody having a property of a monoclonal antibody, low antigenicity and prolonged half-life in blood is preferred as a therapeutic agent.

The gene recombinant antibody according to the present invention recognizes an epitope present in a region of from the N-terminal to the 750^{th} amino acid including a signal sequence of human VEGF receptor Flt-1, more specifically an epitope present in a region of from the N-terminal to the 338^{th} amino acid including a signal sequence of human VEGF receptor Flt-1, and even more specifically an epitope present in a region of the 100^{th} to 204^{th} amino acids from the N-terminal including a signal sequence of human VEGF receptor Flt-1.

The gene recombinant antibody according to the present invention inhibits binding of human VEGF receptor Flt-1 and may have an activity which neutralizes human Flt-1.

The antibody may be selected from a humanized antibody and an antibody fragment.

The humanized antibody of the present invention includes a human chimeric antibody and a human CDR-grafted antibody.

The antibody fragment of the present invention includes a fragment of antigen binding (hereinafter referred to as "Fab"), Fab', F(ab')₂, a single chain antibody (single chain Fv; hereinafter referred to as "scFv"), and a disulfide stabilized antibody (disulfide stabilized Fv; hereinafter referred to as "dsFv"), which specifically react with human VEGF receptor Flt-1. Hereinafter, the antibody or antibody fragment which specifically reacts with human VEGF receptor Flt-1 is referred to as "anti-human VEGF receptor Flt-1 antibody".

According to a particular embodiment, the humanized antibody belongs to a human IgG antibody.

According to a further particular embodiment, the humanized antibody is a human chimeric antibody or a human CDR-grafted antibody.

The human chimeric antibody means an antibody which comprises an antibody heavy chain variable region (hereinafter referred to as "HV" or "VH", in which the heavy chain and the variable region are referred to as "H chain" and "V region", respectively) and V region L chain (hereinafter referred to as "LV" or "VL", in which the light chain is referred to as "L chain") of an antibody of a non-human animal, and a heavy chain constant region (hereinafter also referred to as "CH", in which the constant region is referred to as "C chain") of a human antibody and a light chain constant region (hereinafter also referred to as "CL") of a human antibody.

The human chimeric antibody of the present invention can be produced by isolating cDNAs encoding VH and VL from a hybridoma capable of producing a monoclonal antibody which specifically reacts with human VEGF receptor Flt-1, inserting them into an expression vector for animal cells having genes encoding human antibody CH and human antibody CL to construct a human chimeric antibody expression vector, and then introducing the vector into an animal cell to express the antibody of interest.

The structure of the human chimeric antibody of the present invention may belong to any immunoglobulin (hereinafter referred to as "Ig") class, but preferably contains the C region of IgG type immunoglobulin, particularly of IgG subclasses, such as IgG1, IgG2, IgG3, IgG4, and the like.

The human CDR-grafted antibody of the present invention means an antibody in which the CDRs of VH and VL of a human antibody are replaced with respective CDRs of an antibody of a non-human animal.

The human CDR-grafted antibody of the present invention can be produced by constructing V region-encoding cDNAs in which CDRs of VH and VL of a human antibody are replaced with CDRs of VH and VL of an antibody of a non-human animal, which specifically reacts with human VEGF receptor Flt-1, inserting them into an expression vector for animal cells having genes encoding human antibody CH and human antibody CL to construct a human CDR-grafted antibody expression vector, and then introducing the vector into an animal cell to express the antibody of interest.

The structure of the C region of the human CDR-grafted antibody of the present invention may belong to any immunoglobulin (Ig) class, but preferably contains the C region of IgG type immunoglobulin, particularly of IgG subclasses such as IgG1, IgG2, IgG3, IgG4, and the like.

A human chimeric antibody according to the invention may comprise VH and VL of a monoclonal antibody which specifically reacts with human VEGF receptor Flt-1, and CH and CL of a human antibody, e.g. VH and VL, respectively of a monoclonal antibody selected from monoclonal antibody KM1732 (FERM BP-5698) and monoclonal antibody KM1750 (FERM BP-5700).

According to a particular embodiment, the amino acid sequence of the VH is an amino acid sequence of SEQ ID NO:86 or 88.

According to a further particular embodiment, the amino acid sequence of the VL is an amino acid sequence of SEQ ID NO:87 or 89.

More specifically, the amino acid sequence of the VH comprises an amino acid sequence of SEQ ID NO:86, and the amino acid sequence of the VL comprises an amino acid sequence of SEQ ID NO:87, or the amino acid sequence of the VH comprises an amino acid sequence of SEQ ID NO:88, and the amino acid sequence of the VL comprises an amino acid sequence of SEQ ID NO:89.

Even more specifically, the human chimeric antibody which specifically reacts with human VEGF receptor Flt-1, is selected from KM2532 and KM2550.

The present invention also relates to the following:
A DNA which encodes the human chimeric antibody as defined herein.
A recombinant vector comprising the DNA and tandem cassette vector pKANTEX93.
A transformant obtained by introducing the recombinant vector into a host cell.

A method for producing an antibody, comprising culturing the transformant in a medium to produce and accumulate the human chimeric antibody in a culture; and recovering the antibody from the resulting culture.

The human CDR-grafted antibody according to the invention is an antibody comprising CDRs of VH and VL of a monoclonal antibody which specifically reacts with human VEGF receptor Flt-1, and C region and V region framework regions of an H chain and an L chain of a human antibody.

According to a particular embodiment, the human CDR-grafted antibody comprises amino acid sequences of SEQ ID NOs:5, 6 and 7 as the CDR of the VH, and amino acid sequences of SEQ ID NOs:8, 9 and 10 as the CDR of the VL, or
amino acid sequences of SEQ ID NOs: 11, 12 and 13 as the CDR of the VH, and amino acid sequences of SEQ ID NOs:14, 15 and 16 as the CDR of the VL.

More specifically, the invention includes a human CDR-grafted antibody wherein the amino acid sequence of the VH comprises an amino acid sequence of SEQ ID NO:90, and the amino acid sequence of the VL comprises an amino acid sequence of SEQ ID NO:92; or
wherein the amino acid sequence of the VH comprises an amino acid sequence of SEQ ID NO:91 or 95, and the amino acid sequence of the VL comprises amino acid sequences of SEQ ID NOs:93, 94 or 96.

Even more specifically, the human CDR-grafted antibody is selected from KM8550, KM8551, KM8552, KM8553, KM8554, and KM8555.

The present invention also relates to the following:
A DNA which encodes the human CDR-grafted antibody as defined herein.
A recombinant vector comprising the DNA and tandem cassette vector pKANTEX93.
A transformant obtained by introducing the recombinant vector into a host cell.
A method for producing an antibody, comprising culturing the transformant in a medium to produce and accumulate the human CDR-grafted antibody in a culture; and recovering the antibody from the resulting culture.

The antibody according to the invention may be an antibody fragment selected from the group consisting of Fab, Fab', F(ab')₂, a single chain antibody, and a disulfide stabilized Fv.

The Fab is a fragment having a molecular weight of about 50,000 and antigen-binding activity which comprises about half of the N-terminal side of H chain and a full portion of L chain obtained by digesting, with an enzyme, papain, the peptide moiety of the upper side of two disulfide bonds that cross-link two H chains at the hinge region of IgG.

The Fab of the present invention can be obtained by treating an anti-human VEGF receptor Flt-1 antibody with papain. Alternatively, the Fab can be produced by inserting a DNA fragment which encodes the Fab fragment of the antibody into an expression vector for animal cells, and introducing the vector into an animal cell to express the antibody of interest.

The Fab' is a fragment of about 50,000 in molecular weight having antigen-binding activity, and it is obtained by hydrolyzing the disulfide bond between hinges of the above-described F(ab')₂.

The Fab' of the present invention can be obtained by treating an anti-human VEGF receptor Flt-1 antibody with a reducing agent, dithiothreitol. Alternatively, the Fab' can be produced by inserting a DNA fragment which encodes the Fab' fragment of the antibody into an expression vector for animal cells, and introducing the vector into an animal cell to express the antibody of interest.

The F(ab')₂ is a fragment having a molecular weight of about 100,000 and antigen-binding activity, comprising two Fab regions bonded at the hinge region, which is obtained by digesting, with an enzyme, trypsin, the lower side of two disulfide bonds at the hinge region of IgG.

The F(ab')₂ of the present invention can be obtained by digesting an anti-human VEGF receptor Flt-1 antibody with trypsin. Alternatively, F(ab')₂, can be produced by inserting a DNA fragment which encodes the F(ab')₂ % fragment of the antibody into an expression vector for animal cells, and introducing the vector into an animal cell to express the antibody of interest.

The single chain antibody (hereinafter also referred to as "scFv") means a VH-P-VL or VL-P-VH polypeptide obtained by linking a VH chain with a VL chain using an appropriate peptide linker (hereinafter referred to as "P"). The VH and VL of the scFv of the present invention can be any of the monoclonal antibody and human CDR-grafted antibody of the present invention.

The single chain antibody of the present invention can be produced by isolating cDNAs encoding VH and VL from a hybridoma capable of producing an anti-human VEGF receptor Flt-1 antibody to construct a single chain antibody expression vector, inserting the cDNAs into the scFv expression vector, and introducing the expression vector into *Escherichia coli,* yeast or an animal cell to express the antibody of interest.

The disulfide stabilized antibody (hereinafter also referred to as "dsFv") means an antibody obtained by bonding, via a disulfide bond, polypeptides in which one amino acid residue in each of VH and VL is replaced with a cysteine residue. The amino acid residue to be replaced with a cysteine residue can be selected based on the three-dimensional structure estimation of antibodies in accordance with the method reported by Reiter et al. [Protein Engineering, 7: 697 (1994)]. The VH or VL contained in the disulfide stabilized antibody of the present invention can be any of the monoclonal antibody and human CDR-grafted antibody of the present invention.

The disulfide stabilized antibody of the present invention can be produced by isolating cDNAs encoding VH and VL from a hybridoma capable of producing an anti-human VEGF receptor Flt-1 antibody, inserting the cDNAs into an appropriate expression vector, introducing the expression vector into *Escherichia coli,* yeast or an animal cell to express the antibody of interest.

The single chain antibody according to the invention may comprise VH and VL of an antibody.

The amino acid sequences of the VH and VL of the single chain antibody may comprise amino acid sequences which are the same as amino acid sequences of VH and VL, respectively, of a monoclonal antibody which specifically reacts with human VEGF receptor Flt-1.

The amino acid sequences of the CDRs of the VH and VL of the single chain antibody comprise amino acid sequences which are the same as amino acid sequences of CDRs of VH and VL, respectively, of a monoclonal antibody which specifically reacts with human VEGF receptor Flt-1.

According to a particular embodiment, the amino acid sequences of the VH and VL of the single chain antibody are SEQ ID NOs:86 and 87, respectively, or the amino acid sequences of the VH and VL of the single chain antibody are SEQ ID NOs:88 and 89, respectively.

The single chain antibody according to the invention is in particular one wherein the amino acid sequences of the CDRs of the VH and VL of the single chain antibody comprise amino acid sequences of SEQ ID NOs:5, 6 and 7 as the CDR of the VH, and amino acid sequences of SEQ ID NOs: 8, 9 and 10 as the CDR of the VL, or
wherein the amino acid sequences of the CDRs of the VH and VL of the single chain antibody comprise amino acid sequences of SEQ ID NOS:11, 12 and 13 as the CDR of the VH, and amino acid sequences of SEQ ID NOS: 14, 15 and 16 as the CDR as the VL.

According to a particular embodiment, the amino acid sequence of the VH of the single chain antibody comprises amino acid sequences of SEQ ID NO:90, and the amino acid sequence of the VL thereof comprises an amino acid sequence of SEQ ID NO:92, or
the amino acid sequence of the VH of the single chain antibody comprises amino acid sequences of SEQ ID NO:91 or 95, and the amino acid sequence of the VL thereof comprises an amino acid sequence of SEQ ID NO: 93, 94 or 96.

The disulfide stabilized Fv according to the invention may comprise VH and VL of an antibody.

The amino acid sequences of the VH and VL of the disulfide stabilized antibody may comprise amino acid sequences which are the same as amino acid sequences of VH and VL, respectively, of a monoclonal antibody which specifically reacts with human VEGF receptor Flt-1.

The amino acid sequences of the CDRs of the VH and VL of the disulfide stabilized antibody comprise amino acid sequences which are the same as amino acid sequences of complementarity-determining regions of VH and VL, respectively, of a monoclonal antibody which specifically reacts with human VEGF receptor Flt-1.

According to a particular embodiment, the amino acid sequences of he VH and VL of the disulfide stabilized antibody are SEQ ID NOs:86 amd 87, respectively, or
the amino acid sequences of the VH and VL of the disulfide stabilized antibody are SEQ ID NOs:88 and 89, respectively.

The disulfide stabilized antibody according to the invention is in particular one wherein the amino acid sequences of the CDRs of the VH and VL of the disulfide stabilized antibody comprise amino acid sequences of SEQ ID NOS:5, 6 and 7 as the CDR of the VH, and amino acid sequences of SEQ ID NOS:8, 9 and 10 as the CDR of the VL, or
wherein the amino acid sequences of the CDRs of the VH and VL of the disulfide stabilized antibody comprise amino acid sequences of SEQ ID NOS : 11, 12 and 13 as the CDR of the VH, and amino acid sequences of SEQ ID NOS:14, 15 and 16 as the CDR of the VL.

According to a particular embodiment, the amino acid sequence of the VH of the disulfide stabilized antibody comprises an amino acid sequence of SEQ ID NO:90, and the amino acid sequence of the VL thereof comprises an amino acid sequence of SEQ ID NO:92, or
the amino acid sequence of the VH of the disulfide stabilized antibody comprises an amino acid sequence of SEQ ID NO: 91 or 95, and the amino acid sequence of the VL thereof comprises amino acid sequences of SEQ ID NOs:93, 94 or 96.

The invention also relates to an antibody which is a fusion antibody wherein the antibody as defined herein is chemically or gene-engineeringly bound with a radioisotope, protein or low molecular agent.

The fusion antibody of the present invention can be produced by chemically linking a radioactive isotope, protein or low molecular agent with the antibody which specifically reacts with human VEGF receptor Flt-1 of the present invention.

In case of the fusion antibody with a protein, it can be produced by connecting an antibody-encoding cDNA to a protein-encoding cDNA, inserting the thus ligated cDNA into an appropriate expression vector, and expressing the expression vector in *Escherichia coli,* yeast or an animal cell.

Examples of the low molecular agent include antitumor agents, such as alkylating agents (e.g., nitrogen mustards, cyclophosphamide), antimetabolites (e.g., 5-fluorouracil, methotrexate), antibiotics (e.g., mitomycin C, daunorubicin), plant alkaloids (e.g., vincristine, vinblastine), and hormone agents (e.g., tamoxifen, dexamethasone) [Clinic Oncology, edited by Japan Clinic Oncology Society, published by Cancer and Chemotherapy Corporation (1996)]; antiinflammatory agents, such as steroid drugs (e.g., hydrocortisone, prednisone), non-steroid drugs (e.g., aspirin, indomethacin), immunomodulators (e.g., cyclophosphamide, azathioprine), and antihistamic agents (e.g., chlorpheniramine maleate, clemastine) [Inflammation and Anti-Inflammatory Therapy, published by Ishiyaku Shuppan (1982).

The invention further relates to an in vitro method for immunologically detecting human VEGF receptor Flt-1, using the antibody as defined herein;
an in vitro method for immunologically determining human VEGF receptor Flt-1, using the antibody as defined herein;
an in vitro method for immunologically detecting soluble human VEGF receptor Flt-1, using the antibody as defined herein;
an in vitro method for immunologically determining soluble human VEGF receptor Flt-1, using the antibody as defined herein;
an in vitro method for immunologically detecting cells in which human VEGF receptor Flt-1 ia expressed on the surface thereof, using the antibody as defined herein;
an in vitro method for immunologically determining cells in which human VEGF receptor Flt-1 is expressed on the surface thereof, using the antibody as defined herein;
an in vitro method for inhibiting binding of human VEGF receptor Flt-1, using the antibody as defined herein;
an in vitro method for neutralizing the function of human VEGF receptor Flt-1, using the antibody as defined herein;
an in vitro method for inhibiting migration of vascular endothelial cells, using the antibody as defined herein; and
an in vitro method for detecting a disease in which the morbid states progress by abnormal angiogenesis as defined herein, using the antibody as defined herein.

The invention also relates to the use of an antibody as defined herein for the manufacture of an agent for diagnosing a disease in which the morbid states progress by abnormal angiogenesis as defined herein.

The diseases in which their morbid states progress by abnormal angiogenesis are proliferation or metastasis of solid tumor, arthritis in chronic rheumatoid arthritis, diabetic retinopathy, retinopathy of prematurity, psoriasis. Examples of the solid tumor include breast cancer, prostatic cancer, large bowel cancer, gastric cancer, and lung cancer.

The invention further relates to the use of an antibody as defined herein for the manufacture of an agent for treating a disease in which the morbid states progress by abnormal angiogenesis as defined herein.

The production method of the anti-human VEGF receptor Flt-1 antibody and anti-human VEGF receptor Flt-1 antibody fragment which specifically react with human VEGF receptor Flt-1 or have an activity which neutralizes human Flt-1, and the method detecting and determining human VEGF receptor Flt-1 using the antibody are described below.

### 1. Production method of anti-human VEGF receptor Flt-1 monoclonal antibody

### (1) Preparation of antigen

Examples of the substance useful as the antigen for the production of the anti-human VEGF receptor Flt-1 monoclonal antibody include cells in which human VEGF receptor Flt-1 is expressed on the cell surface or a cell membrane fraction thereof, soluble human VEGF receptor Flt-1 protein having an extracellular region of different length, a fusion protein of the protein with Fc region of the antibody.

Examples of the cells capable of expressing human VEGF receptor Flt-1 on the cell surface include NIH3T3-Flt-1 cells [Oncogene, 10: 135 (1995)].

Also, according to the gene engineering technique, a DNA encoding human VEGF-receptor Flt-1 is obtained, and an antigen is obtained by expressing as soluble human VEGF receptor Flt-1 protein having an extracellular region of different length or a fusion protein of the protein with Fc region of the antibody.

The full length or a partial fragment of cDNA which encodes human VEGF receptor Flt-1 [Oncogene, 5: 519 (1990); Abstract of Papers, the 18th Annual meeting of Japan Molecular Biology Society, 2P-227 (December, 1995)] is inserted into a downstream site of the promoter of an appropriate vector, the thus constructed recombinant vector is inserted into host cells and the thus obtained human VEGF receptor Flt-1 expression cells are cultured in an appropriate medium, thereby producing the full length or a partial fragment of human VEGF receptor Flt-1 in the cells or culture supernatant as such or as a fusion protein.

The hosts can be any one of bacteria, yeast, animal cells, insect cells and the like so long as they can express the gene of interest. Examples of the bacteria include the genus *Escherichia*, and the genus *Bacillus*, such as *Escherichia coli,* and *Bacilllus subtilis*. Examples of the yeast include *Saccharomyces cerevisiae*, and *Schizosaccharomyces pompe*. Examples of the animal cells include namalwa cells which are human cells, COS cells which are monkey cells, CHO cells which are Chinese hamster cells. Examples of the insect cells include Sf9 and Sf21 (manufactured by Pharmingen), and High Five (manufactured by In Vitrogen).

The vector to which the DNA of the present invention is inserted can be any vector so long as the DNA can be inserted and expressed in a host cell.

When a bacterium such as *Escherichia coli* is used as the host, the expression vector may be preferably constructed with a promoter, a ribosome binding sequence, the DNA of the present invention, a transcription termination sequence and, if necessary, a promoter controlling sequence. Examples include commercially available pGEX (manufactured by Pharmacia), and pET System (manufactured by Novagen).

With regard to the method for introducing the recombinant vector into a bacterium, any one of the known methods for introducing DNA into bacteria, such as a method in which calcium ion is used [Proc. Natl. Acad. Sci. USA, 69: 2110 (1972)],and a protoplast method (Japanese Published Unexamined Patent Application No. 248394/91) can be used.

When yeast is used as the host, YEp13 (ATCC 37115), YEp24 (ATCC 37051), or YCp50 (ATCC 37419) is used as the expression vector.

With regard to the method for introducing the recombinant vector into yeast, any one of the known methods for introducing DNA into yeast, such as an electroporation method [Methods. Enzymol., 194: 182 (1990)], a spheroplast method [Proc. Natl. Acad. Sci. USA, 84: 1929 (1978)] , and a lithium acetate method [J. Bacteriol., 153: 163 (1983)] can be used.

When animal cells are used as the host, pAGE107 [Japanese Published Unexamined Patent Application No. 22979/88; Cytotechnology, 33: 133 (1990)], and pAGE103 (J. Biochem. , 101: 1307 (1987)] can be exemplified as the useful expression vector.

Any promoter can be used as long as it can be expressed in animal cells. Examples include the promoter of IE (immediate early) gene of cytomegalovirus (CMV), the SV40 promoter, the metallothionein promoter and the like. Furthermore, the enhancer of the IE gene of human CMV may be used together with the promoter.

With regard to the method for the introduction of the recombinant vector into animal cells, any one of the known methods for introducing DNA into animal cells, such as an electroporation method [Cytotechnology, 3: 133 (1990)], a calcium phosphate method (Japanese Published Unexamined Patent Application No. 227075/90), and a lipofection method [Proc. Natl. Acad. Sci. USA, 84: 7413 (1987)] can be used.

When insect cells are used as the host, the protein can be expressed by the known method described in, for example, *Current Protocols*, Supplement 1-34 and *Baculovirus Expression Vectors, A Laboratory Manual.* That is, the recombinant gene introducing vector and baculovirus described in the following are simultaneously introduced into insect cells to obtain a recombinant virus in the insect cell culture supernatant and then the insect cells are infected with the thus obtained recombinant virus to obtain protein-expressing insect cells.

Examples of the gene introducing vector include pVL1392, pVL1393, and pBlueBacIII (all manufactured by In Vitrogen).

Examples of the baculovirus include Autograph californica nuclear polyhedrosis virus with which insects of the family Barathra are infected.

With regard to the method for the simultaneous introduction of the above-described recombinant gene introducing vector and the above-described baculovirus into insect cells for the production of the recombinant virus, calcium phosphate method (Japanese Published Unexamined Patent Application No. 227075/90), and lipofection method *[*Proc. Natl. Acad. Sci. USA, 84: 7413 (1987)] can be exemplified.

Alternatively, the protein of interest can be produced by producing a recombinant baculovirus with, for example, BaculoGold Starter Kit manufactured by Pharmigen and then infecting the above-described insect cells, such as Sf9, Sf21, or High Five, with the recombinant virus *[*Bio/Technology, 6: 47 (1988)].

With regard to the gene expression method, techniques, such as secretion production, fusion protein expression have been developed, and each of these methods can be used. For example, it can be carried out in accordance with the method described in Molecular Cloning, 2nd edition, Cold Spring Harbor Lab. Press, New York (1989) (hereinafter referred to as "*Molecular Cloning,* 2nd edition").

Examples of the protein to be fused include β-galactosidase, protein A, the IgG binding region of protein A, chloramphenicol-acetyltransferase, poly(Arg), poly(Glu), protein G, maltose-binding protein, glutathione-S-transferase, polyhishistidine chain (His-tag), S peptide, DNA-binding protein domain, Tac antigen, thioredoxin, green-fluorescent-protein, and epitope of any antibody *[*Jikken Igaku, 13: 469-474 (1995)].

The full length or a partial fragment of a human VEGF receptor Flt-1 can be produced as such or as a fusion protein thereof by culturing a transformant obtained in the above-described manner in a culture medium to form and accumulate the protein of the present invention in the resulting culture mixture, and then recovering the protein from the culture mixture.

Culturing of the transformant of the present invention in a culture medium is carried out in accordance with a usual method which is used in the culturing of respective hosts.

With regard to the medium for use in the culturing of the transformant obtained using a microorganism, such as *Escherichia coli,* or yeast, as the host, either a natural medium or a synthetic medium may be used, so long as it contains materials which can be assimilated by the microorganism, such as carbon sources, nitrogen sources, and inorganic salts, and can perform culturing of the transformant efficiently (*Molecular Cloning,* 2nd edition) . The culturing is carried out generally under aerobic conditions, such as a shaking culture,or submerged agitation aeration culture, at 15 to 40°C for 16 to 96 hours. During the culturing, the pH is controlled to 3.0 to 9.0. Adjustment of the pH is carried out using an inorganic or organic acid, an alkali solution, urea, calcium carbonate, and ammonia. During the culturing, if necessary, antibiotics, such as ampicillin, and tetracycline may be added to the medium.

With regard to the medium for use in the culturing of a transformant obtained using animal cells as the host, RPMI 1640 medium, Eagle's MEM medium or any one of these media further supplemented with fetal calf serum may be used. The culturing is carried out generally at 35 to 37°C for 3 to 7 days in the presence of 5% CO₂. During the culturing, if necessary, antibiotics, such as kanamycin, and penicillin may be added to the medium.

With regard to the medium for use in the culturing of a transformant obtained using insect cells as the host, TNM-FH medium (manufactured by Pharmingen), Sf900IISFM (manufactured by Life Technologies), ExCell400 or ExCell405 (both manufactured by JRH Biosciences) may be used. The culturing is carried out generally at 25 to 30°C for 1 to 4 days, and, if necessary, gentamicin and the like antibiotics may be added to the medium during the culturing.

Although media for the culturing of animal cells and insect cells contain serum, it is desirable to use a serum-free medium in order to efficiently purify the full length or a partial fragment of human VEGF receptor Flt-1 as such or as a fusion protein.

When the full length or a partial fragment of human VEGF receptor Flt-1 is accumulated inside the host cells as such or as a fusion protein, the cells after completion of the culturing are collected by centrifugation, suspended in an aqueous buffer and then disrupted using ultrasonic oscillator, or French press, and subsequently recovering the protein from a supernatant fluid produced by centrifuging the thus disrupted cells.

Also, when an insoluble body is formed inside the cells, the insoluble body is solubilized using a protein denaturing agent and then steric three dimentional structure of the protein is formed by diluting or dialyzing the thus solubilized protein in or against a solution which does not contain the protein denaturing agent or contains the agent but in such a low concentration that the protein is not denatured.

When the full length or a partial fragment of human VEGF receptor Flt-1 is secreted outside the cells as such or as a fusion protein, the expressed protein can be collected from the culture supernatant. The isolation and purification can be carried out by employing separation means, such as solvent extraction, fractional precipitation with organic solvents, salting out, dialysis, centrifugation, ultracentrifugation, ion exchange chromatography, gel filtration chromatography, hydrophobic chromatography, affinity chromatography, reverse phase chromatography, crystallization, and electrophoresis, alone or in combination.

### (2) Immunization of animals and preparation of antibody-producing cells

Although any one of animals, such as mice, rats, hamsters, and rabbits, can be used in the immunization, so long as a hybridoma can be produced, an example in which mice and rats are used is described in this invention.

A mouse or rat of 3 to 20 weeks of age is immunized with the protein obtained in the above 1 (1) as the antigen, and antibody-producing cells are collected from the spleen, lymph node or peripheral blood of the animal. The immunization is carried out by administering the antigen several times through subcutaneous, intravenous or intraperitoneal injection together with an appropriate adjuvant. As the adjuvant, a complete Freund's adjuvant or a combination of aluminum hydroxide gel with pertussis vaccine can be exemplified. A blood sample is collected from the fundus of the eye or caudal vein of the animal 3 to 7 days after each administration, the sample is tested, for example, by enzyme immunoassay [*Enzyme-linked Immunosorbent Assay (ELISA),* published by Igaku Shoin (1976)] as to whether it is reactive with the antigen used, namely soluble human VEGF receptor Flt-1 or NIH3T3 cells in which human VEGF receptor Flt-1 is expressed on the cell surface, and then a mouse or rat showing sufficient antibody titer in their sera is submitted for use as the supply source of antibody-producing cells. On the 3rd to 7th day after final administration of the antigen, the spleen is excised from the immunized mouse or rat to carry out fusion of the spleen cells with myeloma cells in accordance with the known method [Antibodies - A Laboratory Manual, Cold Spring Harbor Laboratory (1988); referred to as "*Antibodies* - *A Laboratory Manual*" hereinafter].

### (3) Preparation of myeloma cells

As the myeloma cells, any myeloma cells capable of growing *in vitro* may be used, which include established cells obtained from mouse, such as 8-azaguanine-resistant mouse (BALB/c) myeloma cell line P3-X63Ag8-U1 (P3-U1) [G. Kohler et al., Europ. J. Immunol, 6: 511 (1976)], SP2/O-Ag14 (SP-2) [M. Shulman et al., Nature, 276: 269 (1978)], P3-X63-Ag8653 (653) [J.F. Kearney et al., J. Immunol., 123: 1548 (1979)], and P3-X63-Ag8 (X63) [G. Kohler et al., Nature, 256: 495 (1975)].

These cell lines are cultured and subcultured in accordance with the known method (*Antibodies - A Laboratory Manual*) and 2×10⁷ or more of the cells are secured until cell fusion.

### (4) Cell fusion

The antibody-producing cells and the myeloma cells obtained in the above are washed, respectively, and mixed with cell-aggregating medium, or polyethylene glycol-100 (PEG-1000), to carry out cell fusion and then suspended in a culture medium. For washing of the cells, MEM medium or PBS (1.83 g of disodium hydrogen phosphate, 0.21 g of potassium dihydrogen phosphate, 7.65 g of sodium chloride, 1 liter of distilled water, pH 7.2) is used. In order to obtain the fused cells of interest selectively, HAT medium (normal medium [a medium prepared by adding glutamine (1.5 mM), 2-mercaptoethanol (5×10⁻⁵ M), gentamicin (10 µg/ml) and fetal calf serum (FCS) (10%, manufactured by CSL) to RPMI-1640 medium] further supplemented with hypoxanthine (10⁻⁴ M), thymidine (1.5×10⁻⁵ M) and aminopterin (4×10⁻⁷ M)} is used as the medium for suspending the fused cells.

After the culturing, a portion of the culture supernatant is sampled and a sample which reacts with an antigen protein but does not react a non-antigen protein is selected by an enzyme immunoassay method. Thereafter, cloning is carried out by limiting dilution analysis, and a hybridoma which shows stably high antibody titer is selected as the hybridoma capable of producing the monoclonal antibody.

### (5) Selection of mouse or rat anti-human VEGF receptor Flt-1 monoclonal antibody

Selection of a hybridoma capable of producing the mouse or rat anti-human VEGF receptor Flt-1 monoclonal antibody is carried out according to the method described in *Antibodies, A Laboratory Manual* by the enzyme immunoassay method described below. According to these methods, the binding activities of the anti-human VEGF receptor Flt-1 humanized antibody described below, an anti-human Flt-1 humanized antibody contained in a culture supernatant of a transformant producing the antibody fragment or all purified anti-human Flt-1 humanized antibodies can be measured.

### Enzyme immunoassay

An antigen protein, or cells which express an antigen protein is coated on a 96-well plate, and the reaction is carried out using a hybridoma culture supernatant or a purified antibody obtained in the above method as a first antibody.

After the reaction of the first antibody, the plate is washed and a second antibody is added.

The second antibody is obtained by labeling an antibody which can recognize immunoglobulin of the first antibody or anti-rat immunoglobulin antibody with biotin, an enzyme, a chemiluminescent substance, or a radioactive compound.

Specifically, if a mouse is used for the production of the hybridoma, an antibody which can recognize mouse immunoglobulin is used as a second antibody.

After the reaction, a reaction suitable for the substance used for labeling the second antibody is carried out in order to select a hybridoma producing monoclonal antibody which specifically reacts with the antigen.

Examples of the hybridoma include hybridomas KM1732 (FERM BP-5698), KM1748 (FERM BP-5699) and K1750 (FERM BP-5700).

Also, the above first antibody is mixed with human VEGF labeled with an enzyme, a chemiluminescent substance, or a radioactive compound, the reaction is carried out, and then the activity of inhibiting binding of human VEGF to human VEGF receptor Flt-1 can be measured by carrying out a reaction suitable for the labeled substance. Using such a method, screening of a hybridoma having a high human VEGF inhibition activity can be carried out.

### (6) Preparation of monoclonal antibody

The anti-human VEGF receptor Flt-1 monoclonal antibody-producing hybridoma cells obtained in 1(4) are administered by intraperitoneal injection into 8- to 10-week-old mice or nude mice treated with pristane [by intraperitoneal administration of 0.5 ml of 2,6,10,14-tetramethylpentadecane (pristane) followed by 2 weeks of feeding] at a dose of 2×10⁷ to 5×10⁶ cells/animal. The hybridoma causes ascites tumor in 10 to 21 days. The ascitic fluid is collected from the mice or nude mice, centrifuged, subjected to salting out with 40 to 50% saturated ammonium sulfate or to caprylic acid precipitation and then passed through a DEAE-Sepharose column, protein A column or Cellulofine GSL 2000. (manufactured by Seikagaku Kogyo) to collect an IgG or IgM fraction to give a purified monoclonal antibody.

The subclass of the purified monoclonal antibody can be determined using a mouse monoclonal antibody typing kit or a rat monoclonal antibody typing kit. The amount of protein can be determined by the Lowry method or by calculation based on the optical density at 280 nm.

The subclass of antibody means isotypes within the class, such as IgG1, IgG2a, IgG2b and IgG3 in the case of mouse, and IgG1, IgG2, IgG3 and IgG4 in the case of human.

The mouse IgG1 and IgG2a and human IgG1 types have complement-dependent cytotoxicity activity (hereinafter as "CDC activity") and antibody-dependent cellular cytotoxicity activity (hereinafter as "ADCC activity"), so that they are useful in applying to medical treatments.

### 2. Production method of recombinant antibody (I) - Anti-human VEGF receptor Flt-1 humanized antibody

### (1) Construction of humanized antibody expression vector

In order to produce a humanized antibody from a non-human animal antibody, a humanized antibody expression vector is constructed. The humanized antibody expression vector is a vector for expression in animal cells into which a gene encoding CH and CL, C regions of a human antibody, have been inserted. Such an expression vector is constructed by inserting two genes, one encoding CH of a human antibody and the other encoding CL of a human antibody, into an expression vector for animal cells.

Any C regions of a human antibody such as Cγ1 and Cγ4 of a human antibody H chain, and Cκ of a human antibody L chain can be used. A chromosomal DNA consisting of an exon(s) and an intron(s) or cDNA can be used as a gene encoding a C region of a human antibody. Any expression vectors can be used as expression vectors for animal cells, so long as they can incorporate and express a gene encoding a C region of a human antibody.

Examples include pAGE107 [Cytotechnology, 3: 133 (1990) ] , pAGE103 [J. Biochem., 101: 1307 (1987) ] , pHSG274 [Gene, 27: 223 (1984)], pKCR [Proc. Natl. Acad. Sci. USA, 78: 1527 (1981)] and pSGI βd2-4[Cytotechnology, 4: 173 (1990)].

A promoter and an enhancer to be used for an expression vector for animal cells are exemplified by an SV40 early promoter and enhancer [J. Biochem., 101: 1307 (1987)], a Moloney mouse leukemia virus LTR promoter and enhancer [Biochem. Biophys. Res. Comun., 149: 960 (1987)], an immunoglobulin H chain promoter [Cell, 41: 479 (1985)] and enhancer [Cell, 33: 717 (1983)].

The humanized antibody expression vector may be either of a type in which a gene encoding an antibody H chain and a gene encoding an antibody L chain exist on separate vectors or of a type in which both genes exist on the same vector (tandem type). In terms of ease of construction of a humanized antibody expression vector, easiness of introduction into animal cells, balance between the expression amounts of antibody H and L chains in the animal cells and for other reasons, a tandem type of humanized antibody expression vector is more preferred [J. Immunol. Methods, 167: 271 (1994)] .

### (2) Isolation of cDNA encoding VH and VL of non-human animal antibody

cDNA encoding VH and VL of a non-human animal antibody such as a mouse anti-human VEGF receptor Flt-1 monoclonal antibody is isolated, for example, as follows:
mRNA is extracted from an anti-human VEGF receptor Flt-1 monoclonal antibody-producing cell, such as a mouse anti-human VEGF receptor Flt-1 antibody-producing hybridoma and the like, and used to synthesize cDNA. The synthesized cDNA is inserted into a vector, such as a phage, a plasmid or the like, to produce a cDNA library. From the library, with a portion in a V or C region of a non-human animal antibody such as a mouse antibody being used as a probe, a recombinant phage or plasmid which contains cDNA encoding VH and a recombinant phage or plasmid which contains cDNA encoding VL are isolated separately. The full nucleotide sequences of VH and VL of an antibody of interest which exist on the recombinant phage or plasmid are determined and the full amino acid sequences of the VH and VL are deduced from the nucleotide sequences.

### (3) Construction of human chimeric antibody expression vector

A human chimeric antibody expression vector can be constructed by inserting cDNA encoding VH and VL of a non-human animal antibody in a region upstream of the gene encoding CH and CL of the human antibody on the humanized antibody expression vector which has been constructed in the above 2(1). For example, a restriction enzyme recognition site for cloning of cDNA encoding VH and VL of a non-human animal antibody is created preliminarily in a region upstream of a gene encoding CH and CL of the human antibody on a chimeric antibody expression vector. At the cloning site, cDNA encoding a v region of a non-human animal antibody is inserted through a synthetic DNA (see below) to produce a human chimeric antibody expression vector. The synthetic DNA consists of a nucleotide sequence at the 3' end of a V region of the non-human animal and a nucleotide sequence at the 5' end of a C region of the human antibody and are produced by a DNA synthesizer such that it has appropriate restriction enzyme sites at both ends.

### (4) Identification of CDR sequences of non-human animal antibody

Each of VH and VL which form an antigen-binding site of an antibody consists of 4 framework regions (hereinafter referred to as "FR") having relatively conserved sequences and 3 complementarity-determining regions (CDRs) having a wide variety of sequences which link the FR regions [*Sequences of Proteins of Immunological Interest,* US Dept. Health and Human Services (1991) (hereinafter referred to as "*Sequences of Proteins of Immunological Interest*")]. The amino acid sequence of the respective CDR (CDR sequence) can be identified by comparison with the amino acid sequences of V regions of known antibodies (*Sequences of Proteins of Immunological Interest*).

### (5) Construction of cDNA encoding V region of human CDR-grafted antibody

cDNA encoding VH and VL of a human CDR-grafted antibody can be obtained as follows:

In the first step, for each of VH and VL, the amino acid sequence of FR in a V region of a human antibody to which CDR in a V region of a non-human animal antibody of interest is to be grafted is selected. Any amino acid sequences of FRs in V regions derived from human antibodies can be used as the amino acid sequences of FRs in V regions of human antibodies.

For example, the amino acid sequences of FRs in v regions of human antibodies recorded in Protein Data Bank and amino acid sequences common to subgroups of FRs in V regions of human antibodies (*Sequences of Proteins of Immunological Interest*) can be used. In order to produce a human CDR-grafted antibody having an excellent activity, an amino acid sequence having high homology, preferably homology of 65% or more, with the amino acid sequence of a V region of a non-human animal antibody of interest is desired. In the second step, a DNA sequence encoding the selected amino acid sequence of FR in a V region of a human antibody is ligated to a DNA sequence encoding the amino acid sequence of CDR in a V region of a non-human animal antibody and a DNA sequence encoding the amino acid sequences of VH and VL is designed. In order to obtain a DNA sequence designed to construct a CDR-grafted antibody variable region gene, several synthetic DNAs are designed for each chain such that the full DNA sequence is covered. Using the synthetic DNAs, polymerase chain reaction (hereinafter referred to as "PCR") is performed. For each chain, preferably 6 synthetic DNAs are designed in view of the reaction efficiency of PCR and the lengths of DNAs which can be synthesized. After the reaction, amplified fragments are subcloned into appropriate vectors and their nucleotide sequences are determined to obtain a plasmid which contains cDNA encoding the amino acid sequence of a v region of each chain of a human CDR-grafted antibody of interest. Alternatively, cDNA encoding the amino sequence of a V region of each chain of a human CDR-grafted antibody of interest may be constructed by synthesizing the full sequences of sense and antisense strand using synthetic DNAs consisting of about 100 bases and annealing and ligating them.

### (6) Modification of the amino acid sequence of V region of human CDR-grafted antibody

It is known that if a human CDR-grafted antibody is produced by simply grafting only CDR in a V region of a non-human animal antibody of interest between FRs in a V region of a human antibody, its activity is lower than that of the original non-human animal antibody [BIO/TECHNOLOGY, 9: 266 (1991)]. Hence, among the amino acid sequences of FR in a V region of a human antibody, an amino acid residue which takes part in direct binding to an antigen, an amino acid residue which interacts with an amino acid residue in CDR, or an amino acid residue which may take part in the maintenance of the steric structure of an antibody is modified to an amino acid residue that is found in the original non-human animal antibody such that the activity of the human CDR-grafted antibody is increased. For efficient identification of the amino acid residue, the steric structure of an antibody is constructed and analyzed by X-ray crystallography, or computer-modeling. However, no method for producing a human CDR-grafted antibody which can be applied to any antibodies has yet been established and, therefore, various attempts must currently be made on a case-by-case basis.

The modification of the selected amino acid sequence of FR in a V region of a human antibody can be accomplished using various primers for mutation by PCR described in the above 2(5). Amplified fragments obtained by the PCR are subcloned into appropriate vectors and their nucleotide sequences are determined to obtain a vector containing cDNA into which a mutation of interest has been introduced (hereinafter referred to as "amino acid sequence-modified vector").

Alternatively, the modification of an amino acid sequence in a narrow region may be accomplished by a PCR-mutagenesis method using primers for mutation consisting of 20 to 35 bases. More specifically, a sense mutation primer and an antisense mutation primer which consist of 20 to 35 bases and which contain DNA sequences encoding the amino acid residue to be modified are synthesized and used to perform 2-step PCR using as a plasmid as a template which contains cDNA encoding the amino acid sequence of a V region which is to be modified. The finally amplified fragments are subcloned into appropriate vectors and their nucleotide sequences are determined to obtain an amino acid sequence-modified vector containing cDNA into which a mutation of interest has been introduced.

### (7) Construction of human CDR-grafted antibody expression vector.

A human CDR-grafted antibody expression vector can be constructed by inserting the cDNA encoding VH and VL of the human CDR-grafted antibody obtained in the above 2(5) and 2(6) in a region upstream of the gene encoding CH and CL of the human antibody in the humanized antibody expression vector described in the above 2(1). For example, if recognition sites for appropriate enzymes are introduced at the ends of the 5' and 3' terminal synthetic DNAs during PCR for construction of cDNA encoding the amino acid sequences of VH and VL of the human CDR-grafted antibody, the cDNA can be inserted in a region upstream of a gene encoding a C region of a desired human antibody such that it is expressed in an appropriate form.

### (8) Transient expression of humanized antibodies and evaluation of their activities

In order to evaluate the activities of many humanized antibodies efficiently, the human chimeric antibody expression vector described in the above 2(3), and the human CDR-grafted antibody expression vector described in the above 2(7) or their modified vectors may be introduced into COS-7 cells (ATCC CRL 1651) and humanized antibodies expressed transiently [Methods in Nucleic Acids Res., CRC Press, p. 283 (1991)], followed by determination of their activities.

Examples of the method for introducing the expression vector into a COS-7 cell include a DEAE-dextran method [Methods in Nucleic Acids Res., CRC Press, p. 283 (1991)], a lipofection method [Proc. Natl. Acad. Sci. USA, 84: 7413 (1987)] and the like.

After introduction of the vector, the activities of the humanized antibodies in the culture supernatant can be determined by the enzyme immunoassay (ELISA) described in the above 1(5).

### (9) Stable expression of humanized antibodies and evaluation of their activities

Transformants which produce a humanized antibody stably can be obtained by introducing into appropriate host cells the human chimeric antibody expression vector described in the above 2(3) and the human CDR-grafted antibody expression vector described in the above 2(7).

Examples of the method for introducing the expression vector into host cells include electroporation [Japanese Published Unexamined Patent Application No. 257891/90, Cytotechnology, 3: 133 (1990)].

Any cells can be used as host cells into which the humanized antibody expression vector is to be introduced, so long as they can express a humanized antibody. Examples include mouse SP2/0-Ag14 cell (ATCC CRL1581), mouse P3X63-Ag8.653 cell (ATCC CRL1580), CHO cells which are detective in dihydrofolate reductase gene (hereinafter referred to as "DHFR gene") [Proc. Natl. Acad. Sci. USA, 77: 4216 (1980) ] and rat YB2/3HL.P2.G11.16Ag.20 cell (ATCC CRL1662, hereinafter referred to as "YB2/0 cell").

After introduction of the vector, transformants which express a humanized antibody stably are selected in accordance with the method disclosed in Japanese Published Unexamined Patent Application No. 257891/90, using an RPMI1640 medium containing G418 and FCS. The humanized antibody can be produced and accumulated in a culture medium by culturing the selected transformants in a medium. The activity of the humanized antibody in the culture medium is determined by the method described in the above 1 (5) or the like. The production of the humanized antibody by the transformants can be increased by the method described in Japanese Published Unexamined Patent Application No. 257891/90, utilizing a DHFR gene-amplification system.

The humanized antibody can be purified from the culture supernatant of the transformants by using a protein A column (Antibodies, A Laboratory Manual, Chapter 8). Any other conventional methods for protein purification can be used. For example, the humanized antibody can be purified by a combination of gel filtration, ion-exchange chromatography, and ultrafiltration. The molecular weight of the H chain or L chain of the purified humanized antibody or the antibody molecule as a whole is determined by polyacrylamide gel electrophoresis (SDS-PAGE) [Nature, 227: 680 (1970)], and Western blotting (Antibodies, A Laboratory Manual, Chapter 12).

The reactivity of the purified humanized antibody and the inhibition activity of the humanized antibody against human VEGF receptor Flt-1 can be determined by the method described in the above 1(5).

### 3. Production method of recombinant antibody (II)

### (1) Production method of antibody fragments, Fab, Fab' and F(ab')₂

Antibody fragments are formed by treating the above antibody with an enzyme. Examples of the enzyme include papain, and trypsin.

Alternatively, Fab, Fab' or F(ab')₂ can also be produced by inserting a DNA fragment which encodes the Fab, Fab' or F(ab')₂ fragment of the anti-human VEGF receptor Flt-1 antibody into an expression vector for animal cells and introducing the vector into an animal cell to express the fragment of interest.

The thus formed antibody fragments can be purified by carrying out suitable combination of techniques such as gel filtration, ion exchange chromatography, affinity chromatography and ultrafiltration. Molecular weight of the thus purified Fab, Fab' or F(ab')₂ fragment is measured by polyacrylamide gel electrophoresis (SDS-PAGE) [Nature, 227: 680 (1970)] or (Antibodies, A Laboratory Manual, Chapter 12).

Reactivity of the thus purified Fab, Fab' or F(ab')₂ and binding activity of Fab, Fab' or F(ab')₂ upon VEGF receptor Flt-1 can be measured by the method described in the above 1(5).

### (2) Production method of anti-human VEGF receptor Flt-1 single chain antibody

A vector for expression of a single chain antibody of a non-human animal antibody or a single chain antibody of a human CDR-grafted antibody can be constructed by inserting into scFv expression vector the cDNAs encoding VH and VL of a non-human animal antibody or a human CDR-grafted antibody which are described in the above 2(2), 2(5) and 2(6). Any expression vectors can be used as the single chain antibody expression vectors, so long as they can incorporate and express the cDNAs encoding VH and VL of a non-human animal antibody or a human CDR-grafted antibody.

Examples include pAGE107 *[*Cytotechnology, 3: 133 (1990)], pAGE103 [J. Biochem., 101: 1307 (1987)], pHSG274 [Gene, 27: 223 (1984)], pKCR [Proc. Natl. Acad. Sci. USA, 78: 1527 (1981)] and pSGI βd2-4 [Cytotechnololgy, 4: 173 (1990)].

A host for expressing the single chain antibody can be selected from among *Escherichia coli,* yeast, and animal cells. In this case, an expression vector which is compatible with the specific host should be selected. The single chain antibody can be secreted out of the cell and transported into the periplasm region or retained within the cell by inserting a cDNA encoding an appropriate signal peptide into the expression vector.

An single chain antibody expression vector into which the cDNA encoding the single chain antibody of interest has been inserted can be constructed by inserting the cDNA encoding scFv consisting of VH-P-VL or VL-P-VH (where P is a peptide linker) into the selected expression vector in a region downstream of an appropriate promoter and a signal peptide.

The cDNA encoding the single chain antibody can be obtained by linking a VH encoding cDNA to a VL encoding cDNA through a synthetic DNA encoding a peptide linker having recognition sites for appropriate restriction enzymes at both the ends. It is important to optimize the linker peptide such that its addition does not interfere with the binding of VH and VL to an antigen. For example, the linker descried by Pantoliano et al. [Biochemistry, 30: 10117 (1991)] and its modified versions may be used.

### (3) Production of anti-human VEGF receptor Flt-1 disulfide stabilized antibody

A disulfide stabilized antibody can be produced by a process comprising the steps of providing cDNAs encoding VH and VL of a non-human animal antibody or cDNAs encoding VH and VL of a human CDR-grafted antibody, modifying the DNA sequence which corresponds to a one-amino acid residue at an appropriate position in the respective cDNA with a DNA sequence corresponding to a cysteine residue, expressing the modified cDNAs and purifying the resultant peptide and then forming a disulfide bond. The modification of an amino acid residue to a cysteine residue can be performed by a mutagenesis method using PCR described in the above 2(5).

A disulfide stabilized antibody H chain expression vector and a disulfide stabilized antibody L chain expression vector can be constructed by inserting the resulting cDNAs encoding the modified VH and modified VL into appropriate expression vectors. Any expression vectors can be used as the disulfide stabilized antibody expression vectors, so long as they can incorporate and express cDNAs encoding a modified VH and a modified VL. Examples include pAGE107 [Cytotechnology, 3: 133 (1990)], pAGE103 [J. Biochem., 101: 1307 (1987)], pHSG274 (Gene, 27: 223 (1984)], pKCR [Proc. Natl. Acad. Sci. USA, 78: 1527 (1981)], and pSGI βd2-4 [Cytotechnololgy, 4: 173 (1990)]. A host used to express a disulfide stabilized antibody L chain expression vector and a disulfide stabilized antibody H chain expression vector for formation of the disulfide stabilized antibody can be selected from among *Escherichia coli,* yeast, and animal cells.

In this case, an expression vector which is compatible with the specific host should be selected.

The disulfide stabilized antibody can be secreted out of the cell and transported into the periplasm region or retained within the cell by inserting a cDNA encoding an appropriate signal peptide into the expression vector.

### (4) Expression of various antibodies and evaluation of their activity

A transformant which produces an antibody fragment, scFv, a disulfide stabilized antibody H chain or a disulfide stabilized antibody L chain of interest can be obtained by introducing into a host cell the antibody fragment expression vector, the single chain antibody expression vector, the disulfide stabilized antibody H chain expression vector or the disulfide stabilized antibody L chain expression vector that was constructed in the above 3 (1) to 3(3) by electroporation [Japanese Published Unexamined Patent Application No. 257891/90; Cytotechnology, 3: 133 (1990)].

After introduction of the expression vector, the expression of the antibody fragment, the single chain antibody, the disulfide stabilized antibody H chain, or the disulfide stabilized antibody L chain in the culture supernatant can be confirmed by the method described in the above 1(5).

The collection and purification of the antibody fragment, the single chain antibody, the disulfide stabilized antibody H chain, or the disulfide stabilized antibody L chain can be accomplished by combinations of known techniques. For example, if the antibody fragment, the single chain antibody, the disulfide stabilized antibody H chain, or the disulfide stabilized antibody L chain is secreted in a medium, they can be concentrated by ultrafiltration and their collection and purification can be then performed by various types of chromatography or gel filtration. If the antibody fragment, the single chain antibody, the disulfide stabilized antibody H chain, or the disulfide stabilized antibody L chains is transported into the periplasm region of the host cell, they can be concentrated by ultrafiltration after the application of an osmotic shock to the cell and their collection and purification can be then performed by various types of chromatography or gel filtration. If the antibody fragment, the single chain antibody, the disulfide stabilized antibody H chain, or the disulfide stabilized antibody L chain is insoluble and exists as a granule (i.e., inclusion body), their collection and purification can be performed by lysis of the cells, repeated centrifugation and washing for isolation of the granule, solubilization with guanidine-HCl and subsequent performance of various types of chromatography or gel filtration.

The purified single chain antibody can be measured by the method described in the above 1(5).

The purified disulfide stabilized antibody H chain and disulfide stabilized antibody L chain are mixed and subjected to a refolding procedure for deriving an active structure [Molecular Immunology, 32: 249 (1995)] to form a disulfide bond. Subsequently, the active disulfide stabilized antibody can be purified by antigen affinity chromatography or ion-exchange chromatography or gel filtration. The activity of the disulfide stabilized antibody can be determined by the method described in the above 1(5),

### 4. Production method of fusion antibody

The fusion antibodies produced by chemically or by gene-engineeringly linking a radioactive isotope, a protein or a low molecular agent with the antibody of the present invention can also be used as derivatives of the antibody.

A fusion antibody in which an antibody and a toxic protein are chemically linked can be produced in accordance, for example, with the method described in the literature [Anticancer Research, 11: 2003 (1991), Nature Medicine, 3: 350 (1996)].

A fusion antibody in which an antibody and a toxin or a protein such as cytokine or an enzyme is gene-engineeringly linked can be produced in accordance, for example, with the method described in the literature [Proc. Natl. Acad. Sci. USA, 93: 974 (1996), Proc. Natl. Acad. Sci. USA, 93: 7826 (1996)].

A fusion antibody in which an antibody and a low molecular weight anticancer agent are chemically linked can be produced in accordance, for example, with the method described in the literature [Science, 261: 212 (1993)].

A fusion antibody in which an antibody and a radioactive isotope are chemically linked can be produced in accordance, for example, with the method described in the literature [Antibody Immunoconjugates and Radiopharmaceuticals, 3: 60 (1990), Anticancer Research, 11: 2003 (1991)].

It is expected that these derivatives can provide more effective and side effect-reduced diagnoses and treatments by accumulating a radioactive isotope, a protein, such as cytokine, a toxin, or an enzyme, or a low molecular agent within the peripheral of a target tissue, based on the specificity of antibody molecules.

### 5. Method of use of antibody (I)

The above-described anti-human VEGF receptor Flt-1 antibodies, and fusion antibodies fused with other molecules bind to human VEGF receptor Flt-1 and, via antibody effector activities, such as ADCC, and CDC, destroy cells in which VEGF receptor Flt-1 is expressed on the cell surface, so that they are useful in treating diseases in which the morbid states progress by abnormal angiogenesis, which include proliferation or metastasis of solid tumors, such as breast cancer, prostatic cancer, large bowel cancer, gastric cancer and lung cancer, arthritis in chronic rheumatoid arthritis, diabetic retinopathy, retinopathy of prematurity, and psoriasis.

The pharmaceutical preparation containing the antibody, or fusion antibody of the present invention can be administered directly as a treating agent, but it is generally preferred to provide it in the form of a pharmaceutical medicament produced by mixing it with at least one pharmacologically acceptable carrier in accordance with optional methods well known in the technical field of pharmaceutics.

It is preferred to select a route of administration which is the most effective in carrying out the intended treatment, such as oral administration or parenteral administration that includes tracheal administration, rectal administration, subcutaneous injection, intramuscular injection, and intravenous injection. Intravenous injection is preferred in the case of an antibody preparation.

The dosage form includes sprays, capsules, tablets, granules, syrups, emulsions, suppositories, injections, ointments, and tapes.

Examples of the pharmaceutical preparation suitable for oral administration include emulsions, syrups, capsules, tablets, powders, and granules.

Liquid preparations, such as emulsions and syrups, are produced using additives, such as water, saccharides (e.g., sucrose, sorbitol, fructose, *etc*.), glycols (e.g., polyethylene glycol, propylene glycol, *etc*.), oils (e.g., sesame oil, olive oil, soybean oil, *etc.*), antiseptics (e.g., *p*-hydroxybenzoic acid esters, *etc.*), and flavors (e.g., strawberry flavor, peppermint, *etc*.).

Solid preparations, such as capsules, tablets, powders, and granules, can be produced using additives, such as fillers (e.g., lactose, glucose, sucrose, mannitol, etc.), disintegrating agents (e.g., starch, sodium alginate, etc.), lubricating agents (e.g. magnesium stearate, etc.), binders (e.g., polyvinyl alcohol, hydroxypropylcellulose, gelatine, etc.), surfactants (e.g., fatty acid esters, etc.), and plasticizers (e.g., glycerol, etc.).

Preferred examples of pharmaceutical preparations suitable for parenteral administration include infection, suppositories, and sprays.

Injections are prepared using a carrier such as a salt solution, glucose solution or a mixture thereof.

Suppositories are prepared using a carrier, such as cacao butter, hydrogenated fat, or a carboxylic acid.

Sprays are prepared from the compound itself or using a carrier which does not stimulate oral and airway mucous membranes of patients and can facilitate absorption of the compound by dispersing it as minute

Examples of the carrier include lactose, and glycerol. Depending on the properties of the antibody and the carrier to be used, other preparations, such as aerosols and dry powders, can be produced. The components exemplified as additives of oral preparations can also be added to these parenteral preparations.

The dose and frequency of administration vary depending on the conditions such as intended therapeutic effect, administration method, treating period, age and body weight, but the dose is generally from 10 µg/kg to 8 mg/kg per day per adult.

The antibodies of the present invention specific for human VEGF receptor Flt-1 can be used as a diagnostic agent or a treating agent of diseases in which the morbid states progress by abnormal angiogenesis which are proliferation or metastasis of solid tumors such as breast cancer, prostatic cancer, large bowel cancer, gastric cancer, and lung cancer, arthritis in chronic rheumatoid arthritis, diabetic retinopathy, retinopathy of prematurity, and psoriasis.

In addition, since the antibodies of the present invention can inhibit activity which neutralizes human VEGF, auto-phosphorylation of Flt-1 can be inhibited by inhibiting binding of human VEGF to Flt-1. As a result, they can inhibit VEGF-dependent proliferation of human vascular endothelial cells and therefore can be used as an agent for treating diseases in which their morbid, states progress by abnormal angiogenesis, which are proliferation or metastasis of solid tumors such as breast cancer, prostatic cancer, large bowel cancer, gastric cancer and lung cancer, arthritis in chronic rheumatoid arthritis, diabetic retinopathy, retinopathy of prematurity, and psoriasis.

The methods for the examination of antitumor effects of the antibody of the present invention upon various tumor cells include complement-dependent cytotoxicity activity (CDC activity) assay, and antibody-dependent cellular cytotoxicity activity (ADCC activity) assay as in vitro tests. An antitumor test in which a tumor system of an experimental animal, such as mouse, is used can be exemplified as *in vivo* tests.

The CDC activity assay, the ADCC activity assay and the antitumor test can be carried out in accordance, for example, with the methods described in the literature [Cancer Immunology Immunotherapy, 36: 373 (1993); Cancer Research, 54: 1511 (1994*)*]*.*

### 6. Method of use of antibody (II)

Furthermore, the present invention relates to an in vitro method for immunologically detecting and determining human VEGF receptor Flt-1 or cells, in which human VEGF receptor Flt-1 is expressed on the surface thereof, an in vitro method for immunologically detecting and determining soluble human VEGF receptor Flt-1, an in vitro method for inhibiting binding of a human VEGF to human VEGF receptor Flt-1, an in vitro method for neutralizing the function of human VEGF receptor Flt-1, and an in vitro method for inhibiting migration of vascular endothelial cells.

The method for immunologically detecting and determining human VEGF receptor Flt-1 or cells in which human VEGF receptor Flt-1 is expressed on the surface thereof and the method for immunologically detecting and determining soluble human VEGF receptor Flt-1 using the antibody of the present invention include a fluorescent antibody method, an enzyme-linked immunosorbent assay (ELISA), a radioactive material labeled immunoassay (RIA), an immunocyte chemical staining method, such as an immunocyte staining method, an immunotissue staining method, or the ABC method, CSA method, Western blotting method, an immunoprecipitation method, the above-described enzyme immunoassay, a sandwich ELISA [Monoclonal Antibody Experiment Manual (published by Kodansha Scientific, 1987), Second Series Biochemical Experiment Course, Vol. 7, Immunobiochemistry Research Method, published by Tokyo Kagaku Dojin (1986)].

The fluorescent antibody method comprises reacting a separated cell, on tissue with the antibody of the present invention, reacting the reacted sample with an anti-immunoglobulin antibody or binding fragment labeled with a fluorescence substance, such as fluorescein isothiocyanate (FITC), and then measuring the fluorescence substance with a flow cytometer.

The enzyme-linked immunosorbent assay (ELISA) comprises reacting a separated cell or crushing solution thereof, tissue or crushing solution thereof, serum, preural fluid, ascites fluid, or ocular fluid with the antibody of the present invention, further reacting the reacted sample with an anti-immunoglobulin antibody or binding fragment labeled with an enzyme, such as peroxydase, or biotin, and then measuring the resultant developed dye with an absorption measuring apparatus.

The radioactive material labeled immunoassay (RIA) comprises reacting a separated cell or crushing solution thereof, tissue or crushing solution thereof, serum, preural fluid, ascites fluid, or ocular fluid with the antibody of the present invention, reacting the reacted sample with an anti-immunoglobulin antibody or binding fragment labeled with radioactive ray, and then measuring the radioactive ray with a scintillation counter.

The immunocyte staining and immunotissue staining methods comprise reacting a separated cell, or tissue with the antibody of the present invention; reacting the reacted sample with an anti-immunoglobulin antibody or binding fragment labeled with a fluorescence substance, such as fluorescein isothiocyanate (FITC) or an enzyme, such as peroxydase, or biotin, and then observing the cell, or tissue with a microscope.

Furthermore, the activity of inhibiting binding of human VEGF and human VEGF receptor Flt-1 can be confirmed by carrying out a VEGF-VEGF receptor Flt-1 binding inhibition test using the antibody of the present invention in accordance, for example, with the measuring method of binding between growth factors and receptors described in New Biochemical Experiment Course, Vol. 7, Growth and Differentiation Factors and their Receptors, published by Tokyo Kagaku Dojin (1991).

In this method, VEGF labeled with a radioactive material is allowed to react with cells or tissues in which Flt-1 is expressed, and the VEGF bound to the Flt-1 expressing cells or tissues is measured using a scintillation counter. The activity of inhibiting binding of VEGF labeled with a radioactive material or the like to Flt-1 can be measured by reacting the antibody of the present invention together with the radioactive material-labeled VEGF.

Auto-phosphorylation inhibition activity of VEGF receptor Flt-1 can be confirmed by carrying out a VEGF-VEGF receptor Flt-1 auto-phosphorylation inhibition test using a antibody in accordance, for example, with the measuring method of auto-phosphorylation of growth factor receptors [Second Series Biochemical Experiment Course, Signal Transduction and Cellular Response, published by Tokyo Kagaku Dojin (1986)].

In this method, VEGF is allowed to react with cells or tissues in which Flt-1 is expressed, and auto-phosphorylation of Flt-1 accelerated by the binding of VEGF is detected by immunoprecipitation or Western blotting. The activity of inhibiting auto-phosphorylation of Flt-1 accelerated by the binding of VEGF can be measured by reacting the antibody of the present invention together with VEGF.

The activity of inhibiting human VEGF can be confirmed by carrying out growth, migration and tube formation tests of VEGF dependent vascular endothelial cells [New Biochemical Experiment Course, Vol. 10, Blood Vessels (Endothelium and Smooth Muscle), published by Tokyo Kagaku Dojin (1991)].

The growth test of VEGF dependent vascular endothelial cells is a method in which VEGF is allowed to react with vascular endothelial cells, and the growth enhancing activity of vascular endothelial cells accelerated by the binding of VEGF is measured by counting the number of cells. The activity of inhibiting growth enhancing activity of vascular endothelial cells accelerated by VEGF can be measured by reacting the antibody of the present invention together with VEGF.

The migration test of VEGF dependent vascular endothelial cells is a method in which VEGF is allowed to react with vascular endothelial cells, and the migration enhancing activity of vascular endothelial cells accelerated by the binding of VEGF is observed under a microscope. The activity of inhibiting migration enhancing activity of vascular endothelial cells accelerated by VEGF can be measured by reacting the antibody of the present invention together with VEGF.

The tube formation test of VEGF dependent vascular endothelial cells is a method in which VEGF is allowed to react with vascular endothelial cells, and the tube formation enhancing activity of vascular endothelial cells accelerated by the binding of VEGF is observed under a microscope. The activity of inhibiting tube formation enhancing activity of vascular endothelial cells accelerated by VEGF can be measured by reacting the antibody of the present invention together with VEGF.

An example thereof will be explained below.

### (1) Immunocyte staining using anti-human VEGF receptor Flt-1 antibody

First, the cells in which human VEGF receptor Flt-1 is expressed on the cell surface are prepared. Suspending cells as such or adherent cells after detachment of the cells using trypsin-EDTA are suspended, for example, in a buffer solution for immunocyte staining use (PBS containing 1% BSA, 0.02% EDTA and 0.05% sodium azide) and dispensed in 1×10⁵ to 2×10⁶ portions. A culture supernatant of the anti-human VEGF receptor Flt-1 monoclonal antibody-producing hybridoma obtained in 1(4), a culture supernatant of the anti-human VEGF receptor Flt-1 humanized antibody-producing transformant obtained in 2(9), the purified monoclonal antibody obtained in 1(6), 2(9) or 3(4) or the monoclonal antibody labeled with biotin by a known method [Enzyme Antibody Method: published by Gakusai Kikaku (1985)] is diluted with the buffer solution for immunocyte staining use or the buffer solution for immunocyte staining use further supplemented with 10% animal serum to a concentration of 0.1 to 50 µg/ml and dispensed in 20 to 500 µl portions to carry out the reaction under cooling for 30 minutes. When the culture supernatant of the anti-human VEGF receptor Flt-1 monoclonal antibody-producing hybridoma obtained in 1(4), the culture supernatant of the anti-human VEGF receptor Flt-1 humanized antibody-producing transformant obtained in 2(9), the purified monoclonal antibody obtained in 1(6), 2(9) or 3(4) is used in the reaction, the cells are washed with the buffer solution for immunocyte staining use and then an anti-mouse immunoglobulin antibody or anti-rat immunoglobulin labeled with fluorescence dye, such as FITC, phycoerythrin, or the like, which is dissolved in the buffer solution for immunocyte staining use to a concentration of about 0.1 to 50 µg/ml, is dispensed in 50 to 500 µl portions to carry out the reaction under cooling for 30 minutes. When the monoclonal antibody labeled with biotin is used in the reaction, streptoavidin is dispensed in 50 to 500 µl portions and then the reaction is carried out under cooling in the dark for 30 minutes. After completion of the reaction, the cells are thoroughly washed with the buffer solution for immunocyte staining use and analyzed by a cell sorter.

### (2) Detection of human VEGF receptor Flt-1 by Western blotting

Cell membrane components are prepared from cells in which human VEGF receptor Flt-1 is expressed, such as human VEGF receptor Flt-1-expressing NIH3T3 cells (referred to as "NIH3T3-Flt-1" hereinafter), and control cells such as NIH3T3 cells (referred to as "NIH3T3-Neo" hereinafter) [Oncogene, 10: 135 (1995)] and the membrane components are electrophoresed by the SDS-PAGE method under reducing conditions in an amount of 0.1 to 30 µg as protein per lane. The thus treated proteins are transferred on a PVDF membrane and allowed to react with PBS containing 1% BSA at room temperature for 30 minutes to carry out blocking. They are allowed to react with the culture supernatant of the anti-human VEGF receptor Flt-1 monoclonal antibody-producing hybridoma obtained in 1(4), the culture supernatant of the anti-human VEGF receptor Flt-1 humanized antibody-producing transformant obtained in 2(9), or the purified monoclonal antibody obtained in 1(6), 2 (9) or 3(4), washed with PBS, containing 0.05% Tween and then allowed to react with peroxidase-labeled goat anti-mouse IgG at room temperature for 2 hours. After washing with PBS containing 0.05% Tween, bands to which the anti-human VEGF receptor Flt-1 monoclonal antibody is bound are detected using ECL Western blotting detection reagents (manufactured by Amersham) or the like.

### (3) Determination of soluble VEGF receptor Flt-1 using anti-human VEGF receptor Flt-1 antibody

As a first antibody, the purified monoclonal antibody obtained in 1(6), 2(9) or 3(4) is coated on an appropriate plate and allowed to react with 0.056 to 10,000 ng/ml of a recombinant antibody, such as the human VEGF receptor Flt-1 obtained in 1(1), a fusion protein of the human VEGF receptor Flt-1 and other protein, or the like, or with a sample, such as human serum or the like. After through washing of the plate, this is allowed to react with a second antibody, namely a monoclonal antibody or humanized antibody labeled with biotin, an enzyme, a chemiluminescence substance, a radioactive compound or the like, which is one of the purified monoclonal antibodies obtained in 1(6), 2(9) or 3(4) but recognizes different epitope from that of the monoclonal antibody used as the first antibody, under reaction conditions suitable for binding of the labeled antibody to the epitope with which it binds. A calibration curve is prepared based on the reactivity for the purified soluble VEGF receptor Flt-1, and the concentration of the soluble VEGF receptor Flt-1 in the samples is calculated.

The method for inhibiting binding of human VEGF to human VEGF receptor Flt-1 is exemplified below.

### (4) Inhibition test of binding of VEGF-VEGF receptor Flt-1 using monoclonal antibody

Methanol is dispensed in 100 µl portions into wells of a 96-well MultiScreen-IP Plate (manufactured by Millipore) to give hydrophilic nature to the PVDF membrane on the bottom of the plate. After washing with water, human VEGF receptor Flt-1 or a recombinant protein, such as a fusion protein of human VEGF receptor Flt-1 with other protein or the like, is diluted to a concentration of 0.1 to 10 µg/ml, dispensed in 50 µl/well portions and then allowed to stand overnight at 4°C for its adsorption. After washing, PBS containing 1% bovine serum albumin (BSA) is dispensed in 100 µl/well portions and the reaction is carried out at room temperature for 1 hour to block any remaining active residues. After washing with PBS, the culture supernatant of the anti-human VEGF receptor Flt-1 monoclonal antibody-producing hybridoma obtained in the above 1(4), the culture supernatant of the anti-human VEGF receptor Flt-1 humanized antibody-producing transformant obtained in the above 2(9), or the purified monoclonal antibody obtained in the above 1(6), 2(9) or 3(4) is dispensed in 50 µl/well portions and then 0.1 to 10 ng/ml of ¹²⁵I-labeled VEGF (manufactured by Amersham) is dispensed in 50 µl/well portions, subsequently carrying out the reaction at room temperature for 1.5 hours: After washing witch 0.05% Tween-PBS, the wells are dried at 50°C, and a scintillator is dispensed in 20 to 100 µl/well portions to measure the radioactivity of the ¹²⁵I-labeled VEGF linked to each well using Top Count (manufactured by Packard) or the like.

### 7. Use for treating diseases based on proliferation or

metastasis of solid tumors, such as breast cancer, prostatic cancer, large bowel cancer, gastric cancer, lung cancer, and diseases in which their morbid states progress by abnormal angiogenesis

The use for treating diseases based on proliferation or metastasis of solid tumors, such as breast cancer, prostatic cancer, large bowel cancer, gastric cancer, and lung cancer and diseases in which their morbid states progress by abnormal angiogenesis includes the above use for immunologically detecting or determining human VEGF receptor Flt-1 present in a cell or tissue of a subject using the antibody of the present invention.

### Brief Explanation of the Drawings

Fig. 1 is a graph showing construction steps of plasmid pBS1732H.
Fig. 2 is a graph showing construction steps of plasmid pHS1732L.
Fig. 3 is a graph showing construction steps of plasmid pKANTEX1732H.
Fig. 4 is a graph showing construction steps of plasmid pKANTEX1732.
Fig. 5 is a graph showing construction steps of plasmid pBS1750H.
Fig. 6 is a graph showing construction steps of plasmid pBS1750L.
Fig. 7 is a graph showing construction steps of plasmid pKANTEX1750H.
Fig. 8 is a graph showing construction steps of plasmid pKANTEX1750.
Fig. 9 is a graph showing SDS-PAGE (a 4 to 15% gradient gel was used) electrophoresis patterns of purified anti-human VEGF receptor Flt-1 human chimeric antibodies KM2532 and KM2550. Lane 1: low molecular weight marker, lane 2: KM2532 under reducing conditions, lane 3: KM2550 under reducing conditions, lane 4: high molecular weight marker, lane 5: KM2532 under non-reducing conditions, lane 6: KM2550 under non-reducing conditions.

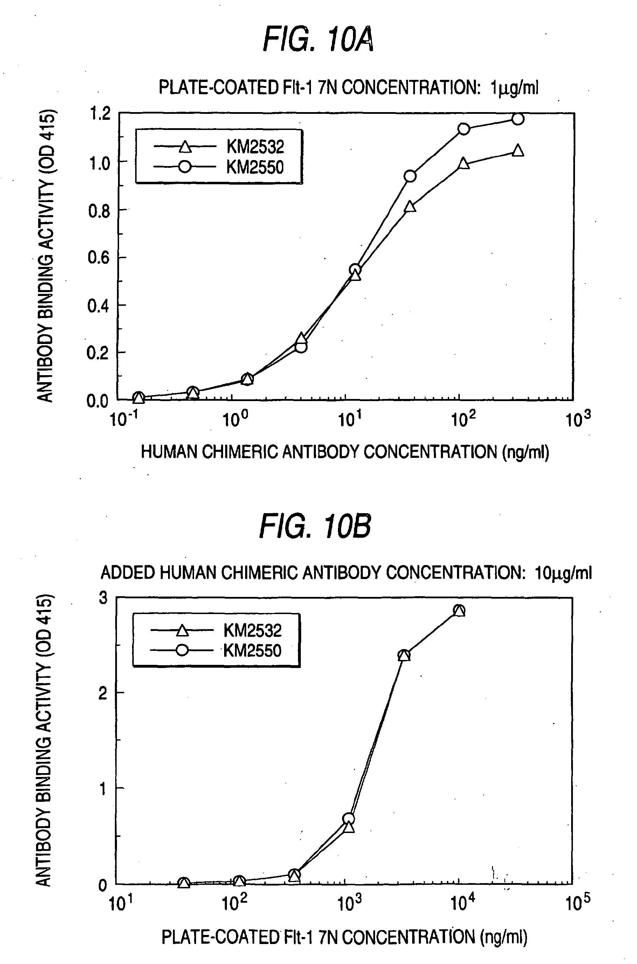

Fig. 10 is a graph showing the activity of purified anti-human VEGF receptor Flt-1 human chimeric antibodies KM2532 and KM2550 to bind to soluble human VEGF receptor Flt-1 7N. (A) shows the results when concentration of soluble human VEGF receptor Flt-1 7N to be adsorbed on the plate is fixed (1 µg/ml) and concentration of each human chimeric antibody to be added is varied. The binding activity with soluble human VEGF receptor Flt-1 7N is plotted as ordinate and the concentration of each human chimeric antibody as abscissa. "Δ" indicates binding activity of KM2532 and "O" indicates that of KM2550. (B) shows the results when binding activity of each human chimeric antibody at a fixed concentration (10 µg/ml) was measured by varying concentration of soluble human VEGF receptor Flt-1 7N to be adsorbed on the plate. The binding activity with soluble human VEGF receptor Flt-1 7N is plotted as ordinate, and the concentration of soluble human VEGF receptor Flt-1 7N adsorbed on the plate as abscissa. "Δ" indicates binding activity of KM2532 and "O" indicates that of KM2550.
Fig. 11 is a graph showing the activity of purified anti-human VEGF receptor Flt-1 human chimeric antibodies, KM2532 and KM2550 to inhibit binding between human VEGF and human VEGF receptor Flt-1. "○", "□", "●" and "■" shows the binding inhibition activities of KM1732, KM1750, KM2532 and KM2550, respectively.
Fig. 12 is a graph showing construction steps of plasmid phKM1732HV0.
Fig. 13 is a graph showing construction steps of plasmid phKM1750HV0.
Fig. 14 is a graph showing construction steps of plasmid phKM1732LV0.
Fig. 15 is a graph showing construction steps of plasmid phKM1750LV0 (IV).
Fig. 16 is a graph showing construction steps of plasmid pKANTEX1732HV0.
Fig. 17 is a graph showing construction steps of plasmid pKANTEX1732HV0LV0.
Fig. 18 is a graph showing plasmid pKANTEX1750HV0LV0(IV).
Fig. 19 is a graph showing construction steps of plasmid pVL1393/Flt 3N.
Fig. 20 is a graph showing construction steps of plasmid pVL1393/Flt 7N.
Fig. 21 is a graph showing patterns of SDS polyacrylamide electrophoresis (a 5 to 20% gradient gel was used) of purified Flt-1 7N and Flt-1 3N. Starting from the left side, electrophoresis patterns of molecular weight markers, Flt-1 3N and Flt-1 7N are shown respectively. The electrophoresis was carried out under reducing conditions.
Fig. 22 is a graph showing results of the analysis of the effect of soluble human VEGF receptors Flt-1 7N and Flt-1 3N to inhibit binding of a ¹²⁵I-human VEGF to a plate-coated soluble human VEGF receptor Flt-1 7N.
Fig. 23 is a graph showing results of the examination of the binding reactivity of anti-human VEGF receptor Flt-1 monoclonal antibody by enzyme immunoassay.

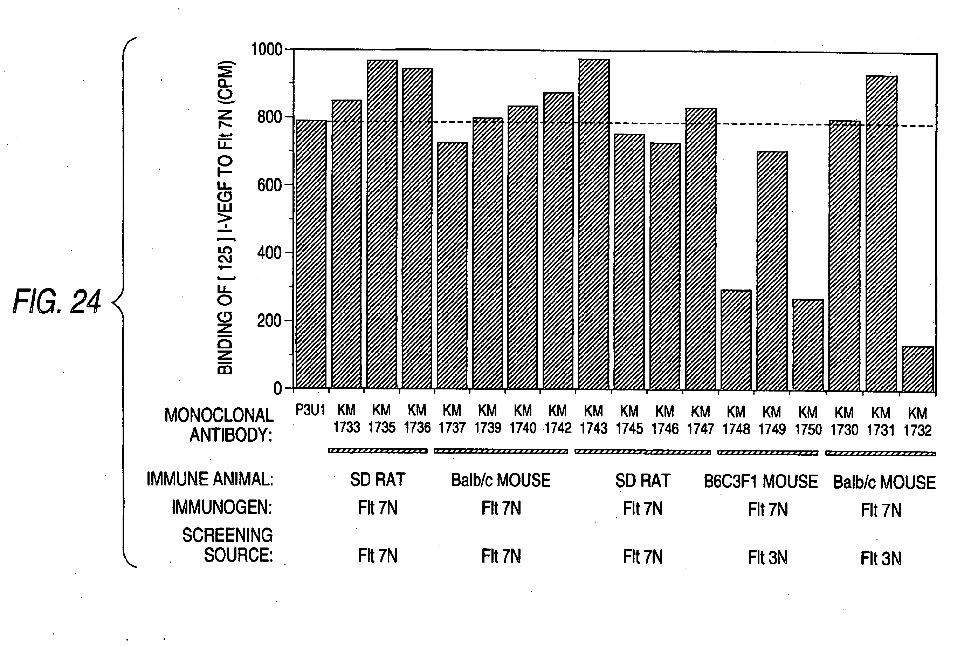

Fig. 24 is a graph showing results of the examination of the activity of anti-human VEGF receptor Flt-1 monoclonal antibody to inhibit binding of VEGF to human VEGF receptor Flt-1.
Fig. 25 is a graph showing results of the examination of the activity of anti-human VEGF receptor Flt-1 monoclonal antibodies KM1732, KM1748 and KM1750 to inhibit binding of human VEGF to human VEGF receptor Flt-1.
Fig. 26 is a graph showing results of the examination of the activity of anti-human VEGF receptor Flt-1 monoclonal antibodies KM1732, KM1748 and KM1750 to inhibit binding of human VEGF to human VEGF receptor Flt-1-expressing cells.
Fig. 27 is a graph showing results of the flow cytometry analysis of the reactivity of anti-human VEGF receptor Flt-1. monoclonal antibodies KM1730, KM1731, KM1732, KM1748 and KM1750 with human VEGF receptor Flt-1-expressing cells NIH3T3-Flt-1 and control cells NIH3T3-Neo cells.
Fig. 28 is a graph showing results of the examination of the reactivity of anti-human VEGF receptor Flt-1 monoclonal antibody KM1737 with human VEGF receptor Flt-1 by Western blotting. Lane 1 shows Western blotting pattern of NIH3T3-Flt-1 cells and lane 2 shows the pattern of NIH3T3-Neo cells.
Fig. 29 is a graph showing results of the examination of the determination system of soluble human VEGF receptors Flt-1 3N and Flt-1 7N, carried out using anti-human VEGF receptor Flt-1 monoclonal antibodies KM1732 and biotinated KM1730.
Fig. 30 is a graph showing the binding activity of anti-human VEGF receptor Flt-1 human monoclonal antibodies KM1732 and KM1750 and anti-human VEGF receptor Flt-1 human chimeric antibodies KM2532 and KM2550 upon soluble human VEGF receptor derivatives Flt-1 7N, Flt-1 3N, Flt-1 2N, Flt-1 7N.K2 and KDR-7N according to the enzyme immunoassay.
Fig. 31 is a graph showing the concentration-dependent binding activity of anti-human VEGF receptor Flt-1 human chimeric antibodies KM2532 and KM2550 upon soluble human VEGF receptor derivatives Flt-1 7N, Flt-1 3N, Flt-1 2N, Flt-1 7N.K2 and KDR-7N according to the enzyme immunoassay.
Fig. 32 is a schematic illustration of soluble human VEGF receptor derivatives.
Fig. 33 is a graph showing construction steps of plasmid phKM1750LV0 (I).
Fig. 34 is a graph showing plasmid pKANTEX1750HV0LV0 (I).
Fig. 35 is a graph showing construction steps of plasmid phKM1750HV3.
Fig. 36 is a graph showing construction steps of plasmid phKM1750LV4.
Fig. 37 is a graph showing plasmid pKANTEX1750HV3LV0 (I).
Fig. 38 is a graph showing plasmid pKANTEX1750HV3LV0 (IV).
Fig. 39 is a graph showing plasmid pKANTEX1750HV0LV4.
Fig. 40 is a graph showing plasmid pKANTEX1750HV3LV4.

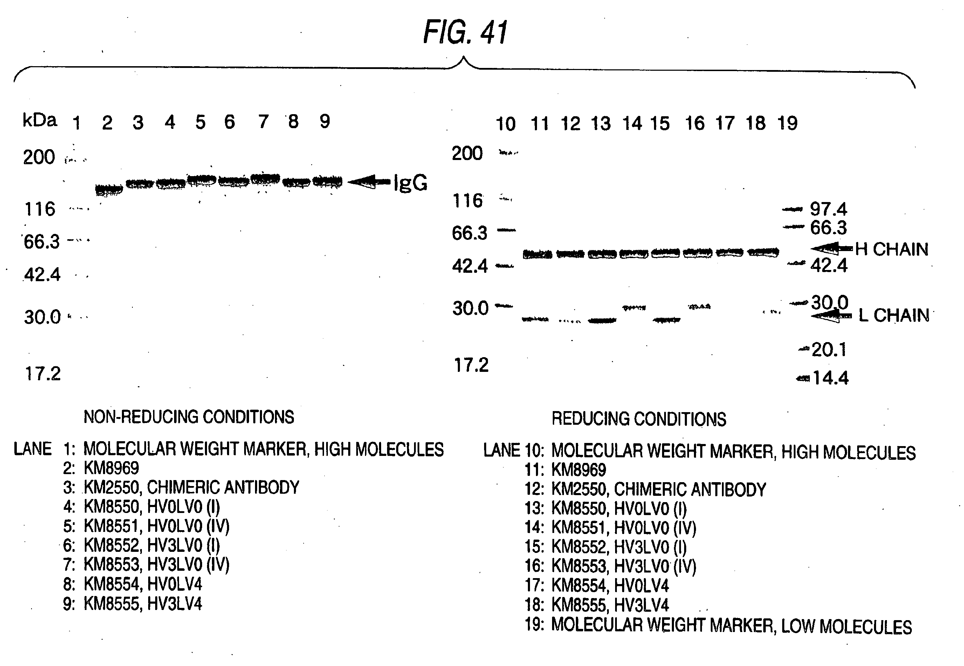

Fig. 41 is a graph showing SDS-PAGE (a 4 to 15% gradient gel was used) electrophoresis patterns of anti-human VEGF receptor Flt-1 human chimeric antibody KM2550 and human CDR-grafted antibodies KM8550, KM8551, KM8552, KM8553, KM8554 and KM8555. Lanes 1 to 9 show electrophoresis patterns under non-reducing conditions and lanes 10 to 19 shows electrophoresis patterns under reducing conditions. Lanes 1 and 10: high molecular weight marker, lane 19: low molecular weight marker, lanes 2 and 11: control antibody KM8969, lanes 3 and 12: KM2550, lanes 4 and 13: KM8550, lanes 5 and 14: KM8551, lanes 6 and 15: KM8552, lanes 7 and 16: KM8553, lanes 8 and 17 : KM8554, and lanes 9 and 18: KM8555.
Fig. 42 is a graph showing the binding activity of anti-human VEGF receptor Flt-1 human chimeric antibody KM2550 and human CDR-grafted antibodies KM8550, KM8551, KM8552, K8553, KM8554 and KM8555 upon soluble human VEGF receptor Flt-1 7N.

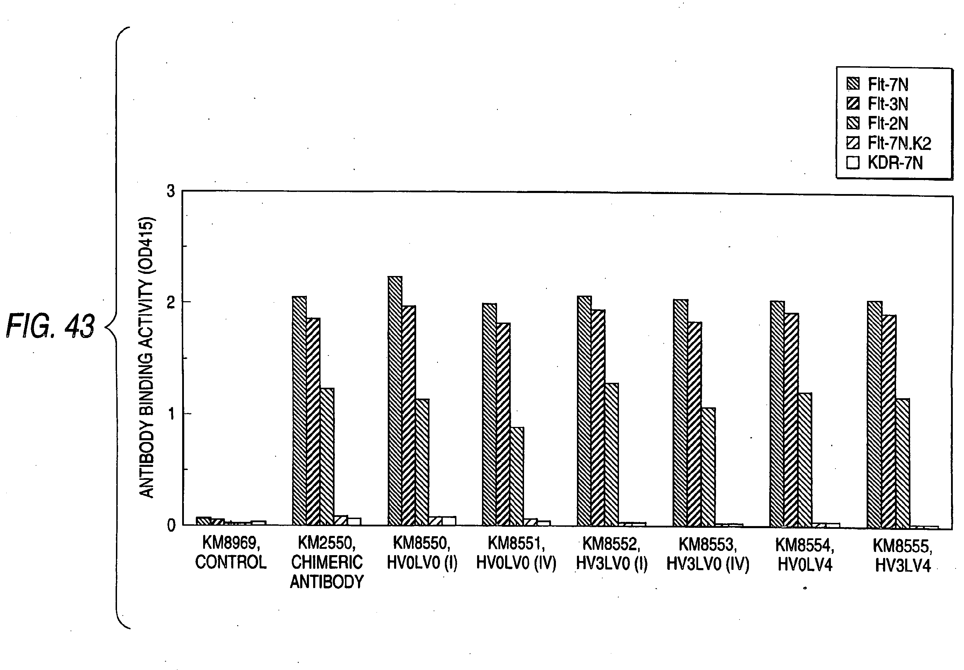

Fig. 43 is a graph showing the binding activity of anti-human VEGF receptor Flt-1 human chimeric antibody KM2550 and human CDR-grafted antibodies KM8550, KM8551, KM8552, KM8553, KM8554 and KM8555 upon soluble human VEGF receptor Flt-1 7N, Flt-1 3N, Flt-1 2N, Flt-1 7N.K2 and KDR 7N.
Fig. 44 is a graph showing the activity of anti-human VEGF receptor Flt-1 human chimeric antibody KM2550 and human CDR-grafted antibodies KM8550, KM8551, KM8552, KM8553, KM8554 and KM8555 to inhibit the binding between human VEGF and human VEGF receptor Flt-1.
Fig. 45 is a graph showing amino acid sequences of the H chain variable regions of anti-human VEGF receptor Flt-1 human chimeric antibody and CDR-grafted antibody. In the drawing, KM1750mouse shows H chain variable region amino acid sequence of KM1750; KM1750HV0 shows an amino acid sequence constituted by inserting CDR of H chain variable region of KM1750 into a human framework; and KM1750HV3 shows an amino acid sequence in which some of the amino acid sequence of the framework of KM1750HV0 are substituted with the amino acids of KM1750mouse.
Fig. 46 is a graph showing amino acid sequences of L chain variable region of anti-human VEGF receptor Flt-1 human chimeric antibody and CDR-grafted antibody. In the drawing, KM1750mouse shows an amino acid sequence of L chain V region of KM1750; KM1750LV0 (I) show an amino acid sequence constituted by inserting CDR of L chain variable region of KM1750 into a human framework; and KM1750LV4 shows an amino acid sequence in which some of the amino acid sequence of the framework of KM1750LV0 (I) are substituted with the amino acids of KM1750mouse.
Fig. 47 is a graph showing results of the comparison of activities of anti-VEGF receptor Flt-1 monoclonal antibodies KM1732 and KM1750 to inhibit migration of vascular endothelial cells.

### Best Mode for Carrying Out the Invention

### Example 1

### 1. Isolation and analysis of cDNA encoding anti-human VEGF receptor Flt-1 mouse monoclonal antibody

### (1) Isolation of mRNA from hybridoma capable of producing anti-human VEGF receptor Flt-1 mouse monoclonal antibody

Using a mRNA extraction kit Fast Track manufactured by In Vitrogen, and in accordance with the manual attached to the kit, mRNA was isolated from 1×10⁸ cells of each of hybridomas capable of producing anti-human VEGF receptor Flt-1 mouse monoclonal antibodies KM1732 and KM1750 (FERM BP-5698 and FERM BP-5700; respectively) obtained in Reference Example 1.

### (2) Production of heavy chain and light chain cDNA libraries of hybridoma capable of producing anti-human VEGF receptor Flt-1 mouse monoclonal antibody

Using cDNA Synthesis Kit (manufactured by Pharmacia Biotech) and in accordance with the manual attached to the kit, cDNA having *EcoR*I-*Not*I adapter on both termini was synthesized from 5 µg of each of KM1732 and KM1750 mRNA samples obtained in 1(1) of Example 1. About 6 µg of each of the thus produced cDNA samples was dissolved in 10 µl of sterilized water and fractionated by agarose gel electrophoresis to recover about 0.1 µg of a cDNA fragment of about 1.5 kb which corresponds to the heavy chain (hereinafter referred to as "H chain") of the IgG type antibody and similar amount of a cDNA fragment of about 1.0 kb that corresponds to the light chain (hereinafter referred to as "L chain") thereof. Next, 0.1 µg of the cDNA fragment of about 1.5 kb or 0.1 µg of the cDNA fragment of about 1.0 kb and 1 µg of Lambda ZAPII Vector (a preparation obtained by digesting Lambda ZAPII Vector with *Eco*RI and then treating it with calf intestine alkaline phosphatase: manufactured by Stratagene) were dissolved in 11.5 µl of a T4 ligase buffer solution (manufactured by Takara Shuzo Co., Ltd.), and the thus prepared solution was mixed with 175 units of T4 DNA ligase (manufactured by Takara Shuzo Co., Ltd.) and incubated at 12°C for 24 hours and then at room temperature for 2 hours. Thereafter, a 4 µl portion of each of the reaction solutions was packaged in lambda phage using Gigapack Gold Packaging Kit (manufactured by Stratagene) in accordance with the conventional method (*Molecular Cloning,* 2nd edition), and *Escherichia coli* XL1-Blue [Biotechniques, 5: 376 (1987)] attached to Gigapack Gold Packaging Kit (manufactured by Stratagene) was infected with the resulting phage in accordance with the conventional method (*Molecular Cloning,* 2nd edition) to obtain about 4×10³ phage clones as each of the H chain cDNA libraries and L chain cDNA libraries of KM1732 and KM1750.

### (3) Cloning of cDNA encoding H chain and L chain of hybridoma capable of producing anti-human VEGF receptor Flt-1 mouse monoclonal antibody

Respective phage particles produced in 1(2) of Example 1 were fixed on nitrocellulose filters in accordance with the conventional method (*Molecular Cloning,* 2nd edition). Each of the resulting nitrocellulose filters was treated in accordance with the manual attached to ECL direct nucleic acid labeling and detection systems (manufactured by Amersham) to obtain phage clones which strongly bonded to a probe cDNA encoding the constant region (hereinafter referred to as "C region") of mouse immunoglobulin [H chain is a fragment of mouse Cγl cDNA [Cell, 18: 559 (1979)] and L chain is a fragment of mouse Cκ cDNA [Cell, 22: 197 (1980)]]. Thereafter, the phage clones were transformed into plasmid pBluescript SK(-) in accordance with the manual attached to Lambda ZAPII Vector (manufactured by Stratagene), thus finally obtaining a recombinant plasmid KM1732HA2 containing cDNA encoding the H chain of KM1732, a recombinant plasmid KM1732L2-1 containing cDNA encoding L chain of KM1732, a recombinant plasmid KM1750H2-1 containing cDNA encoding the H chain of KM1750 and a recombinant plasmid KM1750L3-1 containing cDNA encoding L chain of KM1750. *Escherichia coli* XL1-Blue MRF'/KM1732HA2 containing the recombinant plasmid KM1732HA2, *Escherichia coli* XL1-Blue MRF'/KM1732L2-1 containing the recombinant plasmid KM1732L2-1, Escherichia coli XL1-Blue MRF' /KM1750H2-1 containing the recombinant plasmid KM1750H2-1 and *Escherichia coli* XL1-Blue MRF'/KM1750L3-1 containing the recombinant plasmid KM1750L3-1 have been deposited on May 14, 1998, in National Institute of Bioscience and Human Technology, Agency of Industrial Science and. Technology (Higashi 1-1-3, Tsukuba-shi, Ibaraki, Japan) as FERM BP-6354, FERM BP-6352, FERM BP-6353 and FERM BP-6355, respectively.

### (4) Determination of variable region nucleotide sequence of cDNA encoding H chain and L chain of anti-human VEGF receptor Flt-1 mouse monoclonal antibody

Variable region (hereinafter referred to as "V region") nucleotide sequence of each of the cDNA samples obtained in 1(3) of Example 1 encoding H chain and L chain of anti-human VEGF receptor Flt-1 mouse monoclonal antibody was determined by reacting 0.1 µg of each of the obtained plasmid samples in accordance with the manual attached to BigDye Terminator Cycle Sequencing Ready Reaction Kit (manufactured by Applied Biosystems) and then carrying out electrophoresis using ABI PRISM^{™} 377 (manufactured by Applied Biosystems). Based on the thus determined nucleotide sequence of each cDNA, amino acid sequences of the V region of H chain (hereinafter referred to as "VH") and the V region of L chain (hereinafter referred to as "VL") of KM1732 and KM1750 were determined. The nucleotide sequences of the VH of KM1732, the VL of KM1732, the VH of KM1750 and the VL of KM1750 are shown in SEQ ID NOS:1, 2, 3 and 4, respectively. The amino acid sequences of the VH of KM1732, the VL of KM1732, the VH of KM1750 and of the VL of KM1750 are shown in SEQ ID NOS:86, 87, 88 and 89, respectively.

### (5) Identification of CDR sequences of H chain and L chain of anti-human VEGF receptor Flt-1 mouse monoclonal antibody

Based on the amino acid sequences of the VH and VL of anti-human VEGF receptor Flt-1 mouse monoclonal antibody determined in 1(4) of Example 1, CDR sequences of respective VH and VL were identified by comparing them with known antibody V region amino acid sequences (Sequences of Proteins of Immunological Interest). The amino acid sequences of CDR 1, 2 and 3 of the VH of KM1732 are shown in SEQ ID NOS:5, 6 and 7, respectively; those of CDR 1, 2 and 3 of the VL of KM1732 are shown in SEQ ID NOS:B, 9 and 10, respectively; those of CDR 1, 2 and 3 of the VH of KM1750 are shown in SEQ ID NOS:11, 12 and 13, respectively; and those of CDR 1, 2 and 3 of the VL of KM1750 are shown in SEQ ID NOS:14, 15 and 16, respectively.

### 2. Production of anti-human VEGF receptor Flt-1 human chimeric antibodies

Anti-human VEGF receptor Flt-1 human chimeric antibodies KM2532 and KM2550 originated from the anti-human VEGF receptor Flt-1 mouse monoclonal antibodies KM1732 and KM1750 having neutralization activity for human VEGF receptor Flt-1 were produced in the following manner.

### (1) Construction of expression vector pKANTEX1732 of anti-human VEGF receptor Flt-1 human chimeric antibody

The expression vector pKANTEX1732 of anti-human VEGF receptor Flt-1 human chimeric antibody was constructed in the following manner using a tandem cassette vector pKANTEX93 for humanized antibody expression use described in WO 97/10354 and the plasmids KM1732HA2 and KM1732L2-1 obtained in 1 of Example 1.

A 3 µg portion of plasmid pBluescript SK(-) (manufactured by Stratagene) was added to 10 µl of a buffer solution comprising 10 mM Tris-HCl (pH 7.5), 10 mM magnesium chloride and 1 mM DTT, 10 units of a restriction enzyme *Apa*I (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml bovine serum albumin (hereinafter referred to as "BSA") and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 2 µg of an *Apa*I-*Not*I fragment of about 2.95 kb. Next, 5 µg of the plasmid KM1732HA2 was added to 10 µl of a buffer solution comprising 20 mM Tris-HCl (pH 7.9), 10 mM magnesium acetate, 50 mM potassium acetate, 1 mM DTT and 100 µg/ml BSA, 10 units of a restriction enzyme *Nla*IV (manufactured by New England Biolabs) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Nla*IV-*Not*I fragment of about 0.41 kb. Next, a synthetic DNA having the nucleotide sequence of SEQ ID NO:17 or 18 was synthesized (manufactured by Sawady Technology) , and a 0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution [500 mM Tris-HCl (pH 7.6), 100 mM magnesium chloride and 50 mM DTT] and 2 µl of 10 mM ATP and then with 10 units of T4 polynucleotide kinase (manufactured by Takara Shuzo Co., Ltd.), and the mixture was allowed to react at 37°C for 30 minutes to phosphorylating the 5' end.

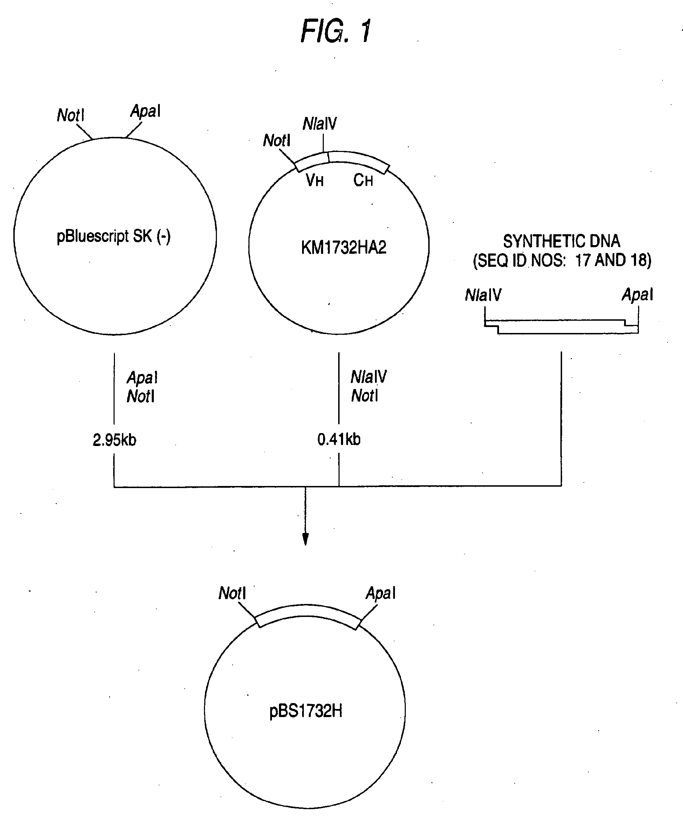

A 0.1 µg portion of the *Apa*I-*Not*I fragment derived from plasmid pBluescript SK(-) , 0.1 µg of the *Nla*IV-*Not*I fragment derived from plasmid KM1732HA2 and 0.05 µg of the phosphorylated synthetic DNA, obtained by the just described procedures, were added to 10 µl in total volume of sterilized water and ligated using DNA Ligation Kit.Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pBS1732H shown in Fig. 1.

Next, 3 µg portion of plasmid phKM1259LV0 described in WO 97/10354 was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *EcoR*I (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 2 µg of an *Eco*RI-*Spl*I fragment of about 2.95 kb. Next, 5 µg of the plasmid KM1732L2-1 was added to 10 µl of a buffer solution comprising 10 mM Tris-HCl (pH 7.5), 100 mM magnesium chloride and 1 mM DTT, 10 units of a restriction enzyme *Mbo*II (manufactured by TOYOBO CO., LTD.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride and 1 mM DTT, 10 units of a restriction enzyme *Eco*RI (manufactured by Takara Shuzo Co., Ltd.) were further added thereto, and then the reaction was carried out at 37°C for 1 hour. The reaction solution was fractionated by agarose gel electrophoresis to recover about 0.5 µg of a *Mbo*II-*Eco*RI fragment of about 0.38 kb. Next, a synthetic DNA having the nucleotide sequence of SEQ ID NO:19 or 20 was synthesized (manufactured by Sawady Technology), and a 0.3 µg portion of each synthetic DNA was added to 15 µl of sterilized water and heated at 65°c for 5 minutes. The reaction solution was allowed to stand at room temperature for 30 minutes, mixed with 2 µl of a 10-fold buffer solution [500 mM Tris-HCl (pH 7.6), 100 mM magnesium chloride and 50 mM DTT] and 2 µl of 10 mM ATP and then with 10 units of T4 polynucleotide kinase (manufactured by Takara Shuzo Co., Ltd.), and the mixture was allowed to react at 37°C for 30 minutes to phosphorylate the 5' end.

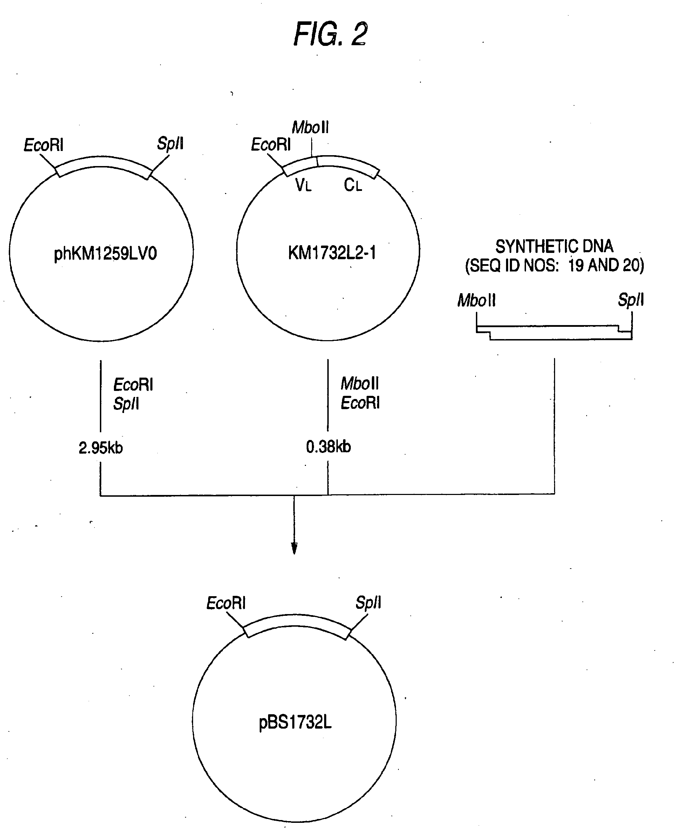

A 0.1 µg portion of the *Eco*RI-*Spl*I fragment derived from plasmid phKM1259LV0, 0.1 µg of the *Mbo*II-*Eco*RI fragment derived from plasmid KM1732L2-1 and 0.05 µg of the phosphorylated synthetic DNA, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO. CO., LTD.) was transformed with the thus obtained recombinant plasmid DNA solution to obtain a plasmid pBS1732L shown in Fig. 2.

Next, 3 µg of the vector pKANTEX93 for humanized antibody expression was added to 10 µl of a buffer solution comprising 10 mM Tris-HCl (pH 7.5), 10 mM magnesium chloride and 1 mM DTT, 10 units of a restriction enzyme *Apa*I (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 1 µg of an *Apa*I-*Not*I fragment of about 12.75 kb. Next, 5 µg of the plasmid pBS1732H obtained in the foregoing was added to 10 µl of a buffer solution comprising 10 mM Tris-HCl (pH 7.5), 10 mM magnesium chloride and 1 mM DTT, 10 units of a restriction enzyme *Apa*I (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Apa*I-*Not*I fragment of about 0.46 kb.

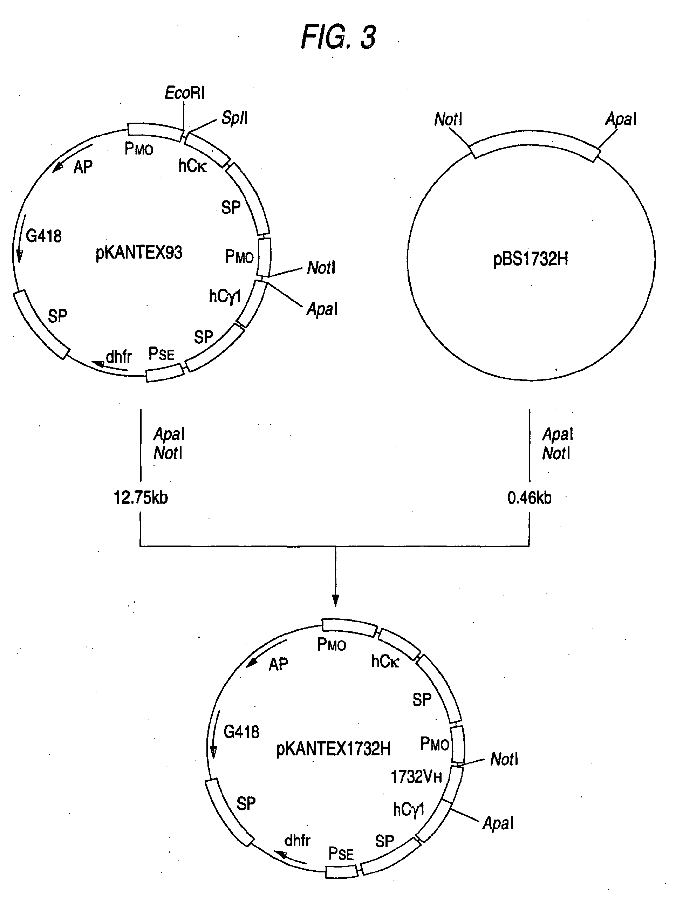

A 0.1 µg portion of the *Apa*I-*Not*I fragment derived from the vector pKANTEX93 for humanized antibody, expression and 0.1 µg of the *Apa*I-*Not*I fragment derived from the plasmid pBS1732H, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1732H shown in Fig. 3.

Next, 3 µg of the just obtained plasmid pKANTEX1732H was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) which were further added thereto, and then the reaction was carried out at 37°C for 1 hour. The reaction solution was fractionated by agarose gel electrophoresis to recover about 1 µg of an *Eco*RI-*Spl*I fragment of about 13.20 kb. Next, 5 µg of the plasmid pBS1732L obtained in the foregoing was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Eco*RI-*Spl*I fragment of about 0.39 kb.

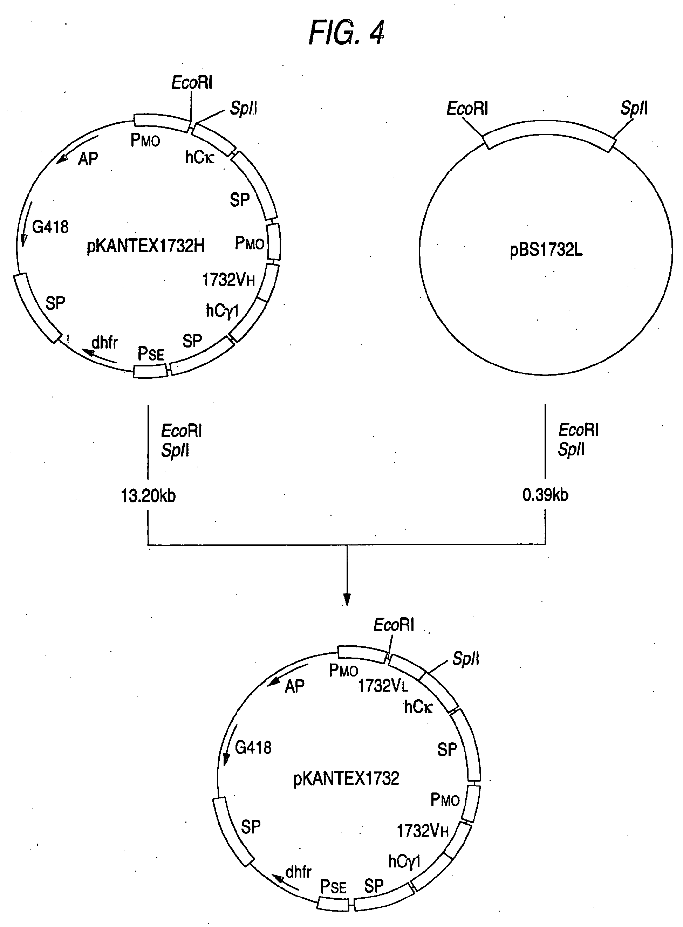

A 0.1 µg portion of the *Eco*RI-*Spl*I fragment derived from the plasmid pKANTEX1732H and 0.1 µg of the *Eco*RI-*Spl*I fragment derived from the plasmid pBS1732L, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1732 shown in Fig. 4.

### (2) Construction of expression vector pKANTEX1750 of anti-human VEGF receptor Flt-1 human chimeric antibody

The expression vector pKANTEX1750 of anti-human VEGF receptor Flt-1 human chimeric antibody was constructed in the following manner using a tandem cassette vector pFtANTEX93 for humanized antibody expression described in WO 97/10354 and the plasmids KM1750H2-1 and KM1750L3-1 obtained in 1 of Example 1,

A 5 µg portion of the plasmid KM1750H2-1 was added to 10 µl of a buffer solution comprising 33 mM Tris-HCl (pH 7.9), 10 mM magnesium acetate, 66 mM potassium acetate and 100 µg/ml BSA, 10 units of a restriction enzyme *Alw*26I (manufactured by New England Biolabs) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Alw*26I*-Not*I fragment of about 0.41 kb. Next, a synthetic DNA having the nucleotide sequence of SEQ ID NO:21 or 22 was synthesized (manufactured by Sawady Technology), and a.0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution [500 mM Tris-HCl (pH 7.6), 100 mM magnesium chloride and 50 mM DTT] and 2 µl of 10 mM ATP and then with 10 units of T4 polynucleotide kinase (manufactured by Takara Shuzo Co., Ltd.), and the mixture was allowed to react at 37°C for 30 minutes to phosphorylate the 5' end.

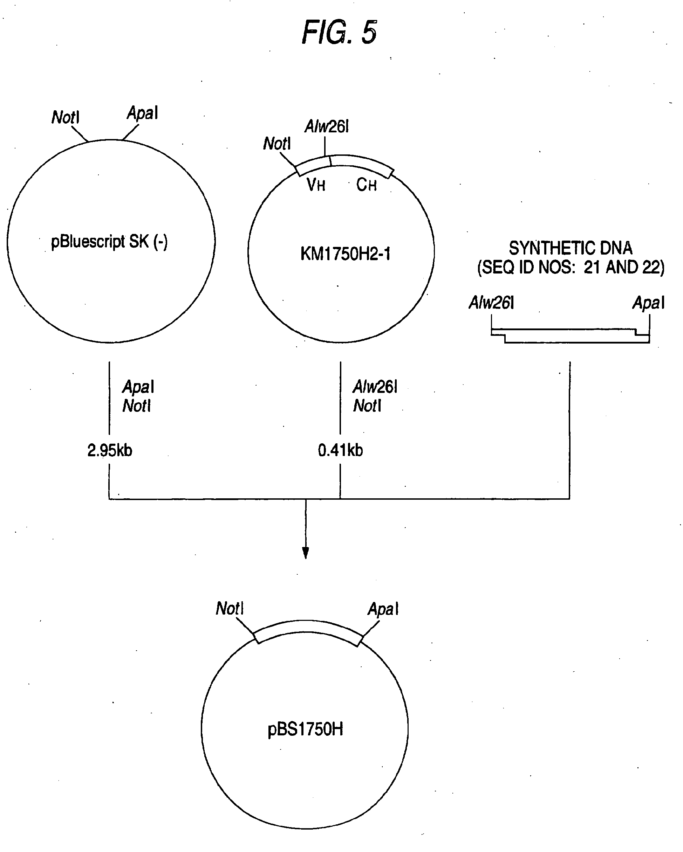

A 0.1 µg portion of the *Apa*I-*Not*I fragment derived from plasmid pBluescript SK(-) in 2 (1) of Example 1, 0.1 µg of the *Alw*26I-*Not*I fragment derived from plasmid KM1750H2-1 and 0.05 µg of the phosphorylated synthetic DNA were added to 10 µl in total volume of sterilized water and ligated using DNA Ligation Kit Ver. 2 (manufactured by Takara Shuzo Co., Ltd.) in accordance with the manual attached thereto. *Escherichia* coli DH5α (manufactured by TOYOBO CO., LTD.) was transformed with the thus obtained recombinant plasmid DNA solution to obtain a plasmid pBS1750H shown in Fig. 5.

Next, 5 µg of the plasmid KM1750L3-1 was added to 10 µl of a buffer solution comprising 100 mM Tris-HCl (pH 8.8) , 440 mM sodium chloride, 12 mM magnesium chloride, 14 mM 2-mercaptoethanol and 200 µg/ml BSA, 10 units of a restriction enzyme *Mae*II (manufactured by Boehringer-Mannheim) were further added thereto, and then the reaction was carried out at 50°C for 1 hour.. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride and 1 mM DTT, 10 units of a restriction enzyme *Eco*RI (manufactured by Takara Shuzo Co., Ltd.) were further added thereto, and then the reaction was carried out at 37°C for 1 hour. The reaction solution was fractionated by agarose gel electrophoresis to recover about 0.5 µg of a *Mae*II-*Eco*RI fragment of about 0.38 kb. Next, a synthetic DNA having the nucleotide sequence of SEQ ID NO:23 or 24 was synthesized (manufactured by Sawady Technology), and a 0.3 µg portion of each synthetic DNA was added to 15 µl of sterilized water and heated at 65°C for 5 minutes. The reaction solution was allowed to stand at room temperature for 30 minutes, mixed with 2 µl of a 10-fold buffer solution [500 mM Tris-HCl (pH 7.6), 100 mM magnesium chloride and 50 mM DTT] and 2 µl of 10 mM ATP and then with 10 units of T4 polynucleotide kinase (manufactured by Takara Shuzo Co., Ltd.), and the mixture was allowed to react at 37°C for 30 minutes to phosphorylate the 5' end.

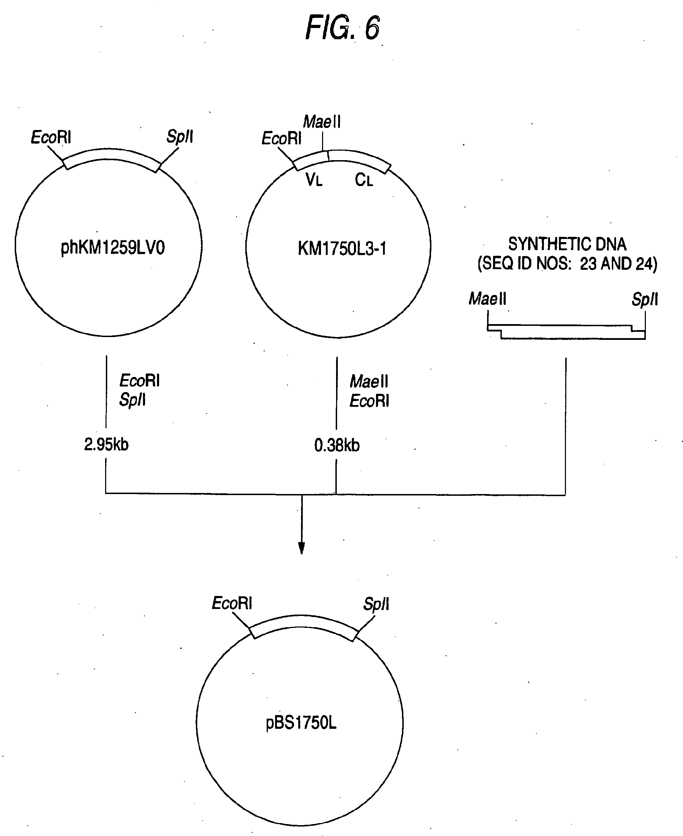

A 0.1 µg portion of the *Eco*RI-*Spl*I fragment derived from plasmid pphKM1259LV0 in 2(1) of Example 1, 0.1 µg of the *Mae*II-*Eco*RI fragment derived from plasmid KM1750L3-1 and 0.05 µg of the phosphorylated synthetic DNA were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pBS1750L shown in Fig. 6.

Next, 5 µg of the plasmid pBS1750H obtained in the foregoing was added to 10 µl of a buffer solution comprising 10 mM Tris-HCl (pH 7.5), 10 mM magnesium chloride and 1 mM DTT, 10 units of a restriction enzyme *Apa*I (manufactured by Takara Shuzo Co., Ltd.) were further added thereto, and then the reaction was carried out at 37°C for 1 hour. The reaction solution was precipitated with ethanol, the thus obtained precipitate was added to 10 µl of a buffer solution comprising 50 mM Tris-HCl (pH 7.5), 100 mM sodium chloride, 10 mM magnesium chloride, 1 mM DTT, 100 µg/ml BSA and 0.01% Triton X-100, 10 units of a restriction enzyme *Not*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Apa*I-*Not*I fragment of about 0.46 kb.

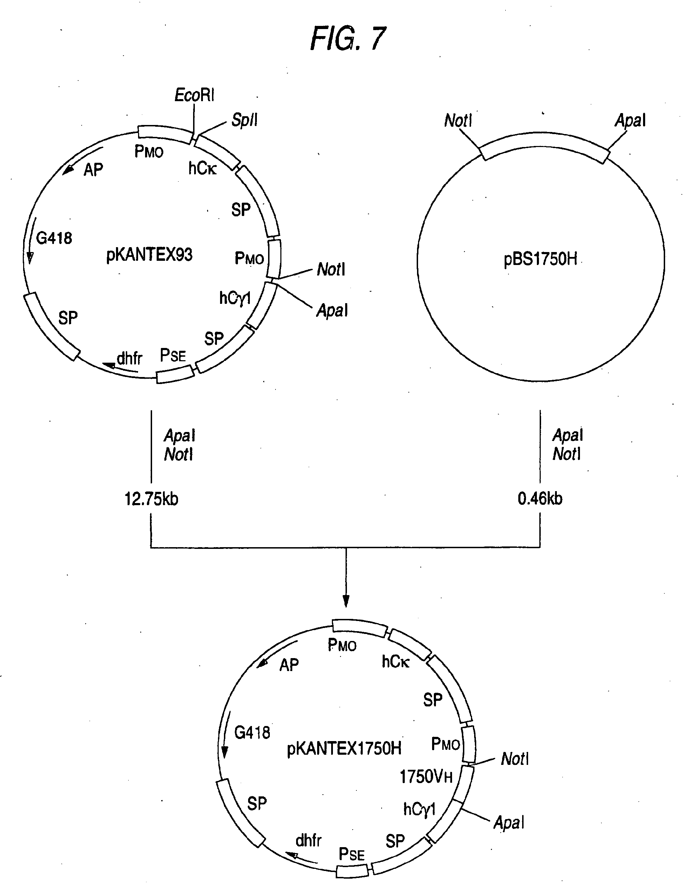

A 0.1 µg portion of the *Apa*I-*Not*I fragment derived in 2(1) of Example 1 from a tandem cassette vector pKANTEX93 for humanized antibody expression and 0.1 µg of the *Apa*I-*Not*I fragment derived from plasmid pBS1750H were added to 10 µl in total volume of sterilized water and ligated using DNA Ligation Kit Very 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1750H shown in Fig. 7.

Next, 3 µg of the just obtained plasmid pKANTEX1750H was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 1 µg of an *Eco*RI-*Spl*I fragment of about 13.20 kb. Next, 5 µg of the plasmid pBS1750L obtained in the foregoing was added to 10 µl of a buffer solution comprising 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were further added thereto, and then the reaction was carried out at 37°C for 1 hour. The reaction solution was fractionated by agarose gel electrophoresis to recover about 0.5 µg of an *Eco*RI-*Spl*I fragment of about 0.39 kb.

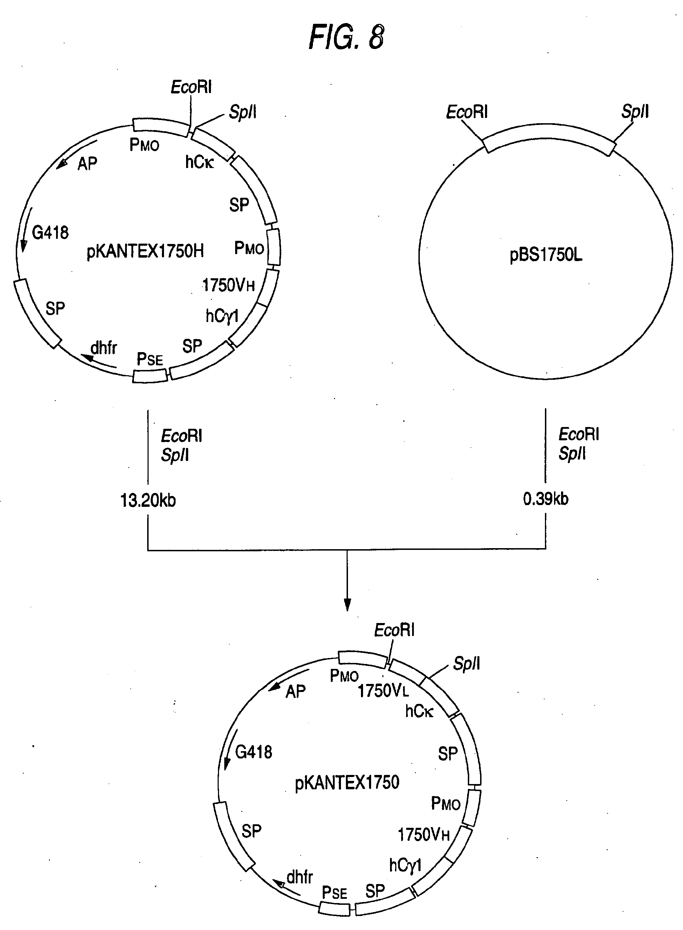

A 0.1 µg portion of the *Eco*RI-*Spl*I fragment derived from plasmid pKANTEX1750H and 0.1 µg of the. *Eco*RI-*Spl*I fragment derived from plasmid pBS1750L, obtained by the just described procedures, were added to 10 µl in total volume of sterilized water and ligated using DNA Ligation Kit Ver. 2 (manufactured by Takara Shuzo Co., Ltd.) in accordance with the manual attached thereto. *Escherichia coli* DH5α (manufactured by TOYOBO CO., LTD.) was transformed with the thus obtained recombinant plasmid DNA solution to obtain a plasmid pKANTEX1750 shown in Fig. 8.

### (3) Expression of anti-human VEGF receptor Flt-1 human chimeric antibodies in rat myeloma YB2/0 cells (ATCC CRL1581) using pKANTEX1732 and pKANTEX1750

Introduction of anti-human VEGF receptor Flt-1 human chimeric antibody expression vectors pKANTEX1732 and pKANTEX1750 into YB2/0 cells was carried out by electroporation in accordance with the method of Miyaji et al. [Cytotechnology, 3: 133 (1990)].

A 5 µg portion of each of pKANTEX1732 and pKANTEX1750 obtained in 2(1) and 2(2) of Example 1 was introduced into 4x10⁶ YB2/0 cells which were subsequently suspended in 40 ml of RPMI 1640-FCS(10) medium [RPMI 1640 medium (manufactured by Nissui Pharmaceutical) containing 10% fetal calf serum (FCS) ] and dispensed in 200 µl portions into wells of a 96 well microtiter plate (manufactured by Sumilon). After 24 hours of culturing at 37°C in a 5% CO₂ incubator, geneticin (hereinafter referred to as "G 418", manufactured by Gibco) was added to a final concentration of 0.5 mg/ml, and the culturing was continued for 1 to 2 weeks. When colonies of G 418-resistant transformants were formed and became confluent, the culture supernatant was recovered from each well, and the activity of anti-human VEGF receptor Flt-1 human chimeric antibody in the supernatant was measured by the following enzyme immunoassay.

### Enzyme immunoassay

Soluble human VEGF receptor Flt-1 7N was prepared in accordance with the method of Tanaka et al. [Japanese Journal of Cancer Research, 88: 867 (1997)]. The soluble human VEGF receptor Flt-1 7N diluted to 1 µg/ml with PBS was dispensed in 50 µl portions into wells of a 96 well plate for EIA (manufactured by Greiner) and allowed to stand overnight at 4°C for its adsorption. After washing with PBS, 100 µl of PBS containing 1% BSA (hereinafter referred to as "1% BSA-PBS") was added to each well, and the remaining active residues was blocked by carrying out the reaction at room temperature for 1 hour. After discarding 1% BSA-PBS, the culture supernatant of each transformant was dispensed in 50 µl portions into the resulting wells to carry out the reaction at room temperature for 1 hour. After washing the plate with PBS containing 0.05% Tween 20 (hereinafter referred to as "0.05% Tween-PBS"), a peroxidase-labeled anti-human IgG antibody (manufactured by American Quarex) which had been diluted 3,000 times with 1% BSA-PBS was dispensed in 50 µl portions into the wells to carry out the reaction at room temperature for 1 hour, the resulting plate was washed with 0.5% Tween-PBS, and then an ABTS [ammonium 2,2-azinobis(3-ethylbenzothiazole-6-sulfonate)] substrate solution was dispensed in 50 µl portions into the wells to develop color and measure its absorbance at OD415nm using Emax (manufactured by Molecular Devices).

In order to increase productivity, each of the transformants which showed the anti-human VEGF receptor Flt-1 human chimeric antibody activity in the culture supernatant was suspended in RPMI 1640-FCS(10) medium supplemented with 0.5 mg/ml of G 418 and 50 nM of methotrexate (hereinafter referred to as "MTX", manufactured by Sigma) and cultured at 37°C for 1 to 2 weeks in a 5% CO₂ incubator to induce transformants having 50 nM MTX resistance. When the transformants became confluent in the wells, the anti-human VEGF receptor Flt-1 human chimeric antibody activity in the culture supernatants was measured by the aforementioned enzyme immunoassay. Transformants in which the activity was found were cultured similarly by further increasing the MTX concentration to 100 nM and then to 200 nM to obtain transformants which can grow in RPMI 1640-FCS(10) medium containing 0.5 mg/ml of G 418 and 200 nM of MTX and are able to produce large amounts of anti-human VEGF receptor Flt-1 human chimeric antibodies. The thus obtained transformants were subjected to two times of cloning by limiting dilution to obtain final transformant cell lines capable of producing anti-human VEGF receptor Flt-1 human chimeric antibodies. KM2532 can be cited as an example of the transformant cell line obtained by introducing the expression vector pKANTEX1732, namely a transformant which produces an anti-human VEGF receptor Flt-1 human chimeric antibody originated from the anti-human VEGF receptor Flt-1 mouse monoclonal antibody KM1732, and the anti-human VEGF receptor Flt-1 human chimeric antibody produced by this transformant was named KM2532. Also, KM2550 can be cited as an example of the transformant cell line obtained by introducing the expression vector pKANTEX1750, namely a transformant which produces an anti-human VEGF receptor Flt-1 human chimeric antibody originated from the anti-human VEGF receptor Flt-1 mouse monoclonal antibody KM1750, and the anti-human VEGF receptor Flt-1 human chimeric antibody produced by this transformant was named KM2550. The productivity of anti-human VEGF receptor Flt-1 human chimeric antibodies by the thus cloned transformants was found to be about 5 µg/10⁶ cells/24 hours.

### (4) Purification of anti-human VEGF receptor Flt-1 human chimeric antibodies from culture supernatants

Each of the anti-human VEGF receptor Flt-1 human chimeric antibody producing cell lines KM2532 and KM2550 obtained in 2(3) of.Example 1 was suspended in GIT medium (manufactured by Japan Pharmaceutical) supplemented with 0.5 mg/ml of G 418 and 200 nM of MTX, to a density of 1×10⁵ to 2×10⁵ cells/ml, and dispensed in 200 ml portions into a total of five 175 cm² flasks (manufactured by Greiner). When the cells became confluent after 5 to 7 days of culturing at 37°C in a 5% CO₂ incubator, about 1.0 liter of each culture supernatant was recovered. A column was packed with about 1 ml of ProSep A (manufactured by Bioprocessing) and washed with 10 ml of 1 M glycine-0.15 M NaCl (pH 8.6) at a flow rate of 1 ml/minute. After the washing, 1,700 ml or 1,800 ml of the culture supernatant prepared in the above containing the anti-human VEGF receptor Flt-1 human chimeric antibody KM2532 or KM2550 was passed through the ProSep A column at a flow rate of 70 ml/hour. This was further washed with 10 ml of 1 M glycine-0.15 M NaCl (pH 8.6) at a flow rate of 1 ml/minute, again washed stepwise with 4 ml of 50 mM citrate buffer of pH 6, 5 and 4, and then 7 ml of 50 mM citrate buffer (pH 3.0) was passed through the column to elute the human chimeric antibody. As a result, 0.4 mg and 0.3 mg of the anti-human VEGF receptor Flt-1 human chimeric antibodies KM2532 and KM2550, respectively, were obtained.

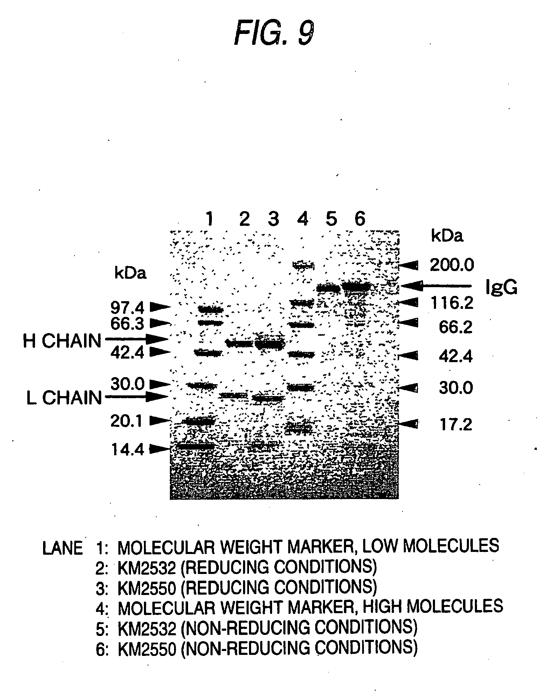

The thus purified anti-human VEGF receptor Flt-1 human chimeric antibodies KM2532 and KM2550 were analyzed by SDS-PAGE in accordance with a known method [Anticancer Research, 12: 1121 (1992)]. As the gel, 5 to 20% gradient gel (manufactured by Atto Corp.) was used, a low molecular weight protein molecular marker "Daiichi" II and a high molecular weight protein molecular marker "Daiichi" III (both manufactured by Daiichi Pure Chemicals) were used as molecular markers, and 2.5 µg as protein per lane of each of the anti-human VEGF receptor Flt-1 human chimeric antibodies KM2532 and KM2550 was electrophoresed under reducing and non-reducing conditions and stained with Coomassie Brilliant Blue. The results are shown in Fig. 9. The human chimeric antibodies KM2532 and KM2550 showed a band of IgG at a position corresponding to a molecular weight of about 150 kDa under the non-reducing condition, and a band of H chain at about 50 kDa and a band of L chain at about 25 kDa were found under the reducing condition. This result coincided with that the IgG type antibody is separated into two H chains and L chains under the reducing conditions caused by the cutting of intermolecular disulfide bond and present as a molecule of 150 kDa, and it is shown that the human chimeric antibodies KM2532 and 2550 are antibody molecules having a correct structure.

### (5) Binding activity of anti-human VEGF receptor Flt-1 human chimeric antibodies upon human VEGF receptor Flt-1

Binding activity of the purified anti-human VEGF receptor Flt-1 human chimeric antibodies KM2532 and KM2550 upon human VEGF receptor Flt-1 was confirmed by the following procedure.

Soluble human VEGF receptor Flt-1 7N was prepared in accordance with the method of Tanaka et al. [Japanese Journal of Cancer Research, 88: 867 (1997)].

First, the binding activity was examined using an enzyme immunoassay by fixing amount of the soluble human VEGF receptor Flt-1 7N to be adsorbed on a 96 well plate for EIA and varying concentration of the human chimeric antibody to be added. The soluble human VEGF receptor Flt-1 7N diluted to 1 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of from 0.152 to 333 ng/ml was dispensed in 50 µl portions into the resulting wells to carry out the reaction at room temperature for 1 hour. After washing the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.5% Tween-PBS, and then an ABTS [ammonium 2,2-azinobis(3-ethylbenzothiazole-6-sulfonate)] substrate solution was dispensed in 50 µl portions into the wells to develop color and measure its absorbance at OD415nm using Emax (manufactured by Molecular Devices).

The results are shown in Fig. 10 (A). The anti-human VEGF receptor Flt-1 human chimeric antibodies KM2532. and KM2550 bound to the human VEGF receptor Flt-1 7N in an antibody concentration dependent manner. In addition, the anti-human VEGF receptor Fit-1 human chimeric antibodies KM2532 and KM2550 showed almost the same binding activity.

Next, the binding activity of human chimeric antibodies was examined using an enzyme immunoassay by varying amount of the soluble human Flt-1 7N to be adsorbed on a 96 well plate for EIA. The soluble human VEGF receptor Flt-1 7N diluted to a concentration of from 0.04 to 10 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of 10 µg/ml was dispensed in 50 µl portions into the resulting wells to carry out the reaction at room temperature for 1 hour. After washing the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.5% Tween-PBS, and then an ABTS [ammonium ,2,2-azinobis(3-ethylbenzothiazole-6-sulfonate)] substrate solution was dispensed in 50 µl portions into the wells to develop color and measure its absorbance at OD415nm using Emax (manufactured by Molecular Devices).

The results are shown in Fig. 10 (B). Each of the anti-human VEGF receptor Fit-1 human chimeric antibodies KM2532 and KM2550 showed its binding activity dependently upon the concentration of the soluble human VEGF receptor Flt-1 7N adsorbed on the plate. In addition, the anti-human VEGF receptor Flt-1 human chimeric antibodies KM2532 and KM2550 showed almost the same binding activity.

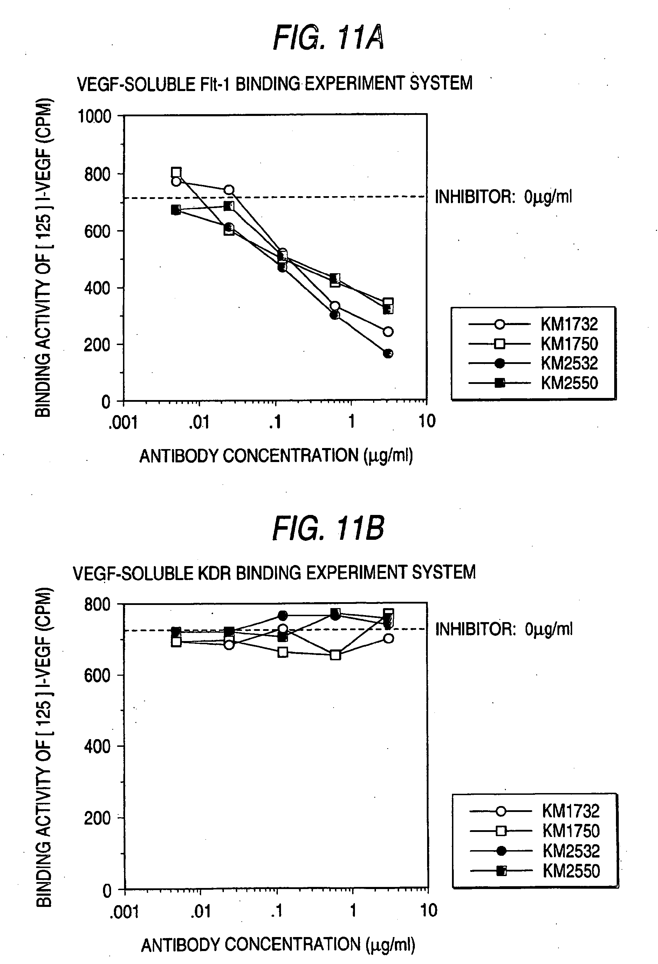

Next, the activity of anti-human VEGF receptor Flt-1 human chimeric antibodies to inhibit binding of human VEGF with human VEGF receptor Flt-1 was examined in the following manner. Methanol was dispensed in 100 µl portions into wells of a 96 well multi-screen IP plate (manufactured by Millipore) to give hydrophilic nature to the PVDF membrane on the bottom of the plate. After washing the plate with water, the soluble human VEGF receptor Flt-1 7N which had been diluted to 0.2 µg/ml with PBS was dispensed in 50 µl portions into the wells and allowed to stand overnight at 4°C for its adsorption. After washing the plate with PBS, 50 µl of 1% BSA-PBS was added to each well, and the remaining active residues was blocked by carrying out the reaction at room temperature for 1 hour. After washing the plate with PBS, each of the purified anti-human VEGF receptor Flt-1 human chimeric antibodies KM2532 and KM2550, and purified anti-human VEGF receptor Flt-1 mouse monoclonal antibodies KM1732 and KM1750, which had been diluted with 1% BSA-PBS to a concentration of from 0.004 to 2 µg/ml, was dispensed in 50 µl portions into the resulting wells and then 3 ng/ml of ¹²⁵I-labeled human VEGF (manufactured by Amersham) was dispensed in 50 µl portions into the wells to carry out the reaction at room temperature for 1.5 hours. After washing the plate with 0.05% Tween-PBS, the wells were dried at 50°C, Microscinti-O (manufactured by Packard) was dispensed in 30 µl portions into the wells and then radioactivity of the ¹²⁵I-labeled human VEGF bonded on each well was measured using Top Count (manufactured by Packard). The results are shown in Fig. 11. As shown in Fig. 11, each of the anti-human VEGF receptor Flt-1 human chimeric antibodies KM2532 and KM2550 inhibited binding of the human VEGF with the human VEGF receptor Flt-1 in an antibody concentration dependent manner. In addition, the activity of the anti-human VEGF receptor Flt-1 human chimeric antibodies, KM2532 and KM2550 to inhibit binding of the human VEGF with the human VEGF receptor Flt-1 was almost the same as that of the anti-human VEGF receptor Flt-1 mouse monoclonal antibodies KM1732 and KM1750, thus showing that the human chimeric antibodies maintain the activity of mouse monoclonal antibodies.

### (6) Analysis of epitope which is recognized by anti-human VEGF receptor Flt-1 human chimeric antibody

### (6-1) Preparation of soluble human VEGF receptor KDR 7N and soluble human VEGF receptor chimeric protein Flt-1 7N.K2

Soluble human VEGF receptor KDR 7N and soluble human VEGF receptor chimeric protein Flt-1 7N.K2 were prepared in the following manner.

### (6-2) Construction of soluble human VEGF receptor KDR 7N expression vector

A vector was produced in the following manner for the expression of 19 amino acids which constitute the signal peptide of human VEGF receptor KDR, a soluble human VEGF receptor KDR fragment (hereinafter referred to as "soluble human VEGF receptor KDR 7N") which corresponds to 738th position from the N-terminal amino acid of the mature protein and two amino acid residues derived from a linker.

The soluble human VEGF receptor KDR 7N consists of the seven immunoglobulin-like moieties from the N-terminal side of the extracellular region of soluble human VEGF receptor KDR.

A cDNA clone which encodes full length cDNA of human VEGF receptor KDR, BCMGS-neo-KDR [A. Sawano et al., Cell Growth & Differentiation, 7: 213-221 (1996)], was digested with *Eco*RI, and a fragment of about 2.8 kb encoding the extracellular region and membrane-binding region of KDR was integrated into the *Eco*RI site of pUC18 to produce pUC-KDR. The pUC-KDR was digested with *Xho*I and treated with Klenow and then an *Xba*I linker (SEQ ID NO:57) was inserted to produce pUC-KDR-Xb. An *Xba*I-*Bam*HI fragment (2.3 kbp) of pUC-KDR-Xb was inserted into the *Xba*I/*Bam*HI site of pBluescriptII KS(+) to prepare an *Sph*I-*Bam*HI fragment (5.2 kbp) into which were subsequently inserted synthetic linkers (SEQ ID NOS:58 and 59) containing SnaBI site to produce pBS-KDR-Xb-S.

The thus obtained pBS-KDR-Xb-S was digested with *SnaB*I/*Bam*HI, and synthetic linkers (SEQ ID NOS:60 and 61) containing terminal codon and *Not*I site were inserted to produce pBS-KDR-Xb-S-N. An *Xba*I-*Not*I fragment (2.3 kb) of pBS-KDR(Xb)-S-N was inserted into downstream 5' side *Xba*I and 3' side *Not*I site of the transcription initiation point of polyhedrin gene of baculovirus recombinant plasmid pVL1393 to obtain soluble human VEGF receptor KDR 7N expression vector pVL-KDR-7N.

### (6-3) Construction of soluble human VEGF receptor chimeric protein Flt-1 7N.K2 expression vector

A vector was constructed in the following manner for the expression of a chimeric protein Flt-1.K2 in which an amino acid sequence of from the 100th amino acid to the 204th amino acid which corresponds to the second immunoglobulin-like moiety counting from the N-terminal side of the soluble human VEGF receptor Flt-1 7N that corresponds to 750 amino acids from the N-terminal containing the signal sequence of human VEGF receptor Flt-1 was replaced with an amino acid sequence of from the 95th amino acid to the 199th amino acid which correspond to the second immunoglobulin-like moiety of the soluble human VEGF receptor KDR, via linkers of two amino acids (Gly-Ala and Gly-Thr).

An *Eco*RI/*Hin*dIII fragment (1.9 kb) of human Flt-1 cDNA [Shibuya et al., Oncogene, 5:519 (1990)] was inserted into the *Eco*RI/*Hind*III site of a vector M13mp1 to produce pM13-flt. *Escherichia coli* XLlBlue was infected with the thus obtained pM13-flt and cultured, and ssDNA was prepared from the resulting culture supernatant in accordance with a manual of a site-specific mutation introduction kit Mutan K supplied by Takara Shuzo. Using an oligonucleotide of 56 bases (SEQ ID NO:62) and a site-specific mutation introducing kit Mutan K (manufactured by Takara Shuzo Co. , Ltd.), site-specific mutation was carried out using the thus obtained ssDNA as the template to produce a plasmid pM13-flt'-D2N containing a Flt-1 mutant gene in which the region encoding the second immunoglobulin-like moiety of the extracellular region of Flt-1 was deleted. Next, using 10 ng of BCMGS-neo-KDR DNA [A. Sawano et al., Cell Growth & Differentiation, 7: 213 (1996)] as the template, PCR was carried out by adding 2.5 units of TaqDNA polymerase to 100 ml of a solution containing 10 pmol of each of primers having nucleotide sequences shown in SEQ ID NOS:63 and 64, TaqDNA polymerase buffer and 10 mM deoxynucleotide triphosphate. A serial reaction of 90 seconds at 95°C, 90 seconds at 50°C and 90 seconds at 72°C was repeated 30 times, and the thus amplified DNA fragment encoding the second immunoglobulin-like moiety counting from the N-terminal side of the extracellular region of KDR was recovered. This fragment was digested with *Nar*I/*Kpn*I to obtain an *Nar*I/*Kpn*I fragment of 340 bp. The thus obtained fragment and an *Sma*I/*Nar*I fragment (0.5 kb) of pM13-flt'-D2N were inserted into the *Sma*I/*Kpn*I site of pBluescriptII to produce pBS-flt-1'-KDR2N. Thereafter, a *Bam*H/*Kpn*I fragment (0.8 kb) of pBS-flt-1'-KDR2N, a *Kpn*I/*Mun*I fragment (80 b) of pM13-flt'-D2N and an *Mun*I/*Not*I fragment (1.5 kb) of pVL-flt-1 were inserted into the *Bam*HII/*Not*I site of pVL1393 to obtain a plasmid PVL-fkf which carries the Flt-1 K2 gene.

### (6-4) Production of recombinant virus for the expression of soluble human VEGF receptor KDR 7N and soluble human VEGF receptor chimeric protein Flt-1 7N.K2 in insect cells

In order to produce a protein in insect cells, it is necessary to obtain a recombinant virus into which the gene of interest is integrated, and such a production process comprises a step in which a cDNA called transfer vector which encodes the protein of interest is inserted into a special plasmid and a subsequent step in which a wild type virus and the transfer vector are co-transfected into insect cells to obtain the recombinant virus by homologous recombination. These steps were carried out in the following manner using BaculoGold Starter Kit manufactured by Fermingen (Product No. PM-21001K) in accordance with the manual attached thereto.

Recombinant baculovirus was produced in the following manner by a lipofectin method [Protein, Nucleic Acid and Enzyme, 37: 2701 (1992)], by introducing filamentous baculovirus DNA (BaculoGold baculovirus DNA, manufactured by Fermingen) and transfer vector DNA into cells of an insect cell line Sf9 (manufactured by Fermingen) which had been cultured using TMN-FH insect medium (manufactured by Fermingen).

A 1 µg portion of the expression vector produced in (6-2) and 20 ng of the filamentous baculovirus DNA were dissolved in 12 µl of distilled water, and a mixture consisting of 6 µl of lipofectin and 6 µl of distilled water was added to the resulting solution and allowed to stand at room temperature for 15 minutes. Separately from this, 1×10⁶ of Sf9 cells were suspended in 2 ml of Sf900-II medium (manufactured by Gibco) and put into a plastic Petri dish for cell culture having a diameter of 35 mm. Entire portion of the aforementioned mixture solution- of plasmid DNA, filamentous baculovirus DNA and lipofectin was added to the dish, the cells were cultured at 27°C for 3 days and then a 1 ml portion of the culture supernatant containing recombinant virus was collected. A 1 ml portion of fresh Sf900-II medium was added to the resulting Petri dish, and the cells were further cultured at 27°C for 3 days to obtain 1.5 ml of the culture supernatant containing recombinant virus. Thereafter, the same procedure was repeated using the expression vector obtained in (6-3).

Next, in order to use it in protein expression, each of the thus obtained recombinant viruses was grown in the following manner.

A total of 2×10⁷ Sf9 cells were suspended in 10 ml of Sf900-II medium, put into a 175 cm² flask (manufactured by Greiner) and then allowed to stand at room temperature for 1 hour to adhere the cells to the flask. Thereafter, the supernatant was discarded and 15 ml of fresh TMN-FH insect medium and a 1 ml of portion of the aforementioned culture supernatant containing recombinant virus were added to the flask to carry out 3 days of culturing at 27°C. After the culturing, the supernatant was centrifuged at 1,500 × g for 10 minutes to remove cells to obtain a recombinant virus solution to be used in protein expression.

The titer of virus in the thus obtained recombinant virus solution was calculated in accordance with the method described in BaculoGold Starter Kit Manual (manufactured by Farmigen).

A total of 6×10⁶ Sf9 cells were suspended in 4 ml of Sf900-II medium, put into a plastic Petri dish for cell culture having a diameter of 60 mm and then allowed to stand at room temperature for 1 hour to adhere the cells to the dish. Next, the supernatant was discarded, 400 µl of fresh Sf900-II medium and the aforementioned recombinant virus solution diluted 1,000 times with Sf900-II medium in advance were added to the dish and allowed to stand at room temperature for 1 hour, and then the medium was discarded and 5 ml of a medium containing 1% low melting point agarose (Agarplaque Agarose, manufactured by Farmigen) (a medium produced by mixing 1 ml of sterilized 5% Agarplaque Agarose aqueous solution with 4 ml of TMN-FH insect medium and kept at 42°C) was poured into the Petri dish. After 15 minutes of standing at room temperature, the Petri dish was sealed with a vinyl tape to prevent drying and put into a sealing type plastic container to carry out 6 days of culturing at 27°C. A 1 ml portion of PBS containing 0.01% Neutral Red was added to the Petri dish, and the culturing was continued for 1 day to count the number of formed plaques. It was found by the above procedure that each of the recombinant virus solutions contained about 1×10⁷ plaque forming units (hereinafter referred to as "PFU")/ml of viral particles.

### (6-5) Expression of soluble human VEGF receptor KDR 7N and soluble human VEGF receptor chimeric protein Flt-1 7N.K2 in insect cells and their purification

The soluble human VEGF receptor KDR 7N and soluble human VEGF receptor chimeric protein Flt-1 7N.K2 were obtained in the following manner. A total of 4×10⁷ High Five cells were suspended in 30 ml of EX-CELL^{™} 400 medium (manufactured by JRH Bioscience) contained in a 175 cm² flask (manufactured by Greener) and adhered to the flask by allowing them to stand at room temperature for 1 hour. A 1 ml portion of a solution containing about 1×10⁸ to 3×10⁸ PFU/ml of the transfer vector-originated recombinant virus obtained in (6-3) and (6-4) was added to the flask to carry out infection at room temperature for 2 hours. The culture supernatant was discarded and replaced with 30 ml of fresh X-CELL^{™} 400 medium to carry out 3 to 4 days of culturing at 27°C. After completion of the culturing, the culture supernatant was collected and centrifuged at 1,500 × g for 10 minutes to recover the supernatant.

The soluble human VEGF receptor KDR 7N was purified in the following manner.

A column packed with 50 ml of DEAE-Sepharose CL-6B (manufactured by Pharmacia Biotech) and a column packed with 40 ml of Heparin Sepharose CL-6B (manufactured by Pharmacia Biotech) were connected in series, the former column on the inlet side and the latter on the outlet side, and washed with 300 ml of 20 mM sodium phosphate buffer (pH 8). After the washing, 400 to 800 ml of a culture solution containing soluble human VEGF receptor KDR was passed through the columns at a flow rate of from 50 to 100 ml/hour. The columns were again washed with 300 ml of 20 mM sodium phosphate buffer (pH 8) and then the adsorbed protein was eluted from the Heparin Sepharose CL-6B column by continuous density gradient using 400 ml of 0 to 1 M NaCl/20 mM sodium phosphate buffer. The eluate was fractionated in 7 ml portions, and 60 to 80 ml of fractions containing soluble human VEGF receptor KDR 7N were recovered by analyzing the protein contained in each fraction by SDS-PAGE. The thus recovered purification fractions were concentrated using CentriPrep 10 (manufactured by Amicon) to obtain 4.8 ml of a soluble human VEGF receptor KDR 7N solution (protein concentration and purity were 815 µg/ml and 70 to 80%, respectively).

The soluble human VEGF receptor chimeric protein Flt-1 7N.K2 was purified in the following manner.

A column was packed with about 20 ml of Heparin-Sepharose CL-6B gel (manufactured by Pharmacia Biotech AB) and washed with 100 ml of 20 mM Tris-HCl buffer (pH 7.5) at a flow rate of 0.5 ml/minute. After the washing, 900 ml of the culture solution containing soluble human VEGF receptor chimeric protein Flt-1 7N.K2 prepared in the above was passed through the Heparin-Sepharose CL-6B column at a flow rate of 0.5 ml/minute. The column was washed again with 100 ml of 20 mM Tris-HCl buffer (pH 7.5) at a flow rate of 0.5 ml/minute, and then 200 ml of 20 mM Tris-HCl buffer (pH 7.5) containing NaCl having a density gradient of from 0 to 1.5 M was passed through the column to elute the protein adsorbed to Heparin-Sepharose, while the eluate was fractionated in 8 ml portions. By analyzing the protein contained in each fraction by SDS polyacrylamide gel electrophoresis (SDS-PAGE), 50 to 70 ml of fractions containing soluble human VEGF receptor chimeric protein Flt-1 7N.K2 were recovered and concentrated using CentriPrep 10 (manufactured by Amicon). After the concentration, human VEGF receptor chimeric protein Flt-1 7N.K2 was obtained as a 5.8 ml of solution (protein concentration, 588 µg/ml; purity, 65-75%).

### (6-6) Analysis of epitope which is recognized by anti-human VEGF receptor Flt-1 human chimeric antibody

The epitope which is recognized by purified anti-human VEGF receptor Flt-1 human chimeric antibodies KM2532 and KM2550 was analyzed in the following manner.

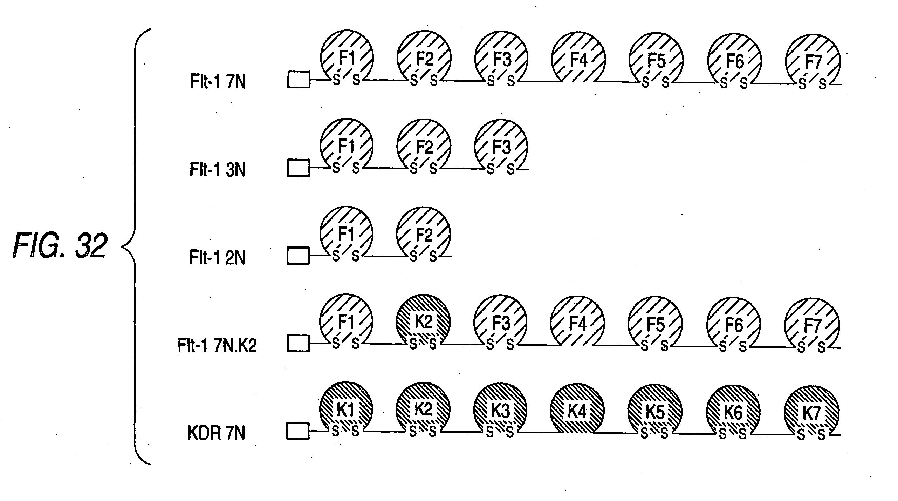

Soluble human VEGF receptor Flt-1 7N (corresponds to a sequence from the N-terminal amino acid to the 750th amino acid, including signal sequence), soluble human VEGF receptor Flt-1 3N (corresponds to a sequence from the N-terminal amino acid to the 338th amino acid, including signal sequence) and soluble human VEGF receptor Flt-1 2N (corresponds to a sequence from the N-terminal amino acid to the 223rd amino acid, including signal sequence) were prepared in accordance with the method of Tanaka et al. [Japanese Journal of Cancer Research, 88: 867 (1997)]. Soluble human VEGF receptor KDR 7N (corresponds to a sequence from the N-terminal amino acid to the 738th amino acid, does not include signal sequence) and soluble human VEGF receptor chimeric protein Flt-1 7N.K2 (100th to 204th amino acids of human Flt-1 7N are replaced with 95th to 199th amino acids of human KDR via links of two amino acids) were prepared in accordance with the method described in (6-5). Schematic illustration of each of the soluble human VEGF receptor derivatives used in the experiment is shown in Fig. 32.

First, the reactivity of anti-human VEGF receptor Plt-1 mouse monoclonal antibodies KM1732 and KM1750 and anti-human VEGF receptor Flt-1 human chimeric antibodies KM2532 and KM2550 was compared using an enzyme immunoassay. Each of Flt-1 7N, Flt-1 3N, Flt-1 2N, Flt-1 7N.K2 and KDR 7N diluted to 4 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 mouse monoclonal antibodies KM1732 or KM1750 or purified anti-human VEGF receptor Flt-1 human chimeric antibodies KM2532 or KM2550, having a concentration of from 0.1 µg/ml, was dispensed in 50 µl portions into the resulting wells to carry out the reaction at room temperature for 1 hour. After washing the plate with 0.05% Tween-PBS, a peroxidase-labeled anti-mouse IgG antibody (manufactured by Dako) which had been diluted 400 times with 1% BSA-PBS, or a peroxidase-labeled anti-human IgG antibody (manufactured by American Qualex) which had been diluted 3,000 times, was dispensed in 50 µl portions into the wells to carry out the reaction at room temperature for 1 hour, the resulting plate was washed with 0.05% Tween-PBS, and then an ABTS [ammonium 2,2-azinobis(3-ethylbenzothiazole-6-sulfonate)] substrate solution was dispensed in 50 µl portions into the wells to develop color and measure its absorbance at OD415nm using Emax (manufactured by Molecular Devices).

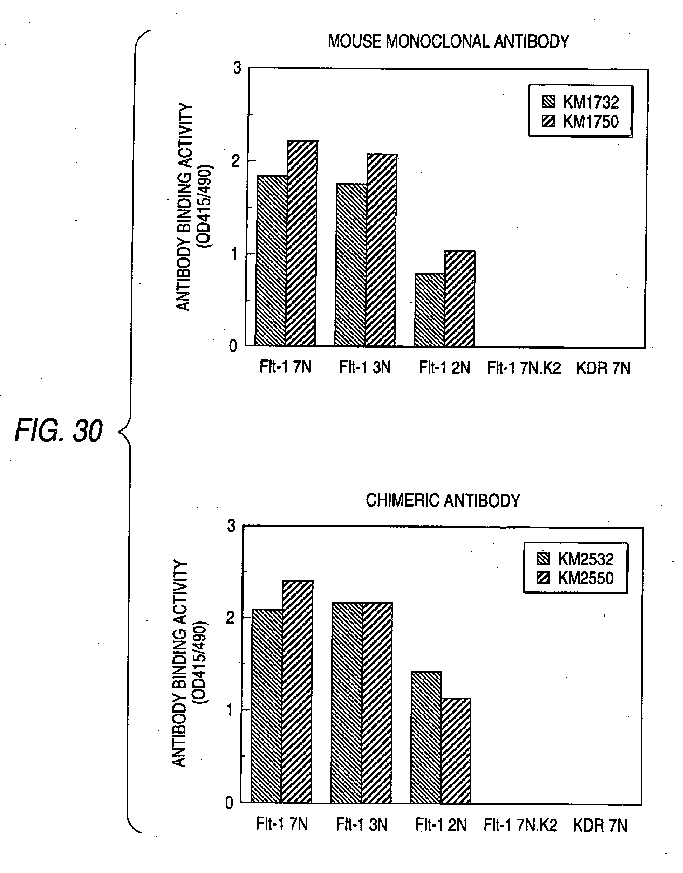

The results are shown in Fig. 30. The anti-human VEGF receptor Flt-1 mouse monoclonal antibodies KM1732 and KM1750 and anti-human VEGF receptor Flt-1 human chimeric antibodies KM2532 and KM2550 bound to Flt-1 7N, Flt-1 3N and Flt-1 2N, but not to Flt-1 7N.K2 and KDR 7N. In consequence, it was confirmed that the epitope which is recognized by anti-human VEGF receptor Flt-1 mouse monoclonal antibodies KM1732 and KM1750 and anti-human VEGF receptor Flt-1 human chimeric antibodies K2532 and KM2550 is contained in a region of from 100th to 204th amino acids of human Flt-1 from the N-terminal including a signal sequence.

Next, the binding activity was examined using an enzyme immunoassay by fixing the amount of each of Flt-1 7N, Flt-1 3N, Flt-1 2N, Flt-1 7N.K2 and KDR 7N to be adsorbed on a 96 well plate for EIA and varying the concentration of human chimeric antibody to be added. Each of Flt-1 7N, Flt-1 3N, Flt-1 2N, Flt-1 7N.K2 and KDR 7N diluted to 4 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of 0.0152 to 100 ng/ml was dispensed in 50 µl portions into the resulting wells to carry out the reaction at room temperature for 1 hour. After washing the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.05% Tween-PBS, and then an ABTS [ammonium 2,2-azinobis(3-ethylbenzothiazole-6-sulfonate)] substrate solution was dispensed in 50 µl portions into the wells to develop color and measure its absorbance at OD415nm using Emax (manufactured by Molecular Devices).

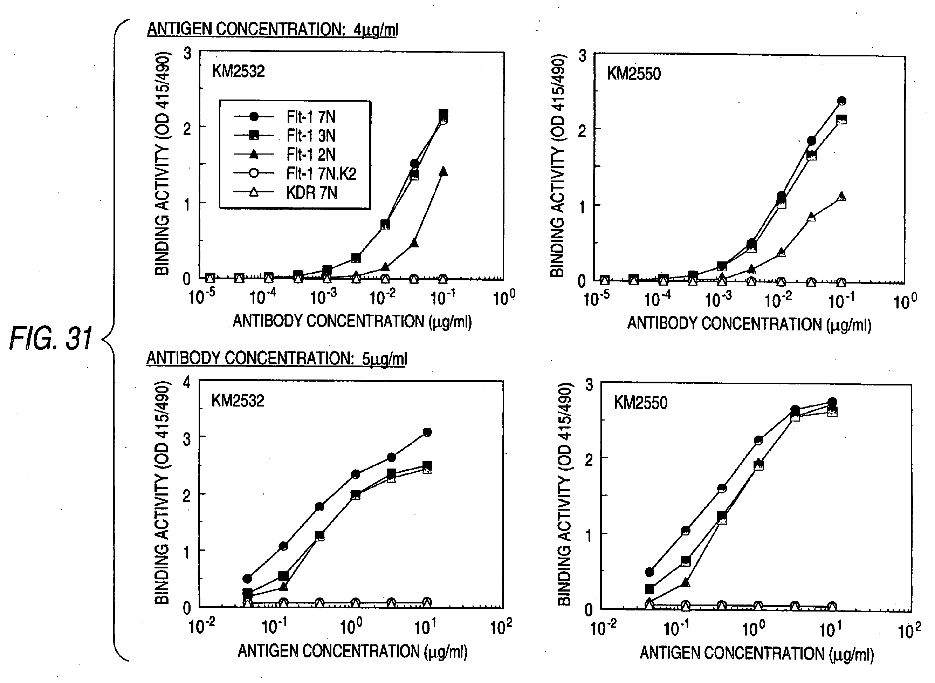

The results are shown in Fig. 31 (upper drawings). The anti-human VEGF receptor Flt-1 human chimeric antibodies KM2532 and KM2550 bound to Flt-1 7N, Flt-1 3N and Flt-1 2N in an antibody concentration dependent manner, but not to Flt-1 7N.K2 and KDR 7N. In addition, the anti-human VEGF receptor Flt-1 human chimeric antibodies KM2532 and KM2550 showed almost the same binding activity.

Next, the binding activity of human chimeric antibodies was examined using an enzyme immunoassay by varying the amount of each of Flt-1 7N, Flt-1 3N, Flt-1 2N, Flt-1 7N.K2 or KDR 7N to be adsorbed on a 96 well plate for EIA. Each of the soluble human VEGF receptors Flt-1 7N, Flt-1 3N, Flt-1 2N, Flt-1 7N.K2 and KDR 7N diluted to a concentration of from 0.041 to 10 µg/ml with PBS was dispensed in 50 µl portions into wells of a 96 well plate for EIA (manufactured by Greiner) and allowed to stand overnight at 4°C for its adsorption. After washing the plate with PBS, 100 µl of 1% BSA-PBS was added to each well, and the remaining active residues was blocked by carrying out the reaction at room temperature for 1 hour. After discarding 1% BSA-PBS, each of the purified anti-human VEGF receptor Flt-1 human chimeric antibodies KM2532 and KM2550 having a concentration of 5 µg/ml was dispensed in 50 µl portions into the resulting wells to carry out the reaction at room temperature for 1 hour. After washing of the plate with 0.05% Tween-PBS, a peroxidase-labeled anti-human IgG antibody (manufactured by American Qualex) which had been diluted 3,000 times with 1% BSA-PBS was dispensed in 50 µl portions into the wells to carry out the reaction at room temperature for 1 hour, the resulting plate was washed with 0.05% Tween-PBS, and then an ABTS [ammonium 2,2-azinobis(3-ethylbenzothiazole-6-sulfonate)] substrate solution was dispensed in 50 µl portions into the wells to develop color and measure its absorbance at OD415nm using Emax (manufactured by Molecular Devices).

The results are shown in Fig. 31 (lower drawings). The anti-human VEGF receptor Flt-1 human chimeric antibodies KM2532 and KM2550 showed concentration dependent binding activity upon the human VEGF receptors Flt-1 7N, Flt-1 3N and Flt-1 2N, but did not react with Flt-1 7N.K2 and KDR 7N. In addition, the anti-human VEGF receptor Flt-1 human chimeric antibodies KM2532 and KM2550 showed almost the same binding activity.

### 3. Production of anti-human VEGF receptor Flt-1 human CDR-grafted antibody

Anti-human VEGF receptor Flt-1 human CDR-grafted antibodies having activity similar to that of anti-human VEGF receptor Flt-1 mouse monoclonal antibodies KM1732 and KM1750 and anti-human VEGF receptor Flt-1 human chimeric antibodies KM2532 and KM2550 having activity which neutralizes human VEGF receptor Flt-1 was produced as follows.

### (1) Construction of cDNA encoding VH of anti-human VEGF receptor Flt-1 human CDR-grafted antibody based on common sequence of VH of known human antibodies

According to Kabat et al. (Sequences of Proteins of Immunological Interest, US Dept. Health and Human Services, 1991, hereinafter the same), known human antibody VHs are classifiable into subgroups I to III (HSG I to III) based on the homology of amino acid sequences, and common sequences have been identified for respective subgroups. An amino acid sequence of VH of an anti-human VEGF receptor Flt-1 human CDR-grafted antibody was designed based on the common sequences. For selecting the common sequences to serve as the base, the homology was examined between the amino acid sequence of framework regions (hereinafter referred to as "FR") of the common sequences of human antibody VH of each subgroup and amino acid sequences of FR of VH of anti-human VEGF receptor Flt-1 mouse monoclonal antibodies KM1732 and KM1750 (Table 1).

**TABLE 1**

| | HSG I | HSG II | HSG III |
|---|---|---|---|
| KM1732 | 65.5 | 51.7 | 55.2 |
| KM1750 | 67.8 | 49.4 | 54.0 |

As a result, it was confirmed that KM1732 and KM1750 shows the highest homology with subgroup I. Thus, based on the common sequence of subgroup I, an anti-human VEGF receptor Flt-1 human CDR-grafted antibody was designed, and the cDNA encoding the amino acid sequence was constructed by using the polymeraze chain reaction method (hereinafter referred to as "PCR") in the following manner.

The construction of cDNA encoding VH of an anti-human VEGF receptor Flt-1 human CDR-grafted antibody derived from the VH of anti-human VEGF mouse receptor Flt-1 mouse monoclonal antibody KM1732 is explained below. Six synthetic DNAs having a nucleotide sequence of SEQ ID NOS:25 to 30 were synthesized (manufactured by Sawady Technology). The DNAs synthesized each was added, to have a final concentration of 0.1 µM, to 50 µl of 1 × Ex Taq buffer (manufactured by Takara Shuzo Co. , Ltd.) containing 200 µM dNTP, 0.5 µM M13primer RV, 0.5 µM M13primer M4 and 2.5 units of TaKaRa Ex Taq DNA polymerase (all manufactured by Takara Shuzo Co., Ltd.), the mixture was covered with 50 µl of mineral oil, DNA thermal cycler PJ480 (manufactured by Perkin Elmer) was loaded with the mixture, and 30 PCR cycles (at 94°C for 2 minutes, at 55°C for 2 minutes and at 72°C for 2 minutes per cycle) were conducted. The reaction mixture was purified using QIAquick PCR Purification Kit (manufactured by Qiagen) according to the manual attached hereto, and the mixture was purified and eluted with 20 µl of sterilized water. Next, the obtained elution was added to 30 µl of a buffer solution comprising 10 mM Tris-HCl (pH 7.5), 10 mM magnesium chloride and 1 mM DTT, 20 units of a restriction enzyme *Apa*I (manufactured by Takara Shuzo Co., Ltd.) were further added thereto, and then the reaction was carried out at 37°C for 1 hour. The reaction mixture was precipitated with ethanol, the precipitate was added to 10 µl of a buffer solution comprising 50 mM Tris-HCl (pH 7.5), 100 mM sodium chloride, 10 mM magnesium chloride, 1 mM DTT, 100 µg/ml BSA, and 0.01% Triton X-100, 10 units of restriction enzyme *Not*I (manufactured by Takara Shuzo Co., Ltd.) were further added thereto, and then the reaction was allowed to proceed at 37°C for 1 hour. The reaction mixture was fractionated by agarose gel electrophoresis, and about 0.5 µg of an *Apa*I-*Not*I fragment about 0.44 kb was recovered.

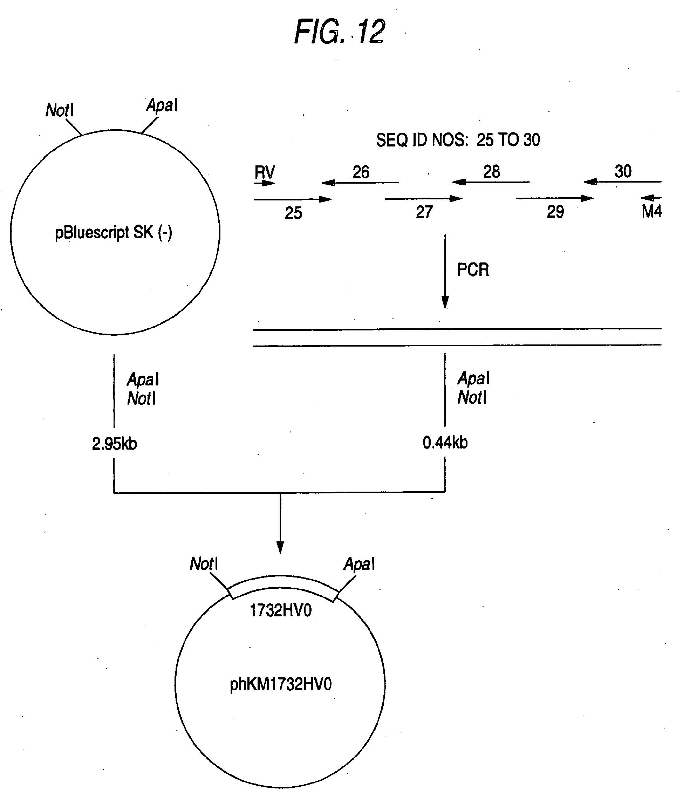

To 10 µl in total volume of sterilized water, 0.1 µg of the *Apa*I-*Not*I fragment derived from plasmid pBluescript SK(-) obtained in 2 (1) of Example 1, and 0.1 µg of the *Apa*I-*Not*I fragment which was the PCR amplified fragment obtained in the above were added, and the fragments were linked using DNA ligation Kit Ver. 2 (manufactured by Takara Shuzo Co., Ltd.) according to the manual attached hereto. *Escherichia coli* DH5α (manufactured by TOYOBO, CO., LTD.) was transformed by using the thus-obtained recombinant plasmid DNA solution. Plasmid DNA was prepared from each of ten Escherichia coli transformants. As a result that the nucleotide sequence was determined according to the method described in 1(4) of Example 1, plasmid phKM1732HVO shown in Fig. 12 and containing cDNA encoding the amino acid sequence of interest was obtained. The nucleotide sequence and the amino acid sequence of the VH of the anti-human VEGF receptor Flt-1 human CDR-grafted antibody (hereinafter referred to as "1732HVO") contained in phKM1732HVO are shown in SEQ ID NOS:31 and 90, respectively.

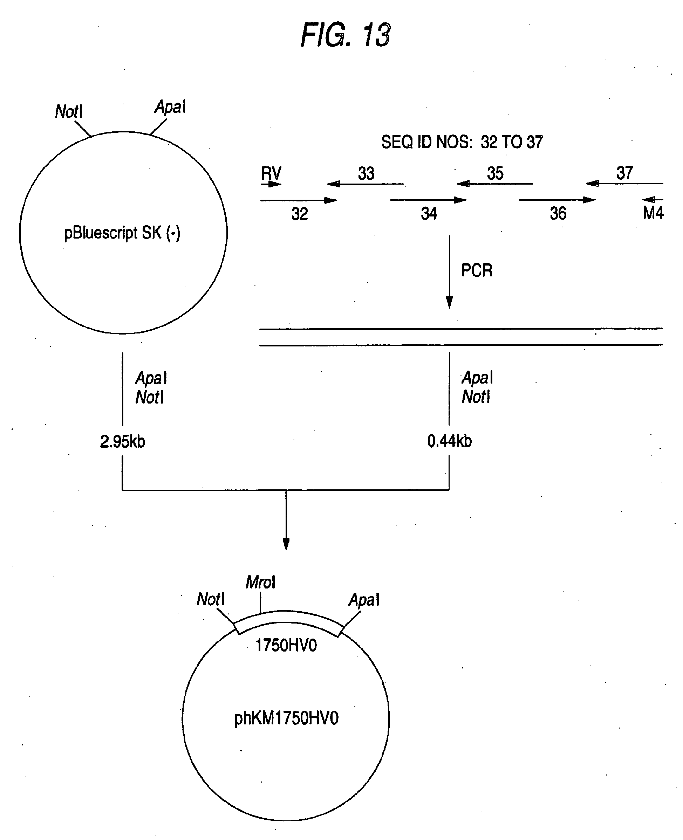

With regard to the construction of cDNA encoding VH of an anti-human VEGF receptor Flt-1 human CDR-grafted antibody derived from the VH of the anti-human VEGF mouse receptor Flt-1 mouse monoclonal antibody KM1750, the reactions were carried out in the same manner as described above using six synthetic DNAs having a nucleotide sequence of SEQ ID NOS:32 to 37 were synthesized (manufactured by Sawady Technology) to obtain plasmid phKM1750HVO containing cDNA encoding an amino acid sequence of interest shown in Fig. 13. The nucleotide sequence and the amino acid sequence of the VH of the anti-human VEGF receptor Flt-1 human CDR-grafted antibody (hereinafter referred to as "1750HV0") contained in phKM1750HVO are shown in SEQ ID NOS:38 and 91, respectively.

### (2) Construction of cDNA encoding VL of anti-human VEGF receptor Flt-1 human CDR-grafted antibody based on common sequence of VL of known human antibodies

According to Kabat *et al*., known human antibody VLs are classifiable into subgroups I to IV (HSG I to IV) based on the homology of amino acid sequences, and the common sequence has been identified for respective subgroups. An amino acid sequence of VL of an anti-human VEGF receptor Flt-1 human CDR-grafted antibody was designed based on the common sequence. For selecting the common sequence to serve as the base, the homology was examined between the amino acid sequence of FR of the common sequence of human antibody VL of each subgroup and amino acid sequences of FR of VL of anti-human VEGF receptor Flt-1 mouse monoclonal antibodies KM1732 and KM1750 (Table 2).

**TABLE 2**

| | HSG I | HSG II | HSG III | HSG IV |
|---|---|---|---|---|
| KM1732 | 66.2 | 57.5 | 62.5 | 63.8 |
| KM1750 | 68.8 | 63.8 | 68.8 | 70.0 |

As a result, it was confirmed that KM1732 shows the highest homology with subgroup I and KM1750 shows almost the same high homology with subgroups I and-IV, so that the amino acid sequence of the VL of anti-human VEGF receptor Flt-1 human CDR-grafted antibody was designed based on the common sequence of subgroup I in the case of KM1732 and the common sequence of subgroups I and IV in the case of KM1750, and cDNA encoding the amino acid sequence was constructed by PCR in the following manner.

The construction of cDNA encoding VL of an anti-human VEGF receptor Flt-1 human CDR-grafted antibody derived from the VL of anti-human VEGF mouse receptor Flt-1 mouse monoclonal antibody KM1732 is explained below. Six synthetic DNAs having a nucleotide sequence of SEQ ID NOS:39 to 44 were synthesized (manufactured by Sawady Technology). The DNAs synthesized each was added, to have a final concentration of 0.1 µM, to 50 µl of 1 x Ex Taq buffer (manufactured by Takara Shuzo Co. , Ltd.) containing 200 µM dNTP, 0.5 µM M13primer RV, 0.5 µM M13primer M4 and 2.5 units of TaKaRa Ex Taq DNA polymerase (all manufactured by Takara Shuzo Co., Ltd.), the mixture was covered with 50 µl of mineral oil, DNA thermal cycler PJ480 (manufactured by Perkin Elmer) was loaded with the mixture, and 30 PCR cycles (at 94°C for 2 minutes, at 55°C for 2 minutes and at 72°C for 2 minutes per cycle) were conducted. The reaction mixture was purified using QIAquick PCR Purification Kit (manufactured by Qiagen) according to the manual attached hereto, and the mixture was purified and eluted with 20 µl of sterilized water. Next, the obtained elution was added to 30 µl of a buffer solution comprising 50 mM Tris-HCl (pH 7.5), 100 mM sodium chloride, 10 mM sodium magnesium, 1 mM DTT, and 100 µg/ml BSA, 10 units of a restriction enzyme *Eco*RI (manufactured by Takara Shuzo Co., Ltd.), 10 units of a restriction enzyme *Spl*I (manufactured by Takara Shuzo Co., Ltd.) were further added thereto, and then the reaction was carried out at 37°C for 1 hour. The reaction mixture was fractionated by agarose gel electrophoresis, and about 0.5 µg of an *Eco*RI-*Spl*I fragment about 0.39 kb was recovered.

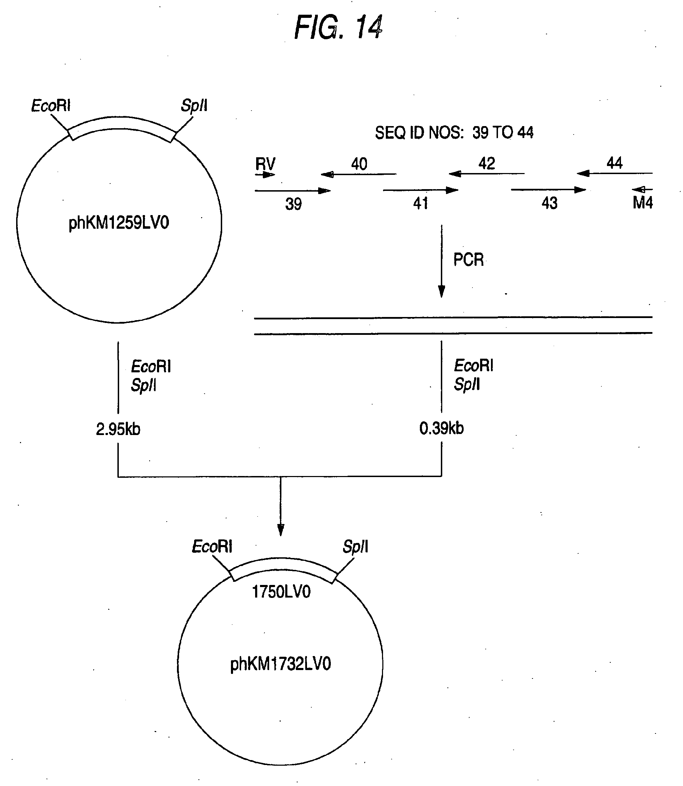

To 10 µl in total volume of sterilized water, 0.1 µg of the *Eco*RI-*Spl*I fragment derived from plasmid phKM1259LV0 obtained in 2(1) of Example 1, and 0.1 µg of the *Eco*RI-*Spl*I fragment which was the PCR amplified fragment obtained in the above were added, and the fragments were linked using DNA ligation Kit Ver. 2 (manufactured by Takara Shuzo Co., Ltd.) according to the manual attached hereto. *Escherichia coli* DH5α (manufactured by TOYOBO CO., LTD.) was transformed by using the thus-obtained recombinant plasmid DNA solution. Plasmid DNA was prepared from each of ten *Escherichia coli* transformants. As a result that the nucleotide sequence was determined according to the method described in 1(4) of Example 1, plasmid phKM1732LV0 shown in Fig. 14 and containing cDNA encoding the amino acid sequence of interest was obtained. The nucleotide sequence and the amino acid sequence of the VL of the anti-human VEGF receptor Flt-1 human CDR-grafted antibody (hereinafter referred to as "1732LV0") contained in phKM1732LV0 are shown in SEQ ID NOS:45 and 92, respectively.

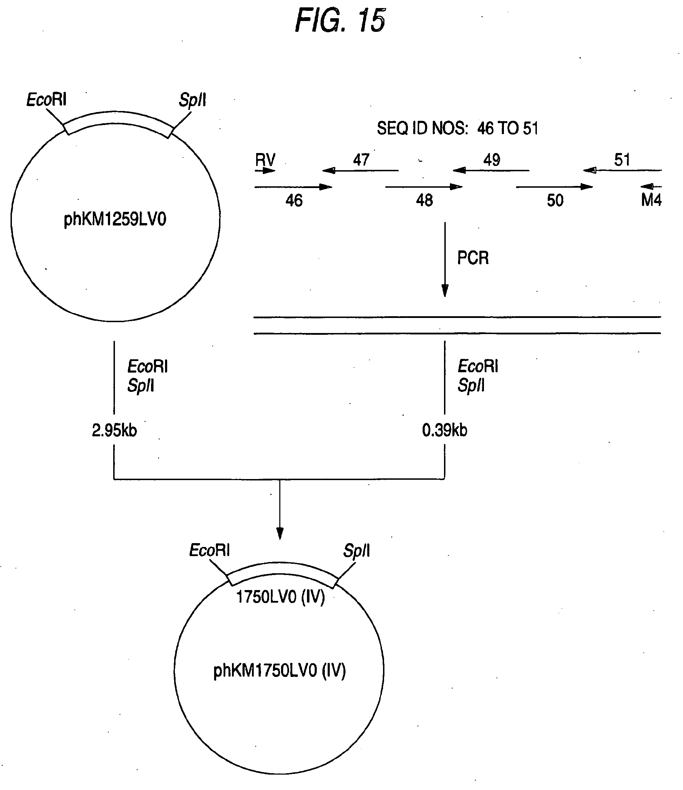

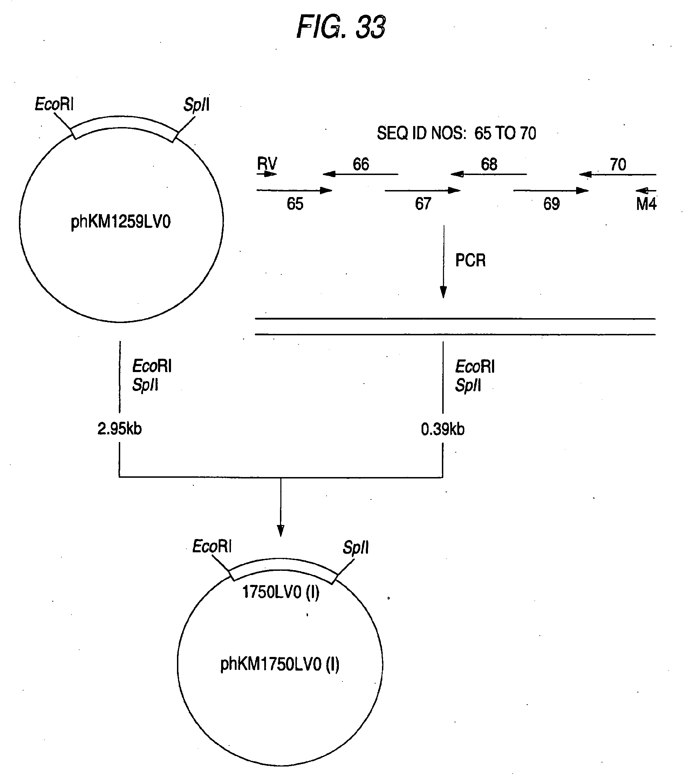

When the common sequence of human subgroup IV was used as the base in constructing cDNA encoding the VL of anti-human VEGF receptor Flt-1 human CDR-grafted antibody derived from the VL of anti-human VEGF receptor Flt-1 mouse monoclonal antibody KM1750, plasmid phKM1750LV0 (IV) containing cDNA encoding the amino acid sequence of interest shown in Fig. 15 was obtained by carrying out the reaction similar to the above using 6 Synthetic DNA fragments (manufactured by Sawady Technology) having nucleotide sequences of SEQ ID NOS:46 to 51. Corresponding nucleotide sequence and amino acid sequence of the VL of anti-human VEGF receptor Flt-1 human CDR-grafted antibody contained in phKM1750LV0 (IV) [hereinafter referred to as "1750LV0(IV)"] are shown in SEQ ID NOS:52 and 93, respectively. When the common sequence of human subgroup I was used as the base, plasmid phKM1750LV0 (I) containing cDNA encoding the amino acid sequence of interest shown in Fig. 33 was obtained by carrying out the reaction similar to the above using 6 synthetic DNA fragments (manufactured by Sawady Technology) having nucleotide sequences of SEQ ID NOS:65 to 70. Corresponding nucleotide sequence and amino acid sequence of the VL of anti-human VEGF receptor Flt-1 human CDR-grafted antibody contained in phKM1750LV0(I) [hereinafter referred to as "1750LV0 (I)"] are shown in SEQ ID NOS:71 and 94, respectively.

### (3) Construction of anti-human VEGF receptor Flt-1 human CDR-grafted antibody expression vectors based on common sequence of V regions of known human antibodies

Expression vectors of anti-human VEGF receptor Flt-1 human CDR-grafted antibody were constructed in the following manner using tandem cassette vector, pKANTEX93, for humanized antibody expression described in WO 97/10354 and plasmids phKM1732HV0, phKM1732LV0, phKM1750HV0, phKM1750LV0 (I) and phKM1750LV0 (IV) containing cDNA encoding the V region of anti-human VEGF receptor Flt-1 human CDR-grafted antibody obtained in 3 (1) and 3 (2) of Example 1.

The construction of an expression vector of anti-human VEGF receptor Flt-1 human CDR-grafted antibody derived from anti-human VEGF receptor Flt-1 mouse monoclonal antibody KM1732 is explained below.

To 10 µl of a buffer solution comprising 10 mM Tris-HCl (pH 7.5), 10 mM magnesium chloride and 1 mM DTT, 5 µg of plasmid phKM1732HV0 was added, 10 units of a restriction enzyme *Apa*I (manufactured by Takara Shuzo Co., Ltd.) were further added thereto, and then the reaction was carried out at 37°C for 1 hour. The reaction mixture was precipitated with ethanol, the precipitate was added to 10 µl of a buffer solution comprising 50 mM Tris-HCl (pH 7.5), 100 mM sodium chloride, 10 mM magnesium chloride, 1 mM DTT, 100 µg/ml BSA, and 0.01% Triton X-100, 10 units of a restriction enzyme *Not*I (Takara Shuzo Co., Ltd.) were further added thereto, and then the reaction was carried out at 37°C for 1 hour. The reaction mixture was fractionated by agarose gel electrophoresis, and about 0.5 µg of an *Apa*I-*Not*I fragment about 0.44 kb was recovered.

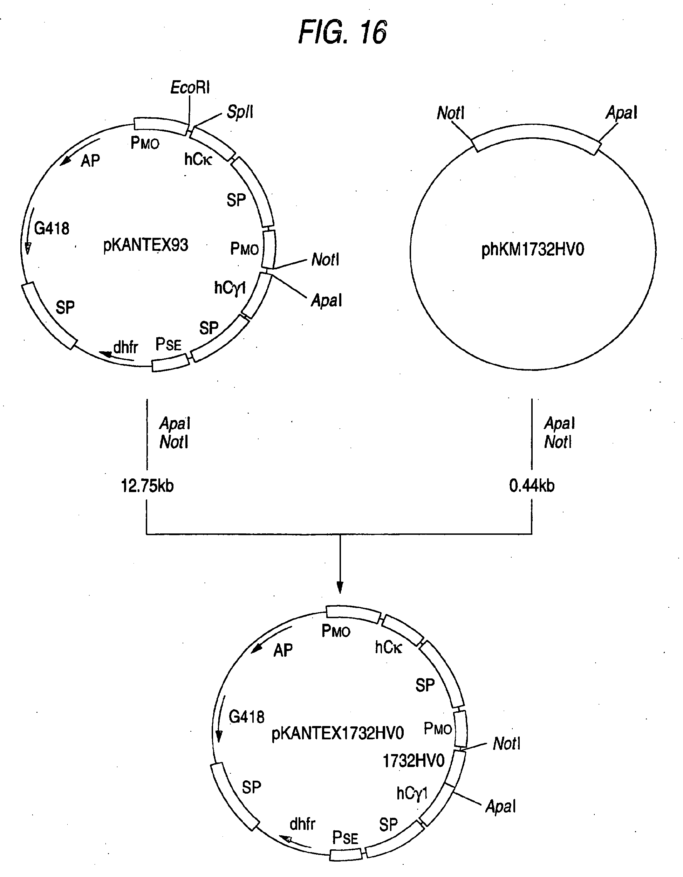

To 10 µl in total volume of sterilized water, the *Apa*RI-*Not*I fragment obtained in 2(1) of Example 1 derived from tandem cassette vector for humanized antibody expression, pKANTEX93, and 0.1 µg of the *Apa*I-*Not*I fragment of phKM1732HV0 obtained in the above were added, and the fragments were linked using DNA ligation Kit Ver. 2 (manufactured by Takara Shuzo Co., Ltd.) according to the manual attached hereto. *Escherichia coli* DH5α (manufactured by TOYOBO CO., LTD.) was transformed by using the thus-obtained recombinant plasmid DNA solution to obtain plasmid pKANTEX1732HV0 shown in Fig. 16.

Next, 3 µg of the thus obtained plasmid pKANTEX1732HV0 was added to a buffer solution comprising 50 mM Tris-Hcl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were added thereto, and the reaction was carried out at 37°C for 1 hour. The reaction mixture was fractionated by agarose gel electrophoresis, and about 1 µg of an *Eco*I-*Spl*I fragment about 13.20 kb in size was recovered. Further, 5 µg of plasmid phKM1732LV0 was added to a buffer solution comprising. 50 mM Tris-HCl (pH 7.5), 100 mM sodium chloride, 10 mM magnesium chloride, 1 mM DTT and 100 µg/ml BSA, 10 units of a restriction enzyme *Eco*RI (manufactured by Takara Shuzo Co., Ltd.) and 10 units of a restriction enzyme *Spl*I (manufactured by Takara Shuzo Co., Ltd.) were added thereto, and the reaction was carried out at 37°C for 1 hour. The reaction mixture was fractionated by agarose gel electrophoresis, and about 0.5 µg of an *Eco*I-*Spl*I fragment about 0.39 kb in size was recovered.

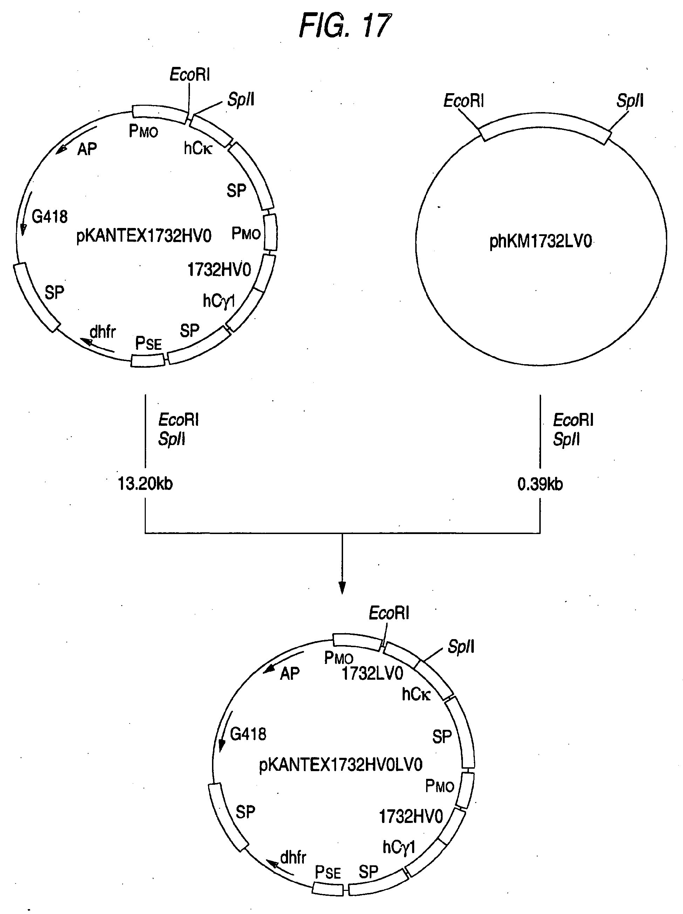

To 10 µl in total volume of sterilized water, 0.1 µg of the thus obtained *Eco*RI-*Spl*I fragment derived from plasmid pKANTEX1732HV0 and 0.1 µg of the *Eco*I-*Spl*I fragment derived from plasmid phKM1732LV0 were added, and the fragments were ligated using DNA ligation Kit Ver. 2 (manufactured by Takara Shuzo Co., Ltd.) according to the manual attached hereto. *Escherichia coli* DH5α (manufactured by TOYOBO CO., LTD.) was transformed by using the thus-obtained recombinant plasmid DNA solution to obtain an expression vector of anti-human VEGF receptor Flt-1 human CDR-grafted antibody, pKANTEX1732HV0LV0, shown in Fig. 17.

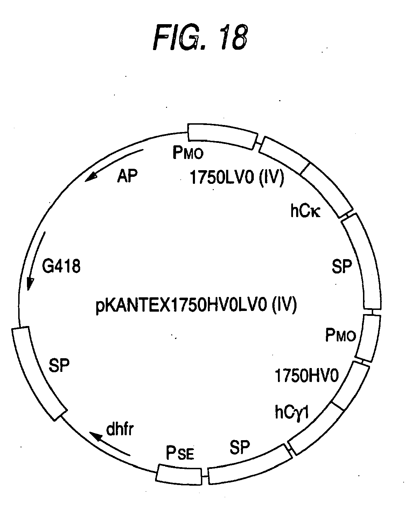

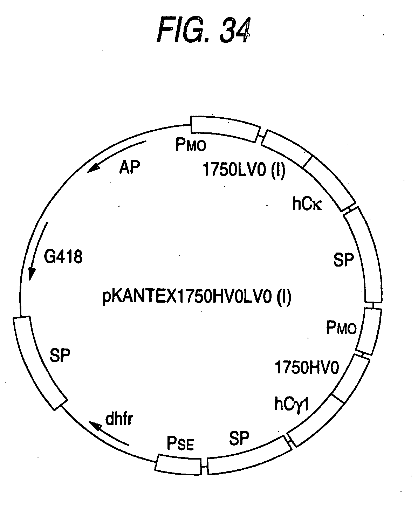

Regarding the construction of expression vectors of anti-human VEGF receptor Flt-1 human CDR-grafted antibody originated from anti-human VEGF receptor Flt-1 mouse monoclonal antibody K1750, the reaction similar to the above was carried out to obtain vectors for anti-human VEGF receptor Flt-1 human CDR-grafted antibody expression, pKANTEX1750HVOLVO(IV) and pKANTEX1750HVOLVO(I), shown in Figs. 18 and 34, respectively.

### 4. Analysis of amino acid sequence of FR of V region of anti-human VEGF receptor Flt-1 human CDR-grafted antibody and production of expression vector for modified human CDR-grafted antibody in which amino acids of FR are modified

Among amino acid residues which are different between the amino acid sequence of the FR of the V region of each anti-human VEGF receptor Flt-1 human CDR-grafted antibody and the amino acid sequence of the FR of the V region of each anti-human VEGF receptor Flt-1 mouse monoclonal antibody, the positions of the amino acid residues which were considered to be important for binding to human VEGF receptor Flt-1 in the amino acid sequence of the FR of the V region of each anti-human VEGF receptor Flt-1 human CDR-grafted antibody were identified by constructing and comparing a computer three-dimensional structure model of each of the V region of anti-human VEGF receptor Flt-1 mouse monoclonal antibody KM1732 and K1750 and that of each of the anti-human VEGF receptor Flt-1 human CDR-grafted antibodies produced in 3 of Example 1. As a result, regarding the anti-human VEGF receptor Flt-1 human CDR-grafted antibody derived from anti-human VEGF receptor Flt-1 mouse monoclonal antibody KM1732, it was considered that the positions of 24-position alanine, 27-position tyrosine, 40-position alanine, 67-position arginine, 69-position threonine, 70-position isoleucine, 82-position glutamic acid and 93-position valine in the amino acid sequence of the VH of SEQ ID NO:31 and the positions of 39-position proline, 45-position leucine, 46-position leucine, 69-position asparatic acid, and 70-position phenylalanine in the amino acid sequence of VL of SEQ ID NO:45 are taking an important role in its binding to the human VEGF receptor Flt-1.

Regarding the anti-human VEGF receptor Flt-1 human CDR-grafted antibody derived from anti-human VEGF receptor Flt-1 mouse monoclonal antibody KM1750, it was considered that the positions of 3-position glutamine, 67-position arginine, 82-position glutamic acid, 84-position serine and 95-position tyrosine in the amino acid sequence of the VH of SEQ ID NO:38 and the positions of 17-position aspartic acid, 18-position arginine, 39-position proline, 59-position serine, 69-position aspartic acid and 70-position phenylalanine in the amino acid sequence of the VL of SEQ ID NO: 71 are taking an important role in its binding to the human VEGF receptor Flt-1. These facts suggest that the activity of anti-human VEGF receptor Flt-1 human CDR-grafted antibody to bind to human VEGF receptor Flt-1 can be modified, for example, by modifying the just described positions of amino acid residues of anti-human VEGF receptor Flt-1 human CDR-grafted antibody into the amino acid residues of the corresponding position of anti-hunian VEGF receptor Flt-1 mouse monoclonal antibody.

### (1) Construction of cDNA encoding VH of modified human CDR-grafted antibody in which amino acids of FR are modified

Regarding the H chain of anti-human VEGF receptor Flt-l human CDR-grafted antibody derived from anti-human VEGF receptor Flt-1 mouse monoclonal antibody K1750, the 3 residues (82-position glutamic acid, 84-position serine and 95-position tyrosine) among the above-described 5 amino acid residues considered to be taking an important role in its binding to the human VEGF receptor Flt-1 were considered to be particularly important, so that cDNA encoding an amino acid sequence which was converted into the corresponding residues (82-position glutamine, 84-position arginine and 95-position phenylalanine) found in KM1750 was constructed by PCR in the following manner.

Using 6 synthetic DNA fragments having the nucleotide sequences of SEQ ID NOS:72 to 77 (manufactured by Sawady Technology), an *Mro*I-*Apa*I fragment of about 0.39 kb was recovered by PCR in accordance with the above-described method.

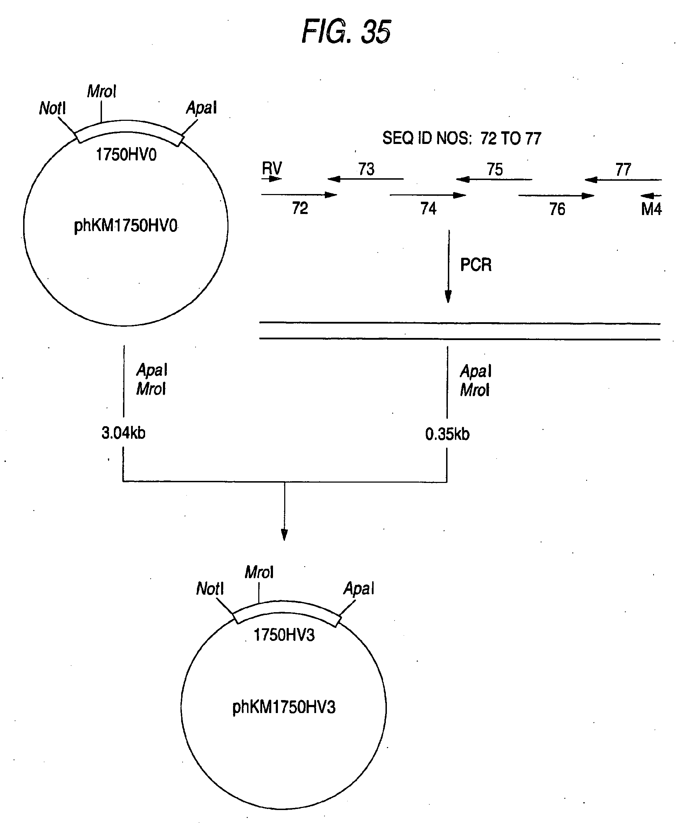

Plasmid phKM1750HV0 was treated with restriction enzymes *Apa*I and *Mro*I to recover an *Apa*I*-Mro*I fragment of about 3.04 kb which was subsequently connected with the PCR amplified fragment obtained in the above, and then *Escherichia col*i DH5α (manufactured by TOYOBO CO.., LTD.) was transformed using the thus obtained recombinant plasmid DNA solution. Plasmid was prepared from each of ten *Escherichia col*i transformants and its nucleotide sequence was determined in accordance with the method described in 1(4) of Example 1 to obtain plasmid phKM1750HV3 shown in Fig. 35 which contains cDNA encoding the amino acid sequence of interest.

Corresponding nucleotide sequence and amino acid sequence of the VH of anti-human VEGF receptor Flt-1 human CDR-grafted antibody contained in phKM1750HV3 (hereinafter referred to as "1750HV3") are shown in SEQ ID NOS:78 and 95, respectively.

### (2) Construction of cDNA encoding VL of modified human cDR-grafted antibody in which amino acids of FR are modified

Regarding L chain of anti-human VEGF receptor Flt-1 human CDR-grafted antibody derived from anti-human VEGF receptor Flt-1 mouse monoclonal antibody KM1750, the 4 residues (17-position aspartic acid, 18-position arginine, 69-position aspartic acid and 70-position phenylalanine) among the above-described 6 amino acid residues considered to be taking an important role in its binding to the human VEGF receptor Flt-1 were considered to be particularly important in KM1750LV0 (I), so that cDNA encoding an amino acid sequence which was converted into the corresponding residues (17-position glutamic acid, 18-position glutamic, 69-position phenylalanine and 70-position tyrosine) found in KM1750 was constructed by PCR in the following manner.

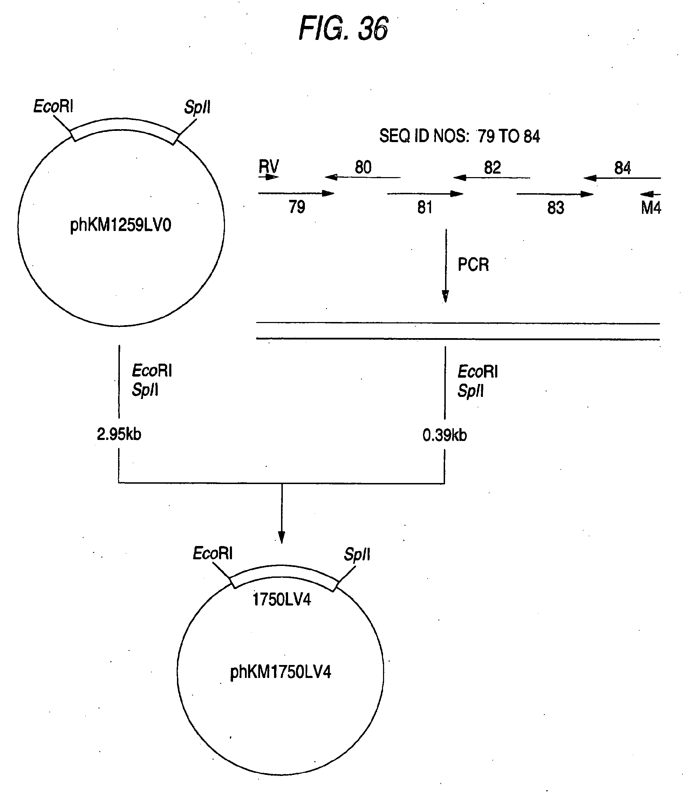

Using 6 synthetic DNA fragments having the nucleotide sequences of SEQ ID NOS:79 to 84 (manufactured by Sawady Technology), the same reaction was carried out to obtain the plasmid phKM1750LV4 shown in Fig. 36 which contains cDNA encoding the amino acid sequence of interest. Corresponding nucleotide sequence and amino acid sequence of the VL of anti-human VEGF receptor Flt-1 human CDR-grafted antibody contained in phKM1750LV4 (hereinafter referred to as "1750LV4") are shown in SEQ ID NOS:85 and 96, respectively.

### (3) Construction of expression vector for modified human CDR-grafted antibody in which amino acids of FR are modified

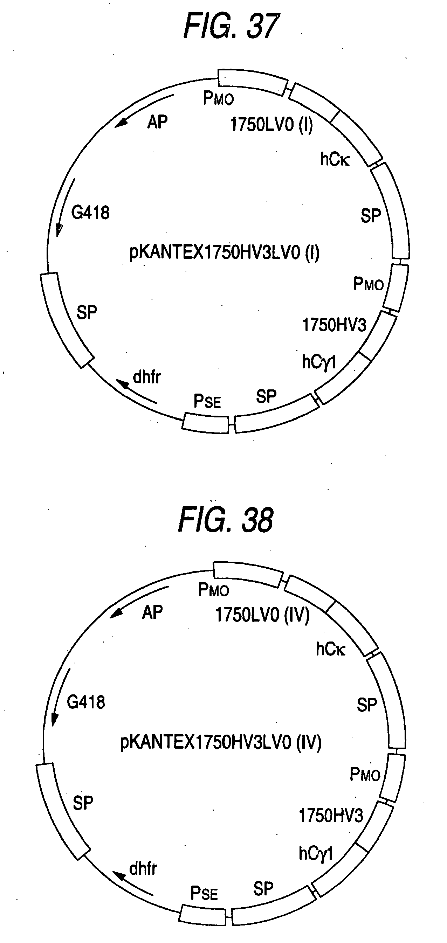

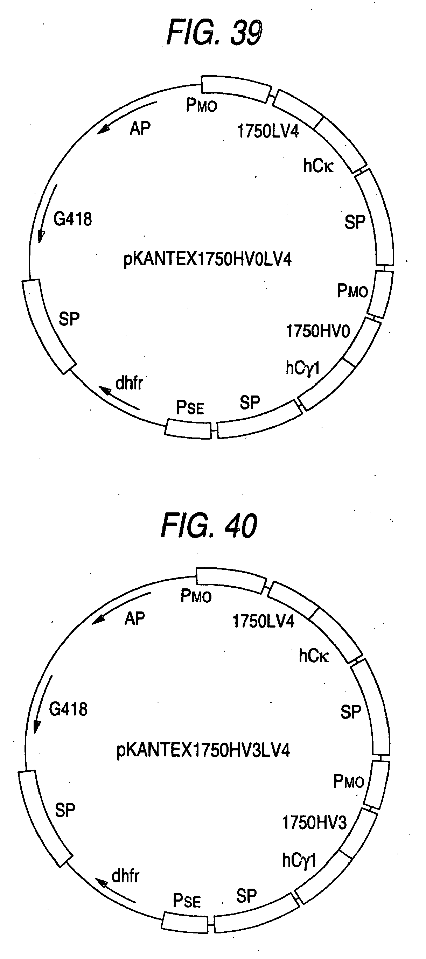

Using plasmids phKM1750HV3 and phKM1750LV4 obtained in 4(3) of Example 1 containing cDNA encoding the V region of anti-human VEGF receptor Flt-1 human CDR-grafted antibody and plasmids phKM1750HV0, phKM1750LVO(I) and phKM1750LV0 (IV) obtained in 3(1) and 3(2) of Example 1, the reaction similar to that in 3(3) of Example 1 was carried out to obtain pKANTEX1750HV3LV0 (I), pKANTEX1750HV3hV0 (IV), pKANTEX1750HV0LV4, and pKANTEX1750HV3LV4 as expression vectors of modified anti-human VEGF receptor Flt-1 human CDR-grafted antibodies in which amino acids of FR were modified shown in Figs. 37, 38, 39, and 40, respectively.

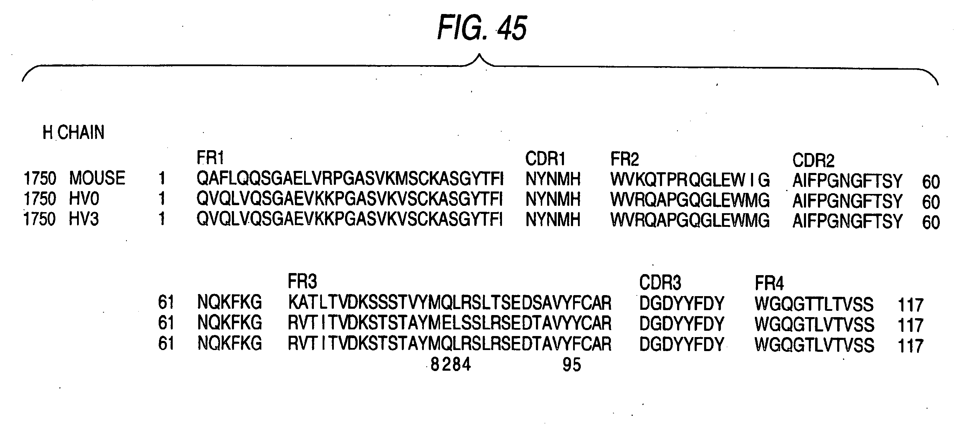

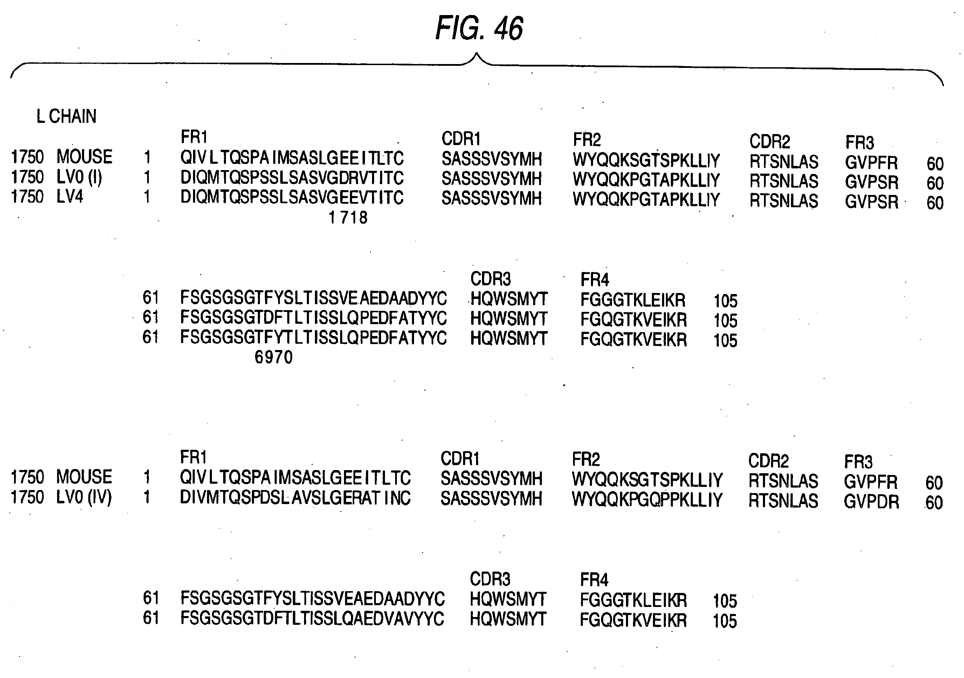

Amino acid sequences of KM1750 mouse H chain, KM1750HV0 and KM1750HV3 are shown in Fig. 45. Amino acid sequences of KM1750 mouse L chain, KM1750LV0 (I), KM1750LV0(IV) and KM1750LV4 are shown in Fig. 46.

*Escherichia coli* DH5α/pHKM1750HV0 in which recombinant plasmid phKM1750HV0 is introduced, *Escherichia coli* DH5α/phKM1750HV3 in which recombinant plasmid phKM1750HV3 is introduced, *Escherichia coli* XL1-Blue/phKM1750LV0 (I) in which recombinant plasmid phKM1750LV0 (I) is introduced, *Escherichia coli* XL1-Blue/phKM1750LV0 (IV) in which recombinant plasmid phFQM1750LV0 (IV) is introduced, and *Escherichia coli* DH5α/phKM1750LV4 in which recombinant plasmid phKM1750LV4 is introduced have been deposited on May 12, 1999, in National Institute of Bioscience and Human Technology, Agency of Industrial Science and Technology (Higashi 1-1-3, Tsukuba-shi, Ibaraki, Japan) as FERM BP-6719, FERM BP-6720, FERM BP-6716, FERM BP-6717, and FERM BP-6718, respectively.

### 5. Production and activity evaluation of anti-human VEGF receptor Flt-1 human CDR-grafted antibody

### (1) Expression of anti-human VEGF receptor Flt-1 human CDR-grafted antibody in rat myeloma YB2/0 cells (ATCC CRL1581) using pKANTEX1750HV0LV0 (I), pKANTEX1750HV0LV0 (IV), pKANTEX1750HV3LV0 (I), pKANTEX1750HV3LV0 (IV), pKANTEX1750HV0LV4 and pKANTEX1750HV3LV4

Introduction of anti-human VEGF receptor Flt-1 human CDR-grafted antibody expression vectors pKANTEX1750HV0LV0 (I), pKANTEX1750HV0LV0 (IV), pKANTEX1750HV3LV0 (I), pKANTEX1750HV3LV0 (IV), pKANTEX1750HV0LV4 and pKANTEX1750HV3LV4 into YB2/0 cells was carried out by the electroporation method [*Cytotechnology, 3*: 133 (1990)] according to 1(1) of Example 1.

A 5 µg portion of each of pKANTEX1750HV0LV0 (I), pKANTEX1750HV0LV0 (IV), pKANTEX1750HV3LV0 (I), pKANTEX1750HV3LV0 (IV), pKANTEX1750HV0LV4 and pKANTEX1750HV3LV4 obtained in 4(3) of Example 1 was introduced into 4×10⁶ of YB2/0 cells, and the resulting cells were suspended in 40 ml of RPMI1640-FCS(10) medium [RPMI1640 medium (manufactured by Nissui Pharmaceutical) containing 10% fetal calf serum (FCS)] and dispensed in 200 µl portions into wells of a 96 well microtiter plate (manufactured by Sumilon). After 24 hours of culturing at 37°C in a 5% CO₂ incubator, G 418 (manufactured by Gibco) was added to a concentration of 0.5 mg/ml, and the culturing was continued for 1 to 2 weeks. Culture supernatants were recovered from wells in which transformant colonies having G 418 resistance were formed and became confluent, and the activity of anti-human VEGF receptor Flt-1 human CDR-grafted antibody in each culture supernatant was measured by the enzyme immunoassay shown in 2(3) of Example 1.

Transformants which showed the activity of anti-human VEGF receptor Flt-1 human CDR-grafted antibody in their culture supernatants were subjected to cloning by carrying out limiting dilution twice to obtain transformant cell lines capable of producing anti-human VEGF receptor Flt-1 human CDR-grafted antibody. Examples of the transformant obtained by introducing the expression vectors pKANTEX1750HV0LV0 (I), pKANTEX1750HV0LV0 (IV), pKANTEX1750HV3LV0 (I), pKANTEX1750HV3LV0 (IV), pKANTEX1750HV0LV4 and pKANTEX1750HV3LV4, namely transformants capable of producing the anti-human VEGF receptor Flt-1 human CDR-grafted antibody originated from anti-human VEGF receptor Flt-1 monoclonal antibody K1750, include KM8550, KM8551, KM8552, KM8553, KM8554 and KM8555, respectively, and the anti-human VEGF receptor Flt-1 human CDR-grafted antibodies produced by these transformants were named KM8550, KM8551, KM8552, KM8553, KM8554 and KM8555, respectively. Each of the thus obtained transformant cell clones showed an anti-human VEGF receptor Flt-1 human CDR-grafted antibody productivity of about from 0.1 to 1 µg/ml.

### (2) Purification of anti-human VEGF receptor Flt-1 human CDR-grafted antibody from culture supernatant

Each of the anti-human VEGF receptor Flt-1 human CDR-grafted antibody-producing cell lines KM8550, KM8551, KM8552, KM8553, KM8554 and KM8555 obtained in 5(1) of Example 1 was suspended in GIT medium (manufactured by Nippon Pharmaceutical) containing 0.5 mg/ml of G 418, to a cell density of 1×10⁵ to 2×10⁵ cells/ml, and the suspension was dispensed in 200 ml portions into a total of five 175 cm² flasks (manufactured by Greiner). When the cells became confluent after 5 to 7 days of culturing at 37°C in a 5% CO₂ incubator, about 1.1 to 2.5 L of each culture supernatant was recovered. A column was packed with about 1 ml of ProSep A (manufactured by Bioprocessing) and washed with 10 ml of 1 M glycine-0.15 M NaCl (pH 8.6) at a flow rate of 1 ml/minute. After washing, 2.3 L, 2.5 L, 1.9 L, 2.4 L, 1.1 L or 2 L of the culture filtrate of anti-human VEGF receptor Flt-1 human CDR-grafted antibody-producing cell line KM8550, KM8551, KM8552, KM8553, KM8554 or KM8555, respectively, prepared in the above was passed through the ProSep A column at a flow rate of 70 ml/hour. After washing with 10 ml of 1 M glycine-0.15 M NaCl (pH 8.6) at a flow rate of 1 ml/minute, the column was further washed step by step with 4 ml each of 50 mM citrate buffer of pH 6, 5 and 4 in that order and then 7 ml of 50 mM citrate buffer (pH 3.0) ways passed through the column to elute the human CDR-grafted antibody. As a result, anti-human VEGF receptor Flt-1 human CDR-grafted antibodies KM8550, KM8551, KM8552, KM8553, KM8554 and KM8555 were obtained in amounts of 1.1 mg, 1.8 mg, 1.6 mg, 2.2 mg, 1.3 mg and 1.6 mg, respectively.

The thus purified anti-human VEGF receptor Flt-1 human CDR-grafted antibodies KM8550, KM8551, KM8552, KM8553, KM8554 and KM855S were analyzed by the SDS-PAGE method shown in 2 (3) of Example 1. Each of the anti-human VEGF receptor Flt-1 human CDR-grafted antibodies KM8550, KM8551, KM8552, KM8553, KM8554 and KM8555, the anti-human VEGF receptor Flt-1 human chimeric antibody KM2550 shown in 2(4) of Example 1 and KM8969 of IgG type (Japanese Published Unexamined Patent Application No. 257893/98) which reacts to ganglioside GM₂ and does not react to human VEGF receptor Flt-1 as a control human CDR-grafted antibody was applied to the electrophoresis in an amount of 2 µg protein per lane under reducing and non-reducing conditions, and then the gels were stained with Coomassie Brilliant Blue. The results are shown in Fig. 41. The human CDR-grafted antibodies KM8550, KM8551, KM8552, KM8553, KM8554 and KM8555 showed a band of IgG at a position of about 150 kDa under the non-reducing condition, and a band of H chain at a position of about 50 kDa and a band of L chain at about 25 kDa under the reducing condition. This result coincided with that the IgG type antibody is present as a molecule of 150 kDa, and separated into two H chains and two L chains under the reducing conditions caused by the cutting of intermolecular disulfide bond, and it is shown that the human CDR-grafted antibodies KM8550, KM8551, KM8552, KM8553, KM8554 and KM8555 are antibody molecules having a correct structure.

### (3) Binding activities of anti-human VEGF receptor Flt-1 human CDR-grafted antibodies upon human VEGF receptor Flt-1

The activity of purified anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 to bind to the human VEGF receptor Flt-1 was confirmed according to the method in 2(5) of Example 1.

Amount of Flt-1 7N to be coated on the plate was fixed to 2 µg/ml, and concentration of each of the purified antibodies was varied from 1.23 to 900 ng/ml (3-fold dilutions).

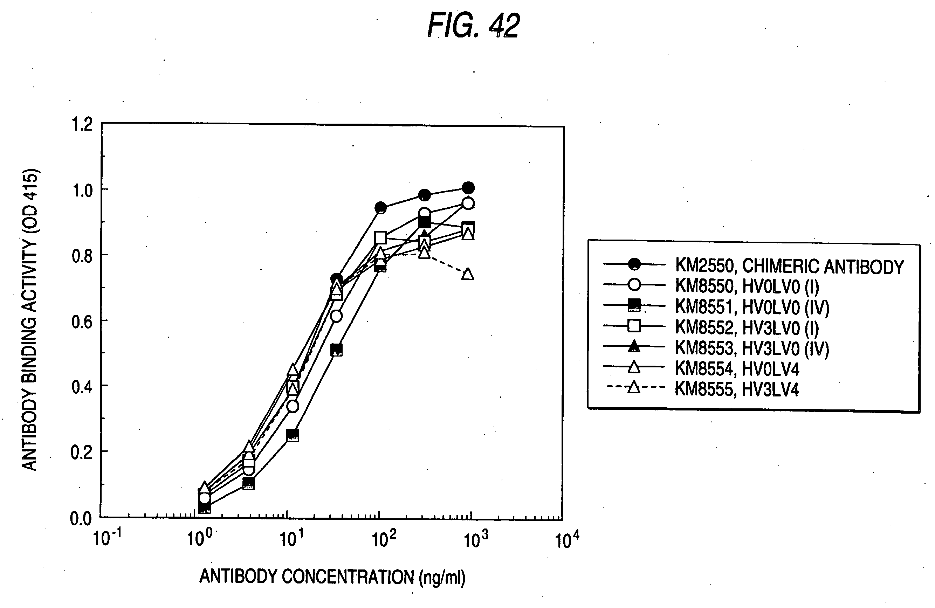

The results are shown in Fig. 42. The anti-human VEGF receptor Flt-1 human CDR-grafted antibodies KM8550, KM8551, KM8552, KM8553 , KM8554 and KM8555 and anti-human VEGF receptor Flt-1 human chimeric antibody KM2550 bound to the human VEGF receptor Flt-1 7N in an antibody, concentration dependent manner. In addition, the anti-human VEGF receptor as a negative control antibody did not react with the soluble human VEGF receptor derivative proteins tested. In consequence, it was revealed that the six human CDR-grafted antibodies of the present invention keep the binding specificity of human chimeric antibody KM2550.

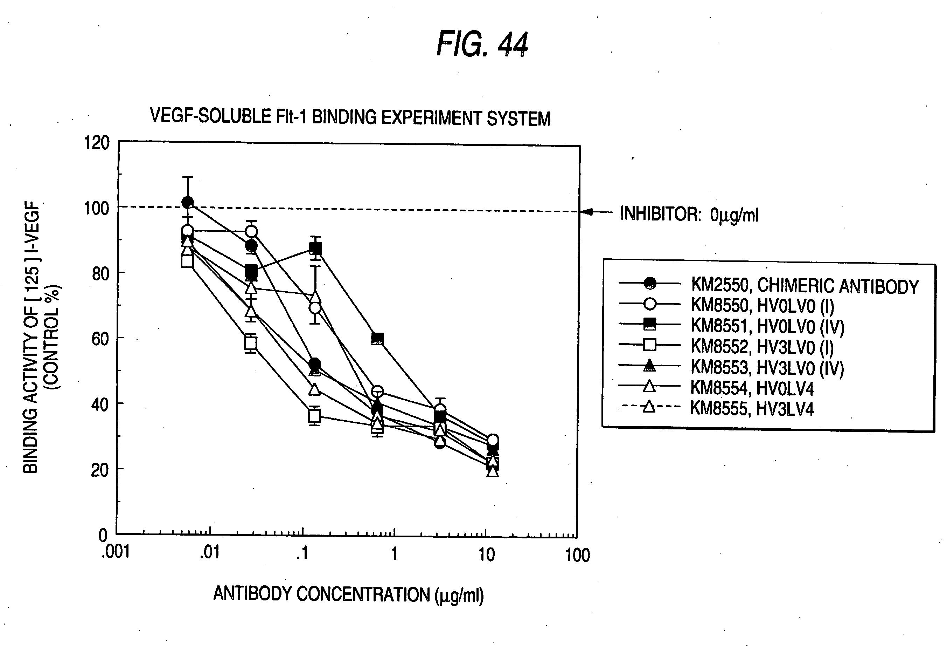

Next, the activity of anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 to inhibit binding between human VEGF and human VEGF receptor flt-1 was examined in accordance with the method described in 2(5) of Example 1. Concentration of each antibody to be added was varied from 0.0048 to 15 µg/ml (5-fold dilutions). The results are shown in Fig. 44. As shown in Fig. 44, anti-human VEGF receptor Flt-1 human CDR-grafted antibodies KM8550, KM8551, KM8552, KM8553, KM8554 and KM8555 and anti-human VEGF receptor Flt-1 human chimeric antibody KM2550 inhibited the binding between human VEGF and human VEGF receptor Flt-1 in an antibody concentration dependent manner. In consequence, it was revealed that the six human CDR-grafted antibodies of the present invention also keep the VEGF-Flt-1 binding inhibition activity of human chimeric antibody KM2550.

### Reference Example 1

### 1. Preparation of antigen

### (1) Construction of soluble human VEGF receptor Flt-1 3N expression vector

A vector was produced in the following manner, for use in the expression of a soluble human VEGF receptor Flt-1 fragment (hereinafter referred to as "soluble human VEGF receptor Flt-1 3N") which corresponds to a region of the 1st to 338th amino acids (including a signal sequence) from the N-terminal of human VEGF receptor Flt-1. The soluble human VEGF receptor Flt-1 3N corresponds to the three immunoglobulin-like regions from the N-terminal of the extracellular domain of the soluble human VEGF receptor Flt-1.

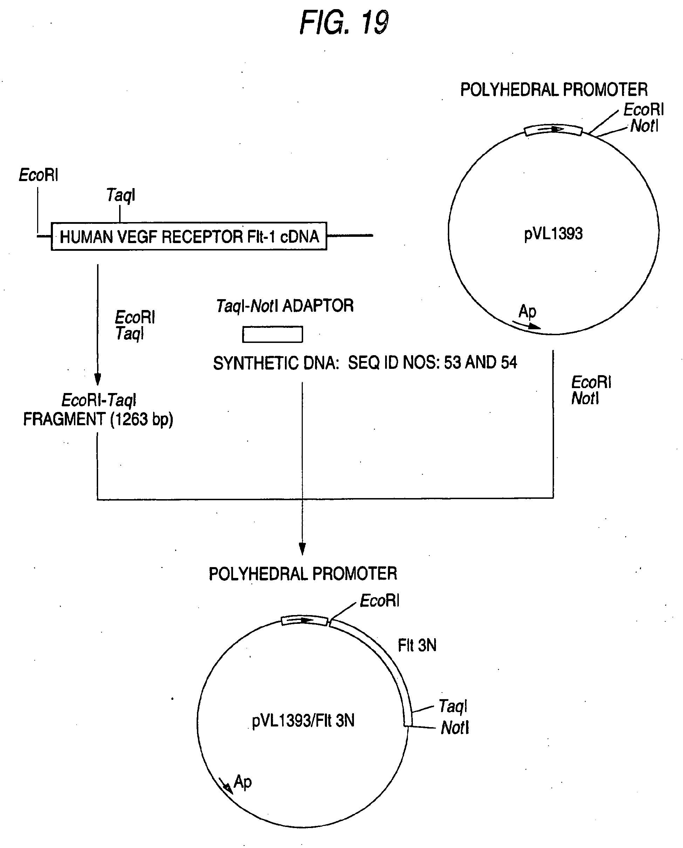

A cDNA clone flt#3-7 [M. Shibuya et al., Oncogene, 5: 519 (1990)] which contains full length cDNA encoding the human VEGF receptor Flt-1 was partially digested with restriction enzymes *Eco*RI and *Taq*I to collect a 1,263 bp *Eco*RI-*Taq*I DNA fragment from the 5'-end, and the thus collected fragment was inserted into the 5' side *Eco*RI site and 3' side *Not*I site downstream of the transcription initiation point of the polyhedrin gene of a baculovirus gene recombinant vector pVL1393 plasmid (manufactured by In Vitrogen) using a *Taq*I*-Not*I adapter into which a termination codon had been artificially introduced (a synthetic DNA fragment having the nucleotide sequences shown in the SEQ ID NOS:53 and 54) to obtain soluble human VEGF receptor Flt-1 3N expression vector pVL1393/Flt 3N (Fig. 19).

### (2) Construction of soluble human VEGF receptor Flt-1 7N expression vector

A vector was produced in the following manner, for use in the expression of a soluble human VEGF receptor Flt-1 fragment (referred to as "soluble human VEGF receptor Flt-1 7N" hereinafter) which corresponds to a region of the 1st to 750th amino acids (including a signal sequence) from the N-terminal of human VEGF receptor Flt-1. The soluble human VEGF preceptor Flt-1 7N corresponds to the seven immunoglobulin-like regions of the extracellular domain of the soluble human VEGF receptor Flt-1.

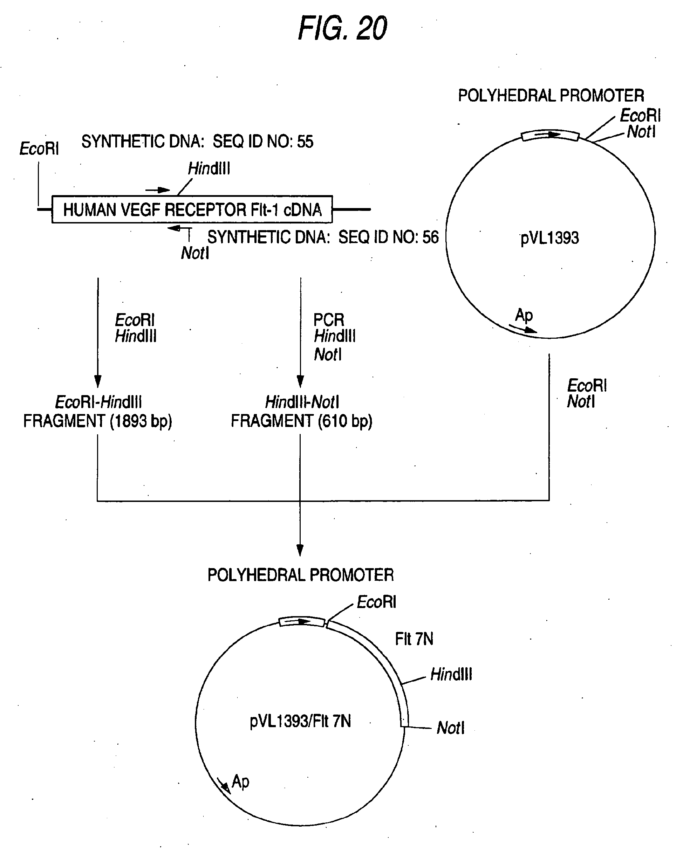

A 2.5 unit portion, of Taq polymerase was added to 100 µl of 0.001% (w/v) gelatin solution of 10 mM MgCl₂ containing 10 pmol of primers having the nucleotide sequences shown in SEQ ID NOS:55 and 56, 10 ng of flt#3-7 clone [Oncogene, 5: 519 (1990)] DNA and 10 mM deoxynucleotide triphosphates. The polymerase chain reaction (PCR) was repeated 30 times in which one reaction consisted, after pretreatment at 95°C for 5 minutes, of treatments at 95°C for 90 seconds, at 50°C for 90 seconds and finally at 72°C for 90 seconds, subsequently collecting a DNA fragment. The DNA fragment was digested with *Hind*III (the 1893 bp position in the flt#3-7 clone) and *Not*I to obtain a 610 bp *Hind*III-*Not*I DNA fragment, namely a DNA fragment containing a 1894-2499 bp fragment of the flt#3-7 clone, termination codon and *Not*I recognition sequence. Next, the flt#3-7 clone was digested with restriction enzymes *Eco*RI and *Hind*III to collect an *Eco*RI-*Hind*III fragment of 1893 bp from the 5'-end. The 610 bp *Hind*III-*Not*I DNA fragment and the 1893 bp *Eco*RI-*Hind*III fragments were then inserted into the 5' side *Eco*RI site and 3' side *Not*I site downstream of the transcription initiation point of the polyhedrin gene of a baculovirus gene recombinant vector pVL1393 plasmid to produce soluble human VEGF receptor Flt-1 7N expression vector pVL1393/Flt 7.N (Fig. 20).

### (3) Production of recombinant virus for use in the expression of soluble human VEGF receptor Flt-1 in insect cells

For the production of protein by insect cells, it is necessary to produce a recombinant virus into which a gene of interest is integrated, and the production process consists of a step in which a cDNA molecule encoding a protein of interest is inserted into a special plasmid, which is called a transfer vector, and a subsequent step in which a wild type virus and the transfer vector are co-transfected into insect cells to obtain a recombinant virus by homologous recombination. These steps were carried out in the following manner using BaculoGold Starter Kit manufactured by Pharmigen (Product No. PM-21001K) in accordance with the attached manual.

A recombinant baculovirus was produced in the following manner by introducing a filamentous baculovirus DNA. (BaculoGold baculovirus DNA, manufactured by Pharmigen) and the thus produced transfer vector DNA into insect cells Sf9 (manufactured by Pharmigen) which had been cultured using TMN-FH insect medium (manufactured by Pharmigen), using a lipofectin method [Protein, Nucleic Acid, Enzyme, 37: 2701 (1992)].

A 1 µg portion of pVL1393/Flt7N produced in (2) or pVL1393/Flt3N produced in (1) and 20 ng of filamentous baculovirus DNA were dissolved in 12 µl of distilled water, the solution was mixed with a mixture of 6 µl lipofectin and 6 µl distilled water and then the resulting mixture was allowed to stand at room temperature for 15 minutes. Separately from this, 1×10⁶ of Sf9 cells were suspended in 2 ml of Sf900-II medium (manufactured by Gibco) and put into a cell culture plastic Petri dish of 35 mm in diameter. To this was added whole volume of the just described solution of plasmid DNA, filamentous baculovirus DNA and lipofectin mixture, followed by 3 days of culturing at 27°C to collect 1 ml of the culture supernatant containing the recombinant virus. A 1 ml portion of fresh Sf900-II medium was added to the resulting Petri dish and 3 days of culturing was carried out at 27°C to obtain an additional 1.5 ml of the culture supernatant containing the recombinant virus.

Next, the thus obtained recombinant virus for use in the protein expression was grown in the following manner.

A 2×10⁷ portion of Sf9 cells were suspended in 10 ml of Sf900-II medium, put into a 175 cm² flask (manufactured by Greiner) and allowed to stand at room temperature for 1 hour to adhere the cells to the flask. The supernatant fluid was subsequently discarded and 15 ml of fresh TMN-FH insect medium and a 1 ml portion of the culture supernatant containing the recombinant virus described above were added and cultured for 3 days at 27°C. After the culturing, the supernatant fluid was centrifuged at 1,500 × g for 10 minutes to remove the cells to obtain a recombinant virus solution for use in the protein expression.

The titer of virus in the thus obtained recombinant virus solution was calculated by the method described in BaculoGold Starter Kit Manual (manufactured by Pharmigen).

A 6×10⁶ portion of Sf9 cells were suspended in 4 ml of Sf900-II medium, put into a cell culture plastic Petri dishes of 60 mm in diameter and allowed to stand at room temperature for 1 hour to adhere the cells to the dish. Next, the supernatant fluid was discarded, 400 µl of fresh Sf900-II medium and the above-described recombinant virus solution diluted 10,000 times with Sf900-II medium were added to the dish and allowed to stand at room temperature for 1 hour, the medium was removed and then 5 ml of a medium containing 1% low melting point agarose (Agarplaque Agarose, manufactured by Pharmigen) (produced by mixing 1 ml of sterilized 5%, Agarplaque plus agarose aqueous solution with 4 ml of TMN-FH insect medium and stored at 42°C) was poured into the dish. After standing at room temperature for 15 minutes, the dish was tied with a vinyl tape to prevent drying, put into a sealable plastic container and then cultured at 27°C for 6 days. A 1 ml portion of PBS containing 0.01% of Neutral Red was added to the dish to carry out the additional culturing for 1 day and then the number of the thus formed plaques was counted. By the above procedure, it was found that each of the recombinant virus solutions contained virus particles of about 1×10⁷ PFU per ml.

### (4) Expression of soluble human VEGF receptors Flt-1 7N and Flt-1 3N in insect cells and purification thereof

Soluble human VEGF receptors Flt-1 7N and Flt-1 3N were obtained in the following manner. A 4×10⁷ portion of High Five cells were suspended in 30 ml of EX-CELL™ 400 medium (manufactured by JRH Biosciences) contained in a 175 cm² flask (manufactured by Greiner) and allowed to stand at room temperature for 1 hour to adhere the cells to the flask. A 1 ml portion of a solution containing about 1×10⁸ to 3×10⁸ PFU/ml of recombinant virus particles obtained in (3) derived from the transfer vectors pVL1393/Flt 7N and pVL1393/Flt 3N was added to the flask to carry out infection at room temperature for 2 hours. The culture supernatant was removed and 30 ml of fresh EX-CELL^{™} 400 medium was added to carry out 3 to 4 days of culturing at 27°C. After completion of the culturing, the culture supernatant was collected and centrifuged at 1,500 × g for 10 minutes to obtain a supernatant fluid.

A column was packed with about 60 ml of heparin-Sepharose CL-6B gel (manufactured by Pharmacia Biotech AB) and washed with 600 ml of 20 mM Tris-HCl (pH 7.5) buffer at a flow rate of 0.5 ml/minute. After the washing, 1,000 ml of the culture medium containing soluble human VEGF receptors Flt-1 7N and Flt-1 3N, which had been prepared in the above-described manner, was passed through the heparin-Sepharose CL-6B column at a flow rate of 0.5 ml/minute. After washing with 600 ml of 20 mM Tris-HCl (pH 7.5) buffer at a flow rate of 0.5 ml/minute, 600 ml of 20 mM Tris-HCl (pH 7.5) buffer having a density gradient of 0 M to 1.1 M NaCl was passed through the column to carry out elution of the proteins adsorbed to the heparin-Sepharose, and the eluate was fractionated in 8 ml portions. Proteins contained in each fraction were analyzed by SDS polyacrylamide gel electrophoresis (SDS-PAGE), and 60 to 80 ml of fractions containing soluble human VEGF receptors Flt-1 7N and Flt-1 3N were collected and concentrated using CentriPrep 10 (manufactured by Amicon). After the concentration, soluble human Flt-1 7N and Flt-1 3N were obtained as solutions of 5 ml and 13 ml, respectively (protein concentrations were 331 µg/ml and 204 µg/ml).

### (5) Confirmation of the purity of soluble human VEGF receptors Flt-1 7N and Flt-1 3N

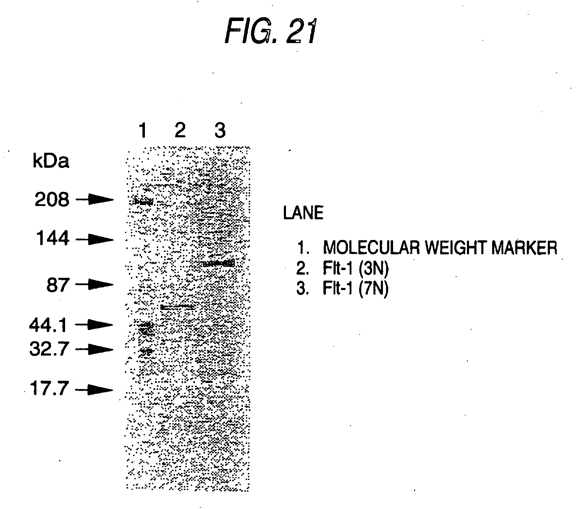

Purity of the thus purified soluble human VEGF receptors Flt-1 7N and Flt-1 3N was confirmed by SDS-PAGE. The SDS-PAGE was carried out in accordance with a known method [Anticancer Research, 12: 1121 (1992)]. Using a 5 to 20% gradient gel (manufactured by Atto) as the gel, electrophoresis of Flt-1 7N and Flt-1 3N, each 2 µg as protein per.lane, was carried out under reducing conditions, and the resulting gel was stained with Coomassie Brilliant Blue. The results are shown in Fig. 21. Purity of Flt-1 7N and Flt-1 3N was found to be 95% or more.

### (6) Purification of control antigen protein of soluble human VEGF receptors Flt-1 7N and Flt-1 3N

The control antigen protein (negative control protein) of soluble human VEGF receptors Flt-1 7N and Flt-1 3N was obtained in the following manner. A 4×10⁷ portion of High Five cells were suspended in 30 ml of EX-CELL^{™} 400 medium (manufactured by JRH Biosciences) contained in a 175 cm² flask (manufactured by Greiner), allowed to stand at room temperature for 1 hour to adhere the cells to the flask and then cultured at 27°C for 3 to 4 days. After completion of the culturing, the culture supernatant was collected and centrifuged at 1,500 × g for 10 minutes to obtain a supernatant fluid.

A column was packed with about 20 ml of heparin-Sepharose CL-6B gel (manufactured by Pharmacia Biotech AB) and washed with 200 ml of 20 mM Tris-HCl (pH 7.5) buffer at a flow rate of 0.5 ml/minute. After the washing, 500 ml of the culture medium of High Five cells was passed through the heparin-Sepharose CL-6B column at a flow rate of 0.5 ml/minute. After washing with 200 ml of 20 mM Tris-HCl (pH 7.5) buffer at a flow rate of 0.5 ml/minute, 200 ml of 20 mM Tris-HCl (pH 7.5) buffer containing 1 M NaCl was passed through the column to carry out elution of the protein adsorbed to the heparin-Sepharose. The 1 M NaCl elution fraction was concentrated using CentriPrep 10 (manufactured by Amicon) to obtain 7 ml of the control antigen protein (867 µg/ml as protein concentration).

### (7) Confirmation of human VEGF binding activity of soluble human VEGF receptors Flt-1 7N and Flt-1 3N

The human VEGF binding activity of soluble human VEGF receptors Flt-1 7N and Flt-1 3N was confirmed in the following manner.

Methanol was dispensed in 100 µl portions into wells of a 96 well Immobilon^{™}-P Filtration Plate (manufactured by Millipore) to give a hydrophilic nature to the PVDF membrane on the bottom of the plate. After washing with water, the soluble human Flt-1 7N diluted with PBS to a concentration of 2 µg/ml was dispensed in 50 µl/well portions and allowed to stand overnight at 4°C for its adsorption. After washing, PBS containing 1% bovine serum albumin (BSA) was dispensed in 100 µl/well portions and the reaction was carried out at room temperature for 1 hour to block the remained active residues. After washing with PBS, each of the purified soluble human VEGF receptors Flt-1 7N and Flt-1 3N' obtained in (4) was dispensed in 50 µl/well portions (final concentration, 1 to 1,000 ng/ml) and then ¹²⁵I-labeled human VEGF (final concentration, 3 ng/ml: manufactured by Amersham) was dispensed in 50 µl/well portions, subsequently carrying out the reaction at room temperature for 1.5 hours. After washing with 0.05% Tween-PBS, the wells were dried at 50°C, and Microscinti-O (manufactured by Packard) was dispensed in 20 µl/well portions to measure the radioactivity of the ¹²⁵I-labeled human VEGF bound to each well using Top Count (manufactured by Packard).

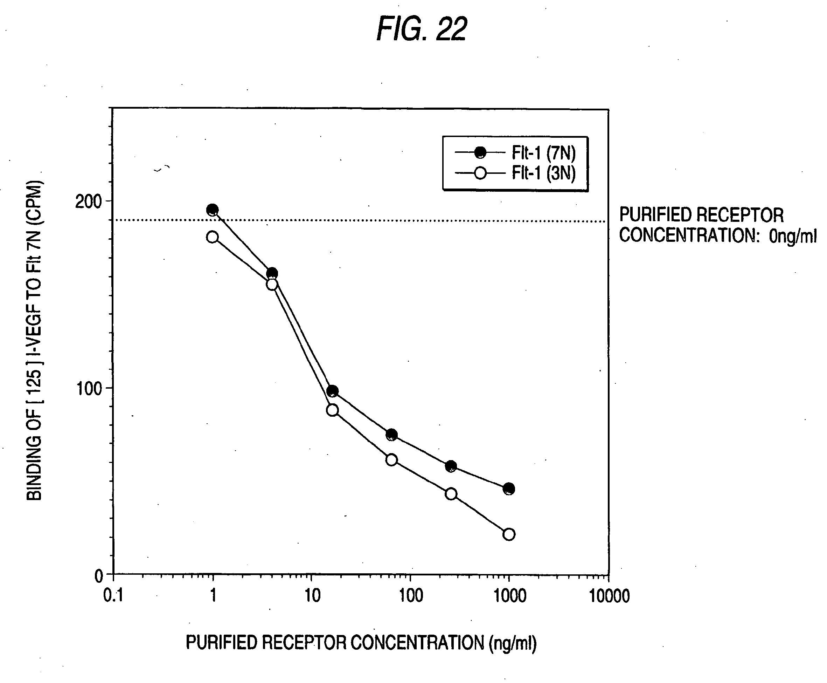

The results are shown in Fig. 22. It was shown that soluble human VEGF receptors Flt-1 7N and Flt-1 3N inhibit binding of ¹²⁵I-labeled human VEGF to soluble human VEGF receptor Flt-1 7N in a concentration dependent manner. Since the soluble human VEGF receptors Flt-1 7N and Flt-1 3N showed similar degree of the human VEGF binding activity, it was revealed that the human VEGF binds to the Flt-1 3N moiety (the 1st to 338th amino acids from the N-terminal including signal sequence).

### (8) Expression of human VEGF in insect cells

The human VEGF was obtained in the following manner. A 4×10⁷ portion of High Five cells were suspended in 30 ml of EX-CELL^{™} 400 medium (manufactured by JRH Biosciences) contained in a 175 cm² flask (manufactured by Greiner) and allowed to stand at room temperature for 1 hour to adhere the cells to the flask. A 1 ml portion of a solution containing about 1×10⁸ to 3×10⁸ PFU/ml of human VEGF recombinant baculovirus particles obtained in accordance with the known method [Cell Growth & Differentiation, 7: 213 (1996)] was added to the flask to carry out infection at room temperature for 2 hours. The culture supernatant was removed and 30 ml of fresh EX-CELL^{™} 400 medium was added to carry out 3 to 4 days of culturing at 27°C. After completion of the culturing, the culture supernatant was collected and centrifuged at 1,500 × g for 10 minutes to obtain a supernatant fluid.

A column was packed with about 40 ml of heparin-Sepharose CL-6B gel (manufactured by Pharmacia Biotech AB) and washed with 400 ml of 20 mM Tris-HCl (pH 7.5) buffer at a flow rate of 0.5 ml/minute. After washing, 1,500 ml of the culture medium containing human VEGF prepared in the above-described manner was passed through the heparin-Sepharose CL-6B column at a flow rate of 0.5 ml/minute. After washing with 400 ml of 20 mM Tris-HCl (pH 7.5) buffer at a flow rate of 0.5 ml/minute, 120 ml of each of 20 mM Tris-HCl (pH 7.5) buffers containing 0.2 M, 0.5 M and 1 M NaCI was passed through the column in that order to carry out stepwise elution of the proteins adsorbed to the heparin-Sepharose, and the eluate was fractionated in 8 ml portions. Proteins contained in each fraction were analyzed by SDS polyacrylamide gel electrophoresis, and 120 ml of fractions (0.5 to 1 M NaCl fractions) containing human VEGF were collected. After concentration using centriPrep-10 (manufactured by Amicon), human VEGF was obtained as 4 ml of solution (protein concentration, 1.2 mg/ml).

### 2. Immunization of animals and preparation of antibody-producing cells

A 50 µg portion of each of the antigens obtained in 1(4) was administered, together with 2 mg of aluminum hydroxide gel and 1×10⁹ cells of pertussis vaccine (manufactured by Chiba Serum Institute), into 5-week-old female BALB/c mice (SLC Japan), B6C3F1 mice (Charles River Japan) or female SD rats (SLC Japan), and, starting on 2 weeks thereafter, 10 to 50 µg of the protein was administered once a week for a total of four times. Also, 1×10⁷ of NIH3T3-Flt-1 cells were administered 6 times into three, 5 week old female BALB/c (SLC Japan) mice. Blood samples were collected from the fundus of the eye or the caudal vein, their serum antibody titers were examined by the enzyme immunoassay described in the following, and spleens were excised from mice or rats showing sufficient antibody titer 3 days after the final immunization. In this connection, immunization was not induced in the 5-week-old female BALB/c to which NIH3T3-Flt-1 cells were administered, so that the antibody titer upon soluble Flt-1 7N was not increased.

The thus excised spleen was cut to pieces in MEM medium (manufactured by Nissui Pharmaceutical), unbound using a pair of forceps and then centrifuged (1,200 rpm for 5 minutes). The resulting supernatant was discarded, and the thus obtained sediment was treated with Tris-ammonium chloride buffer (pH 7.65) for 1 to 2 minutes to eliminate erythrocytes, washed three times with MEM medium and used in cell fusion.

### 3. Enzyme immunoassay

With regard to the measurement of antisera derived from mice or rats immunized with the soluble human Flt-1 7N and Flt-1 3N obtained in 1(4) and culture supernatants of hybridomas, the soluble human VEGF receptors Flt-1 7N and Flt-1 3N obtained from the insect cell culture supernatant of 1(4) were used as antigens. A 1 to 10 µg/ml PBS-diluted solution of each of the soluble human VEGF receptors Flt-1 7N and Flt-1 3N and the heparin column adsorption fraction of High Five cell culture supernatant obtained in 1(6) as a control antigen was dispensed in 50 µl/well portions into a 96 well plate for EIA (manufactured by Greiner) and allowed to stand overnight at 4°C for coating. After washing, PBS containing 1% bovine serum albumin (BSA) was dispensed in 100 µl/well portions and the reaction was carried out at room temperature for 1 hour to block the remained active residues. After discarding 1% BSA-PBS, antiserum of immunized mouse or immunized rat and culture supernatant of a hybridoma were dispensed in 50 µl/well portions to carry out the reaction for 2 hours. After washing with 0.05% Tween-PBS, peroxidase-labeled rabbit anti-mouse immunoglobulin or peroxidase-labeled rabbit anti-rat immunoglobulin (both manufactured by DAKO) was dispensed in 50 µl/well portions and the reaction was carried out at room temperature for 1 hour, the plate was washed with 0.05% Tween-PBS and then color development was caused using ABTS substrate solution [2,2-azinobis(3-ethylbenzothiazole-6-sulfonic acid) ammonium salt] to measure maximum absorbance at OD415nm using E max (manufactured by Molecular Devices).

### 4. Preparation of mouse myeloma cells

8-Azaguanine-resistant mouse myeloma cell line P3U1 was cultured using normal medium to secure 2×10⁷ or more of the cells for use in cell fusion as the parent cell line.

### 5. Production of hybridoma

The mouse spleen cells or rat spleen cells obtained in 2 and the myeloma cells obtained in 4 were mixed to a ratio of 10:1 and centrifuged (1,200 rpm for 5 minutes), the supernatant was discarded, the precipitated cells were thoroughly loosened to which, while stirring at 37°C, were subsequently added a mixed solution of 2 g polyethylene glycol-1000 (PEG-1000), 2 ml MEM medium and 0.7 ml DMSO in an amount of 0.2 to 1 ml/10⁸ mouse myeloma cells and then 1 to 2 ml of MEM medium several times at 1 to 2 minute intervals, and then the total volume was adjusted to 50 ml by adding MEM medium. After centrifugation (900 rpm for 5 minutes), the supernatant was discarded and the thus obtained cells were gently loosened and then gently suspended in 100 ml of HAT medium by repeated drawing up into and discharging from a graduated pipette.

The suspension was dispensed in 100 µl portions into wells of a 96 well culture plate and cultured at 37°C for 10 to 14 days in an atmosphere of 5% CO₂ in a 5% CO₂ incubator. The resulting culture supernatant was examined by the enzyme immunoassay described in 2(3) of Example 1 to select wells which reacted specifically with the soluble human VEGF receptor Flt-1 7N or Flt-1 3N obtained in 1(4) but did not react with the control antigen obtained in 1(6), and then cloning was repeated twice by changing the medium to HT medium and normal medium to establish hybridomas capable of producing anti-human VEGF receptor Flt-1 monoclonal antibodies. The results are shown below.

**Table 3**

| Animal | Head | Immunogen | Screening source | Wells screened | The number of hybridomas established |
|---|---|---|---|---|---|
| Balb/c mouse | 3 | NIH3T3-Flt-1 | Flt 7N | - | - |
| | | | | | |
| SD rat | 1 | Flt 7N | Flt 7N | 1008 | 3 (KM1733,1735,1736) |
| | | | | | |
| Balb/c mouse | 1 | Flt 7N | Flt 7N | 672 | 5 (KM1737,1739,1740, 1742,1743) |
| | | | | | |
| SD rat | 1 | Flt 7N | Flt 7N | 1176 | 3 (KM1745,1746,1747) |
| | | | | | |
| B3,C3F1 mouse | 1 | Flt 7N | Flt 3N | 672 | 3 (KM1748,1749,1750) |
| | | | | | |
| Balb/c mouse | 1 | Flt 7N | Flt 3N | 420 | 3 (KM1730,1731,1732) |

When hybridomas obtained from one Balb/c mouse and two SD rats immunized with the soluble human VEGF receptor Flt-1 7N obtained in 1 (4) were screened for about 672 wells and about 2,184 wells, respectively, using the soluble human VEGF receptor Flt-1 7N, respective 5 clones and 6 clones of anti-human VEGF receptor Flt-1 monoclonal antibodies were obtained, and they were named KM1737, KM1739, KM1740, KM1742 and KM1743 and KM1733, KM1735, KM1736, KM1745, KM1746 and KM1747, respectively. None of these clones showed the activity to inhibit binding of human VEGF to Flt-1 as shown 8. Additionally, KM1735, KM1736, KM1742, KM1743 and KM1745 reacted with human VEGF receptor Flt-1 expression cells by the immunocyte staining method described in the following 10, but the reaction was extremely weak in comparison with KM1730, KM1731 and KM1732.

On the other hand, when hybridomas obtained from one B3C3F1 mouse and one Balb/c mouse immunized with the soluble human VEGF receptor Flt-1 7N obtained in 1(4) were screened for about 672 wells and about 420 wells, respectively, using the soluble human VEGF receptor Flt-1 3N, 3 clones for each of anti-human VEGF receptor Flt-1 monoclonal antibodies were obtained, and they were named KM1748, KM1749 and KM1750 and KM1730, KM1731 and KM1732, respectively. Of these clones, three clones KM1732, KM1748 and KM1750 showed the activity to inhibit binding of human VEGF to Flt-1 as shown in the following 8. Additionally, three clones KM1730, KM1731 and KM1732 reacted markedly strongly with human VEGF receptor Flt-1 expression cells by the immunocyte staining method described in the following 10.

The antibody class of these monoclonal antibodies was determined by enzyme immunoassay using Subclass Typing Kit (manufactured by Zymed). The results are shown in the following Table 4.

**Table 4**

| Monoclonal antibody | Antibody subclass |
|---|---|
| KM1733 | mouse IgG2a |
| KM1735 | rat IgG1 |
| KM1736 | rat IgG2a |
| KM1737 | mouse IgG1 |
| KM1739 | mouse IgG1 |
| KM1740 | mouse IgG1 |
| KM1742 | mouse IgG1 |
| KM1743 | mouse IgG1 |
| KM1745 | rat IgG2a |
| KM1746 | rat IgG1 |
| KM1747 | rat IgG1 |
| KM1748 | mouse IgG2b |
| KM1749 | mouse IgG1 |
| KM1750 | mouse IgG2b |
| KM1730 | mouse IgG1 |
| KM1731 | mouse IgG2a |
| KM1732 | mouse IgG1 |

All of the monoclonal antibodies established in the present invention were IgG class.

### 6. Purification of monoclonal antibody

The hybridomas obtained in 5 were respectively administered to pristane-treated female nude mice (Balb/c) of 8 weeks of age by intraperitoneal injection at a dose of 5×10⁶ to 20×10⁶ cells per animal. The hybridomas caused ascites tumor formation in 10 to 21 days. The ascitic fluid was collected from each ascitic fluid-carrying mouse (1 to 8 ml per animal), centrifuged (3,000 rpm for 5 minutes) for removing solid matter and then purified by a caprylic acid precipitation method (antibodies - A Laboratory Manual).

### 7. Confirmation of the specificity of monoclonal antibodies

Specificity of the anti-human VEGF receptor Flt-1 monoclonal antibodies described in 5 was confirmed using the enzyme immunoassay described in 3.

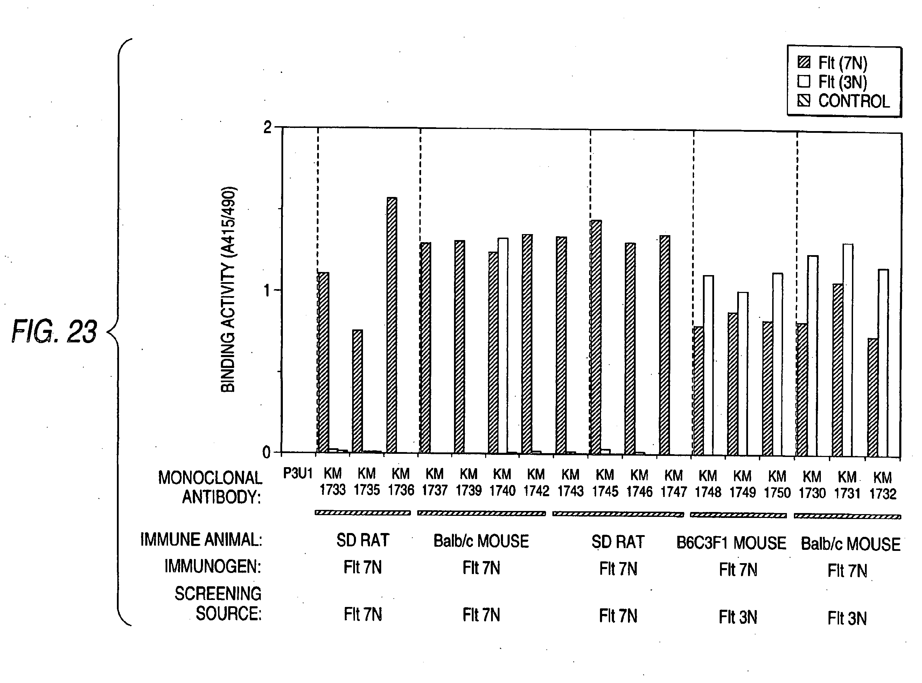

The results are shown in Fig. 23. Among the monoclonal antibodies obtained by.. producing hybridomas from mice and rats immunized with Flt-1 7N and selected using Flt-1 7N (KM1733, KM1735, KM1736, KM1737, KM1739, KM1740, KM1742, KM1743, KM1745, KM1746 and KM1747), only KM1740 reacted with Flt-1 7N and Flt-1 3N, revealing that it recognizes an epitope which is present in a region of the 1st to 338th amino acids from the N-terminal amino acid of Flt-1 (including signals sequence). Since the remaining 10 clones reacted with Flt-1 7N but not with Flt-1 3N, it was revealed that they recognize an epitope which is present in a region of the 339th to 750th amino acids from the N-terminal of Flt-1 (including signal sequence). On the other hand, since all of the monoclonal antibodies obtained by producing hybridomas from mice immunized with Flt-1 7N and selecting using Flt-1 3N (KM1748, KM1749, KM1750, KM1730, KM1731 and KM1732) reacted with Flt-1 7N and Flt-1 3N, it was revealed that they recognize an epitope which is present in a region of the 1st to 338th amino acids from the N-terminal amino acid of Flt-1 (including signal sequence).

### 8. Confirmation of the activity of anti-Flt-1 monoclonal antibodies to inhibit binding of a human VEGF to a human VEGF receptor Flt-1

The activity of the anti-human VEGF receptor Flt-1 monoclonal antibodies described in 5 to inhibit binding of human VEGF to human VEGF receptor Flt-1 was confirmed in the following manner.

Methanol was dispensed in 100 µl portions into wells of a 96 well MultiScreen-IP Plate (manufactured by Millipore) to give hydrophilic nature to the PVDF membrane on the bottom of the plate. After washing with water, the soluble human VEGF receptor Flt-1 7N diluted with PBS to a concentration of 1.6 µg/ml was dispensed in 50 µl/well portions and then allowed to stand overnight at 4°C for its adsorption. After washing, PBS containing 1% bovine serum albumin (BSA) is dispensed in 50 µl/well portions and the reaction was carried out at room temperature for 1 hour to block the remained active residues. After washing with PBS, each hybridoma culture supernatant or a purified monoclonal antibody diluted with 1% BSA-PBS containing 0.5 M NaCl (0.01 to 7.29 µg/ml) was dispensed in 50 µl/well portions and then 3 ng/ml of ¹²⁵I-labeled human VEGF (manufactured by Amersham) was dispensed in 50 µl/well portions, subsequently carrying out the reaction at room temperature for 1.5 hours. After washing with 0.05% Tween-PBS, the wells were dried at 50°C, and Microscinti-O (manufactured by Packard) was dispensed in 30 µl/well portions to measure the radioactivity of the ¹²⁵I-labeled human VEGF linked to each well using Top Count (manufactured by Packard).

Results of the examination of activities of hybridoma culture supernatants are shown in Fig.. 24. Among 17 established monoclonal antibodies, three monoclonal antibodies, KM1748, KM1750 and KM1732 inhibited binding of human VEGF to human VEGF receptor Flt-1 at inhibition ratios of 62.6%, 66.3% and 83.1%, respectively.

In general, screening of monoclonal antibody producing hybridomas is carried out using the same protein as the antigen used as the immunogen. A total of 11 monoclonal antibodies selected using Flt-1 7N as the immunogen showed no binding inhibition activity, and, among 6 monoclonal antibodies selected using Flt-1 3N (KM1748, KM1749, KM1750, KM1730, KM1731 and KM1732), KM1748, KM1750 and KM1732 showed the binding inhibition activity. It was an unexpected effect that monoclonal antibodies having the binding inhibition activity were obtained by the use of Flt-1 3N in the screening of hybridomas. Thus, it was revealed that Flt-1 3N is markedly important in establishing monoclonal antibodies having the binding inhibition activity.

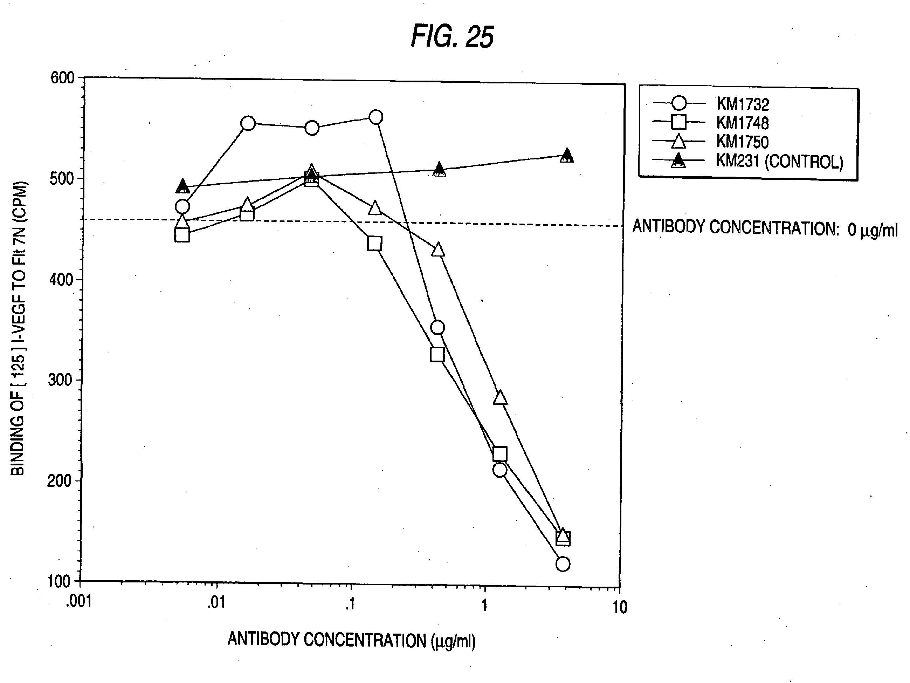

Fig. 25 shows results of the examination of binding inhibition activity using purified anti-Flt-1 monoclonal antibodies KM1732, KM1748 and KM1750. These antibodies KM1732, KM1748 and KM1750 inhibited binding of human VEGF to human VEGF receptor Flt-1 in a concentration dependent manner. Concentrations of KM1732, KM1748 and KM1750, which indicate 50% inhibition of the binding of human VEGF to human VEGF receptor Flt-1 (IC₅₀), were 1.1, 1.3 and 2.0 µg/ml, respectively. On the other hand, an anti-sialyl-Lewis A monoclonal antibody KM231 of a mouse IgG1 class [Anticancer Research, 10: 1579 (1990)] used as the control showed no inhibition activity.

### 9. Confirmation of the activity of anti-Flt-1 monoclonal antibodies to inhibit binding of human VEGF to human VEGF receptor Flt-1 expression cells

The activity of the anti-human VEGF receptor Flt-1g monoclonal antibodies KM1732, KM1748 and KM1750 to inhibit binding of human VEGF to human VEGF receptor Flt-1 was confirmed in the following manner.

PBS containing 1% bovine serum albumin (BSA) was dispensed in 100 µl portions into wells of a 96 well MultiScreen-HV Plate (manufactured by Millipore), the reaction was carried out at room temperature for 1 hour to block the active residues the wells and then NIH3T3-Flt-1 cells suspended in 1% BSA-PBS containing 0.05% NaN₃ were dispensed in 5×10⁴ cells/well portions. After washing with 1% BSA-PBS, a purified monoclonal antibody (0.01 to 7.29 µg/ml) was dispensed in 50 µl/well portions and then 3 ng/ml of ¹²⁵I-labeled human VEGF (manufactured by Amersham) was dispensed in 50 µl/well portions and the reaction was carried out under cooling for 2 hours. After washing with PBS, the wells were dried at 50°C, and Microscinti-O (manufactured by Packard) was dispensed in 30 µl/well portions to measure the radioactivity of the ¹²⁵I-labeled human VEGF bound to each well using Top Count (manufactured by Packard).

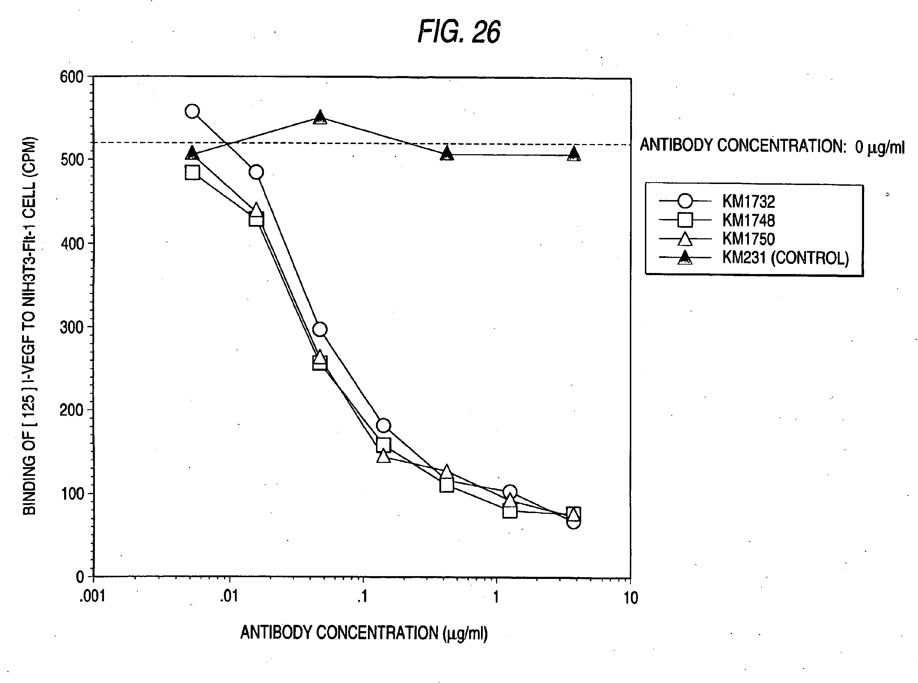

Fig. 26 shows results of the examination of binding inhibition activity using purified anti-Flt-1 monoclonal antibodies KM1732, KM1748 and KM1750. These antibodies KM1732, KM1748 and KM1750 inhibited binding of human VEGF to NIH3T3-Flt-1 cells in a concentration dependent manner. Concentrations of KM1732, KM1748 and KM1750, which indicate 50% inhibition of the binding of human VEGF to NIH3T3-Flt-1 cells (IC₅₀), were 0.050, 0.037 and 0.041 µg/ml, respectively. On the other hand, the anti-sialyl-Lewis A monoclonal antibody K231 of a mouse IgG1 class used as the control showed no inhibition activity.

### 10. Confirmation of the reactivity of monoclonal antibodies with human VEGF receptor Flt-1 expression cells

Specificity of the anti-human VEGF receptor Flt-1 monoclonal antibodies described in 5 was confirmed using immunocyte staining method in accordance the following procedure.

A total of 5×10⁵ cells of each of human VEGF receptor Flt-1 expression NIH3T3 cells (NIH3T3-Flt-1) and control NIH3T3 cells (NIH3T3-Neo) [Oncogene, 10: 135 (1995)] were suspended in 100 µl of a buffer solution for immunocyte staining use (PBS containing 1% BSA, 0.02% EDTA and 0.05% sodium azide) and dispensed in a round bottom 96 well plate. After centrifugation at 4°C and at 350 × g for 1 minute, the supernatant fluid was discarded and the resulting cells were mixed with 50 µl of a hybridoma culture supernatant or purified antibody (10 µg/ml) and reaction was carried out at 4°C for 30 minutes. After the reaction, 200 µl of the buffer solution for immunocyte staining use was added to each well, and the cells were washed by centrifugation at 4°C and 350 × g for 1 minute followed by discarding the resulting supernatant. After repeating this washing step twice, the cells were mixed with 50 µl of the buffer solution for immunocyte staining use containing 1 µg/ml of an FITC-labeled anti-mouse immunoglobulin antibody or FITC-labeled anti-rat immunoglobulin antibody (manufactured by Wako Pure Chemical Industries), and the reaction was carried out at 4°C for 30 minutes. After this reaction, the above-described washing step was repeated three times and then analysis was carried out using Flow Cytometer (manufactured by Coulter).

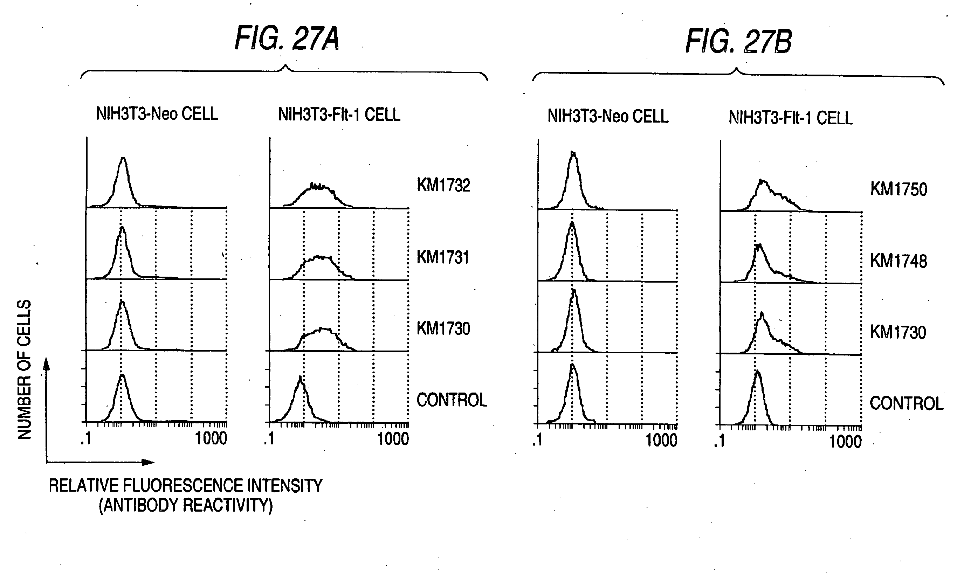

The results are shown in Fig. 27. The anti-human VEGF receptor Flt-1 monoclonal antibodies KM1730, KM1731 and KM1732 did not react with the control cells but specifically reacted in significant amounts with the Flt-1 expression cells (A). Neither the anti-human VEGF receptor Flt-1 monoclonal antibody KM1748 (10 µg/ml) nor the hybridoma culture supernatant KM1748 reacted with the control cells. Each specifically reacted in significant amounts with the Flt-1 expression cells (B). As a result, it was discovered that the monoclonal antibodies KM1730, KM1731, KM1732, KM1748 and KM1750 specifically recognize the human VEGF receptor Flt-1 on the cell surface. On the other hand, KM1735, KM1736, KM1742, KM1743 and KM1745 only weakly reacted with the human VEGF receptor Flt-1 expression cells in comparison with KM1730, KM1731, KM1732, KM1748 and KM1750.

### 11. Detection of human VEGF receptor Flt-1 by Western blotting using monoclonal antibody

Cell membrane components were prepared from NIH3T3-Flt-1 cells and control NIH3T3 cells (NIH3T3-Neo) in accordance with a known method [Cancer Research, 46: 4438 (1986)] and electrophoresed by the SDS-PAGE method. The SDS-PAGE was carried out in accordance with a known method [Anticancer Research, 12: 1121 (1992)] by subjecting 15 µg, as protein per lane, of the cell membrane components to the electrophoresis using a 5 to 20% gradient gel (manufactured by Atto) under reducing conditions. The thus treated proteins were transferred to a PVDF membrane in accordance with a known method [Anticancer Research, 12: 1121 (1992)]. Next, the PVDF membrane was allowed to react with PBS containing 1% BSA at room temperature for 30 minutes to carry out blocking and then to react with the culture supernatant of the anti-human VEGF receptor Flt-1 monoclonal antibody KM1737 overnight at 4°C. The thus treated membrane was washed with PBS containing 0.05% Tween and then allowed to react with peroxidase-labeled goat anti-mouse IgG (5,000 times dilution: manufactured by Chemicon) at room temperature for 2 hours. After washing with 0.05% Tween-containing PBS, bands to which the anti-human VEGF receptor Flt-1 monoclonal antibody KM1737 was bound were detected using ECL^{™} Western blotting detection reagents (manufactured by Amersham).

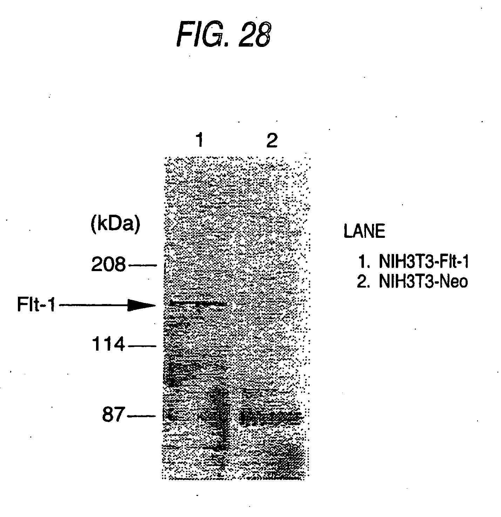

The results are shown in Fig. 28. It was confirmed that the anti-human VEGF receptor Flt-1 monoclonal antibody KM1737 can detect the human VEGF receptor Felt-1 of 180 kilodalton in molecular weight expressed in the NIH3T3-Flt-1 cells.

### 12. Detection of soluble human VEGF receptor Flt-1 using monoclonal antibody

The anti-human VEGF receptor Flt-1 monoclonal antibody KM1732 was diluted with PBS to a concentration of 10 µg/ml and dispensed in 50 µl/well portions into a 96 well plate for EIA (manufactured by Greiner) and allowed to stand overnight at 4°C for coating. After washing, PBS containing 1% bovine serum albumin (BSA) was dispensed in 100 µl/well portions and the reaction was carried out at room temperature for 1 hour to block the remained active residues. After discarding 1% BSA-PBS, the purified soluble human VEGF receptors Flt-1 7N and Flt-1 3N obtained in 1(4) and diluted with 1% BSA-PBS to a concentration of 1,000 to 0.0056 ng/ml were allowed to react with the antibody overnight at 4°C. After washing with 0.05% Tween-PBS, the anti-human VEGF receptor Flt-1 monoclonal antibody KM1730 labeled with biotin by a known method [enzyme Antibody Method: published by Gakusai Kikaku (1985)] was diluted with 1% BSA-PBS to a concentration of 0.1 µg/ml and dispensed in 50 µl/well portions to carry out the reaction at room temperature for 2 hours. After washing with 0.05% Tween-PBS, avidin-labeled peroxidase (manufactured by Vector) diluted 4,000 times with 1% BSA-PBS was dispensed in 50 µl/well portions to carry out the reaction at room temperature for 1 hour. After washing with 0.05% Tween-PBS, color development was caused using ABTS substrate solution [2,2-azinobis(3-ethylbenzothiazole-6-sulfonic acid) ammonium salt] to measure absorbance at OD415 nm using E max (manufactured by Molecular Devices).

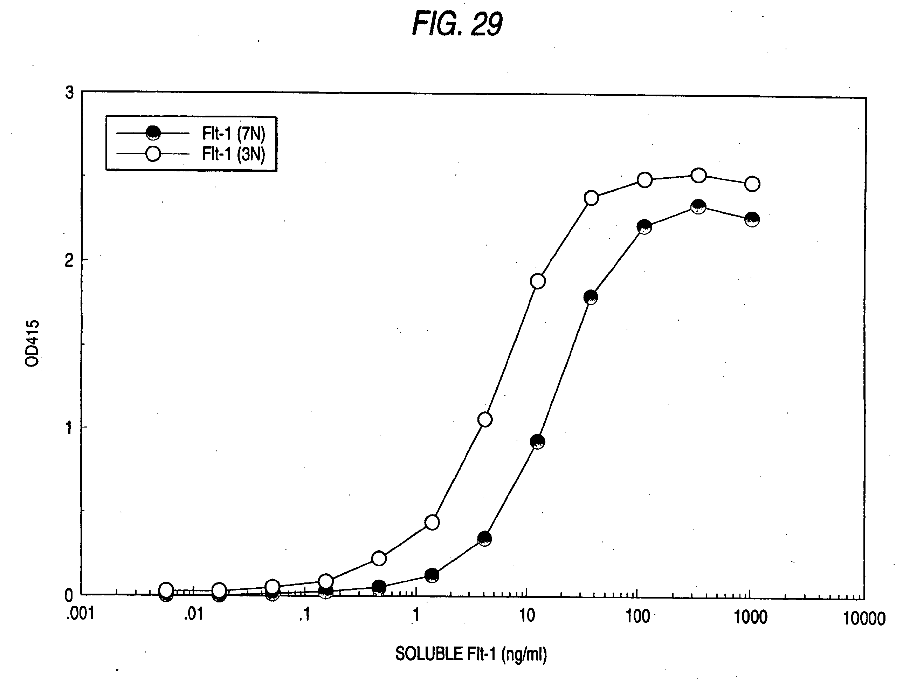

The results are shown in Fig. 29. As a result, it was found that the soluble human VEGF receptors Flt-1 3N and Flt-1 7N can be measured from minimum concentrations of 0.46 ng/ml and 1.37 ng/ml, respectively, by the use of the anti-human VEGF receptor Flt-1 monoclonal antibody KM1732 and the biotin-labeled anti-human VEGF receptor Flt-1 monoclonal antibody KM1730.

### 13. VEGF-dependent cell-migration inhibition test using anti-VEGF receptor.Flt-1 monoclonal antibody

Effect of anti-VEGF receptor Flt-1 monoclonal antibody on the VEGF-dependent migration activity of human vascular endothelial cells, as an index of *in vitro* angiogenesis activity, was examined.

The cell migration test was carried out in accordance with the method of Sato et al. [J. Cell Biology, 107: 1199 (1988)]. HUVEC cultured in a 3.5 cm dish until they became confluent was scratched with a razor's edge and then washed with PBS. A 1.5 ml portion of a 5% FCS-containing M-199 medium was added thereto, and VEGF (final concentration: 10 ng/ml) and the anti-VEGF receptor Flt-1 monoclonal antibody KM1750 or KM1732 obtained in 5 (final concentration: 0, 1 or 10 µg/ml) were further added thereto, followed by culturing for 24 hours. After culturing, the number of cells migrated from the scratched position was measured.

As a result, cell migration ability of HUVEC increased by adding VEGF, but the migration was completely inhibited by adding the anti-VEGF receptor Flt-1 monoclonal antibody KM1750 or KM1732 (final concentration: 1 µg/ml). Thus, it was confirmed that Flt-1 is a main receptor relating to the migration of vascular endothelial cells.

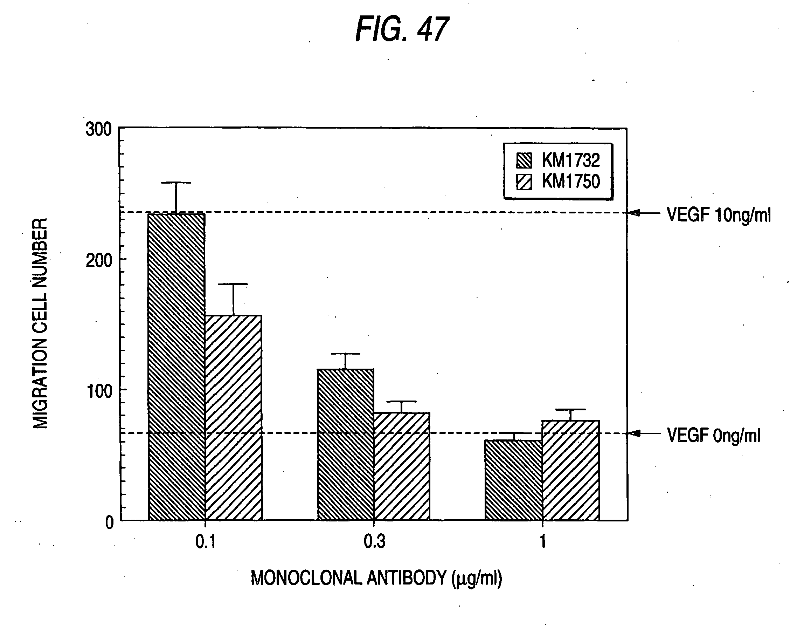

Fig. 47 shows results of the comparison of activities of anti-VEGF receptor Flt-1 monoclonal antibodies KM1750 and KM1732 to inhibit the migration of vascular endothelial cells. Both of the monoclonal antibodies showed the activity to inhibit the migration of vascular endothelial cells in a concentration dependent manner at a monoclonal antibody concentration of 0.1 to 1 µg/ml.

Accordingly, it was found that migration of vascular endothelial cells induced by VEGF is completely inhibited by the anti-VEGF receptor Flt-1 monoclonal antibody.

### Industrial Applicability

The present invention provides gene recombinant antibodies which specifically react with human VEGF receptor Flt-1 which is considered to be expressed in human neovascular endothelial cells. Also, the present invention provides a gene recombinant antibody having activity which neutralizes human VEGF receptor Flt-1. The gene recombinant antibody is useful for diagnosis, treatment of diseases with diabetic retinopathy, such as cancer, and diabetic retinopathy, and especially has lower immunogenicity in human than mouse monoclonal antibodies, and it is expected that the effect lasts for a long time.

### Free Text of Sequence Listings

SEQ ID NO:17-Explanation of artificial sequence: Synthetic DNA
SEQ ID NO:18-Explanation of artificial sequence: Synthetic DNA
SEQ ID NO:19-Explanation of artificial sequence: Synthetic DNA
SEQ ID NO:20-Explanation of artificial sequence: Synthetic DNA
SEQ ID NO:21-Explanation of artificial sequence: Synthetic DNA
SEQ ID NO:22-Explanation of artificial sequence: Synthetic DNA
SEQ ID NO:23-Explanation of artificial sequence: Synthetic DNA
SEQ ID NO:24-Explanation of artificial sequence: Synthetic DNA .
SEQ ID NO:25-Explanation of artificial sequence: Synthetic DNA
SEQ ID NO:26-Explanation of artificial sequence: Synthetic DNA
SEQ ID NO:27-Explanation of artificial sequence: Synthetic DNA
SEQ ID NO:28-Explanation of artificial sequence: Synthetic DNA
SEQ ID NO:29-Explanation of artificial sequence: Synthetic DNA
SEQ ID NO:30-Explanation of artificial sequence: Synthetic DNA
SEQ ID NO:31-Explanation of artificial sequence: Synthetic DNA
SEQ ID NO:32-Explanation of artificial sequence: Synthetic DNA
SEQ ID NO:33-Explanation of artificial sequence: Synthetic DNA
SEQ ID NO:34-Explanation of artificial sequence: Synthetic DNA
SEQ ID NO:35-Explanation of artificial sequence: Synthetic DNA
SEQ ID NO:36-Explanation of artificial sequence: Synthetic DNA
SEQ ID NO:37-Explanation of artificial sequence: Synthetic DNA
SEQ ID NO:38-Explanation of artificial sequence: Synthetic DNA
SEQ ID NO:39-Explanation of artificial sequence: Synthetic DNA
SEQ ID NO:40-Explanation of artificial sequence: Synthetic DNA
SEQ ID NO:41-Explanation of artificial sequence: Synthetic DNA
SEQ ID NO:42-Explanation of artificial sequence: Synthetic DNA
SEQ ID NO:43-Explanation of artificial sequence: Synthetic DNA
SEQ ID NO:44-Explanation of artificial sequence: Synthetic DNA
SEQ ID NO:45-Explanation of artificial sequence: synthetic DNA
SEQ ID NO:46-Explanation of artificial sequence: Synthetic DNA
SEQ ID NO:47-Explanation of artificial sequence: Synthetic DNA
SEQ ID NO:48-Explanation of artificial sequence: Synthetic DNA
SEQ ID NO:49-Explanation of artificial sequence: Synthetic DNA
SEQ ID NO:50-Explanation of artificial sequence: Synthetic DNA
SEQ ID NO:51-Explanation of artificial sequence: Synthetic DNA
SEQ ID NO:52-Explanation of artificial sequence: Synthetic DNA
SEQ ID NO:53-Explanation of artificial sequence: Synthetic DNA
SEQ ID NO:54-Explanation of artificial sequence: Synthetic DNA
SEQ ID NO:55-Explanation of artificial sequence: Synthetic DNA
SEQ ID NO:56-Explanation of artificial sequence: Synthetic DNA
SEQ ID NO:57-Explanation of artificial sequence: Synthetic DNA
SEQ ID NO:58-Explanation of artificial sequence: Synthetic DNA
SEQ ID NO:59-Explanation of artificial sequence: Synthetic DNA
SEQ ID NO:60-Explanation of Artificial sequence: Synthetic DNA
SEQ ID NO:61-Explanation of artificial sequence: Synthetic DNA
SEQ ID NO:62-Explanation of artificial sequence: Synthetic DNA
SEQ ID NO:63-Explanation of artificial sequence: Synthetic DNA
SEQ ID NO:64-Explanation of artificial sequence: Synthetic DNA
SEQ ID NO:65-Explanation of artificial sequence: Synthetic DNA
SEQ ID NO:66-Explanation of artificial sequence: Synthetic DNA
SEQ ID NO:67-Explanation of artificial sequence: Synthetic DNA
SEQ ID NO:68-Explanation of artificial sequence: Synthetic DNA
SEQ ID NO:69-Explanation of artificial sequence: Synthetic DNA
SEQ ID NO:70-Explanation of artificial sequence: Synthetic DNA
SEQ ID NO:71-Explanation of artificial sequence: Synthetic DNA
SEQ ID NO:72-Explanation of artificial sequence: Synthetic DNA
SEQ ID NO:73-Explanation of artificial sequence: Synthetic DNA
SEQ ID NO:74-Explanation of artificial sequence: Synthetic DNA
SEQ ID NO:75-Explanation of artificial sequence: Synthetic DNA
SEQ ID NO:76-Explanation of artificial sequence: Synthetic DNA
SEQ ID NO:77-Explanation of artificial sequence: Synthetic DNA
SEQ ID NO:78-Explanation of artificial sequence: Synthetic DNA
SEQ ID NO:79-Explanation of artificial sequence: Synthetic DNA
SEQ ID NO:80-Explanation of artificial sequence: Synthetic DNA
SEQ ID NO:81-Explanation of artificial sequence: Synthetic DNA
SEQ ID NO:82-Explanation of artificial sequence: Synthetic DNA
SEQ ID NO:83-Explanation of artificial sequence: synthetic DNA
SEQ ID NO:84-Explanation of artificial sequence: Synthetic DNA
SEQ ID NO:85-Explanation of artificial sequence: Synthetic DNA
SEQ ID NO:86-Explanation of artificial sequence: Humanized antibody heavy chain variable region
SEQ ID NO:87-Explanation of artificial sequence: Humanized antibody light chain variable region
SEQ ID NO:88-Explanation of artificial sequence: Humanized antibody heavy chain variable region
SEQ ID NO:89-Explanation of artificial sequence: Humanized antibody light chain variable region
SEQ ID NO:90-Explanation of artificial sequence: Humanized antibody heavy chain variable region
SEQ ID NO:91-Explanation of artificial sequence: Humanized antibody heavy chain variable region
SEQ ID NO:92-Explanation of artificial sequence: Humanized antibody light chain variable region
SEQ ID NO:93-Explanation of artificial sequence: Humanized antibody light chain variable region
SEQ ID NO: 94 -Explanation of artificial sequence: Humanized antibody light:chain.variable region
SEQ ID NO: 95-Explanation of artificial sequence: Humanized antibody heavy chain variable region
SEQ ID NO:96-Explanation of artificial sequence: Humanized antibody light chain variable region

## Claims

1. A gene recombinant antibody which (i) specifically reacts with human VEGF receptor Flt-1, (ii) recognizes an epitope present in a region defined by the N-terminal amino acid sequence consisting of the amino acid residues 1 to 338 of human VEGF receptor Flt-1 including its signal sequence and (iii) inhibits binding of human VEGF to human VEGF receptor Flt-1, wherein the amino acid sequence of the H chain V region of the gene recombinant antibody comprises the amino acid sequences of SEQ ID NOS:5, 6 and 7 or the amino acid sequences of SEQ ID NOS:11, 12 and 13 as CDRs, and the amino acid sequence of the L chain V region of the gene recombinant antibody comprises the amino acid sequences of SEQ ID NOS:8, 9 and 10 or the amino acid sequences of SEQ ID, NOS:14, 15 and 16 as CDRs, provided that monoclonal antibody KM1732 as deposited under accession number FERM BP-5698 and monoclonal antibody KM1750 as deposited under accession number FERM BP-5700 are excluded.

2. The gene recombinant antibody according to claim 1, wherein the epitope region is defined by the N-terminal amino acid sequence consisting of the amino acid residues 100 to 204 of human VEGF receptor Flt-1 including its signal sequence.

3. The gene recombinant antibody according to claim 1 or 2, wherein the gene recombinant antibody is selected from a humanized antibody and an antibody fragment.

4. The gene recombinant antibody according to claim 3, wherein the humanized antibody belongs to the human IgG immunoglobulin class.

5. The gene recombinant antibody according to claim 3, wherein the humanized antibody is a human chimeric antibody or a human CDR-grafted antibody.

6. The human chimeric antibody according to claim 5, which further comprises an H chain C region and L chain C region of a human antibody.

7. The human chimeric antibody according to claim 6, wherein the amino acid sequences of the H chain V region and L chain V region of the human chimeric antibody comprise the amino acid sequences of the H chain V region and L chain V region, respectively, of a monoclonal antibody selected from monoclonal antibody KM1732 as deposited under accession number FERM BP-5698 and monoclonal antibody KM1750 as deposited under accession number FERM BP-5700.

8. The human chimeric antibody according to claim 6, wherein the amino acid sequence of the H chain V region of the human chimeric antibody is the amino acid sequence of SEQ ID NO:86 or 88.

9. The human chimeric antibody according to claim 6, wherein the amino acid sequence of the L chain V region of the human chimeric antibody is the amino acid sequence of SEQ ID NO:87 or 89.

10. The human chimeric antibody according to claim 6, wherein the amino acid sequence of the H chain V region of the human chimeric antibody comprises the amino acid sequence of SEQ ID NO:86, and the amino acid sequence of the L chain V region comprises the amino acid sequence of SEQ ID NO:87.

11. The human chimeric antibody according to claim 6, wherein the amino acid sequence of the H chain V region of the human chimeric antibody comprises the amino acid sequence of SEQ ID NO:88, and the amino acid sequence of the L chain V region comprises the amino acid sequence of SEQ ID NO:89.

12. The human chimeric antibody according to claim 6, which is selected from KM2532 as deposited under accession numbers FERM BP-6352 and -6354 and KM2550 as deposited under accession numbers FERM BP-6353 and - 6355.

13. A DNA which encodes the human chimeric antibody of any one of claims 6 to 12.

14. A recombinant vector comprising the DNA of claim 13 and tandem cassette vector pKANTEX93.

15. A transformant cell obtained by introducing the recombinant vector of claim 14 into a host cell.

16. A method for producing an antibody, comprising culturing the transformant cell of claim 15 in a medium to produce and accumulate the human chimeric antibody of claims 6 to 12 in a culture; and recovering the antibody from the resulting culture.

17. The human CDR-grafted antibody according to claim 5, which further comprises C regions and V region framework regions of an H chain and an L chain of a human antibody.

18. The human CDR-grafted antibody according to claim 17, wherein the amino acid sequence of the H chain V region of the human CDR-grafted antibody comprises the amino acid sequences of SEQ ID NOS:5, 6 and 7 as CDRs, and the amino acid sequence of the L chain V region of the human CDR-grafted antibody comprises the amino acid sequences of SEQ ID NOS:8, 9 and 10 as CDRs.

19. The human CDR-grafted antibody according to claim 17, wherein the amino acid sequence of the H chain V region of the human CDR-grafted antibody comprises the amino acid sequences of SEQ ID NOS:11, 12 and 13 as CDRs, and the amino acid sequence of the L chain V region of the human CDR-grafted antibody comprises the amino acid sequences of SEQ ID NOS:14, 15 and 16 as CDRs.

20. The human CDR-grafted antibody according to claim 17, wherein the amino acid sequence of the H chain V region of the human CDR-grafted antibody comprises the amino acid sequence of SEQ ID NO:90, and the amino acid sequence of the L chain V region of the human CDR-grafted antibody comprises the amino acid sequence of SEQ ID NO:92.

21. The human CDR-grafted antibody according to claim 17, wherein the amino acid sequence of the H chain V region of the human CDR-grafted antibody comprises the amino acid sequence of SEQ ID NO:91 or 95, and the amino acid sequence of the L chain V region of the human CDR-grafted antibody comprises the amino acid sequence of SEQ ID NO:93, 94 or 96.

22. The human CDR-grafted antibody according to claim 17, which is selected from KM8550 as deposited under accession numbers FERM BP-6719 and - 6716, KM8551 as deposited under accession numbers FERM BP-6719 and - 6717, KM8552 as deposited under accession numbers FERM BP-6720 and - 6716, KM8553 as deposited under accession numbers FERM BP-6720 and - 6717, KM8554 as deposited under accession numbers FERM BP-6719 and - 6718, and KM 85555 as deposited under accession numbers FERM BP-6720 and -6718.

23. A DNA which encodes the human CDR-grafted antibody of any one of claims 17-22.

24. A recombinant vector comprising the DNA of claim 23 and tandem cassette vector pKANTEX93.

25. A transformant cell obtained by introducing the recombinant vector of claim 24 into a host cell.

26. A method for producing an antibody, comprising culturing the transformant of claim 25 in a medium to produce and accumulate the human CDR-grafted antibody of claims 17 to 22 in a culture, and recovering the antibody from the resulting culture.

27. The gene recombinant antibody according to claim 3, wherein the antibody fragment is any one selected from the group consisting of Fab, Fab', F(ab')₂, a single chain antibody, and a disulfide stabilized Fv.

28. The single chain antibody according to claim 27, wherein the single chain antibody comprises an H chain and L chain V antibody region.

29. The single chain antibody according to claim 28, wherein the amino acid sequences of the H chain V region and L chain V region of the single chain antibody are SEQ ID NOS:86 and 87, respectively.

30. The single chain antibody according to claim 28, wherein the amino acid sequences of the H chain V region and L chain V region of the single chain antibody are SEQ ID NOS:88 and 89, respectively.

31. The single chain antibody according to claim 28, wherein the amino acid sequence of the H chain V region of the single chain antibody comprises the amino acid sequences of SEQ ID NOS:5, 6 and 7 as CDRs, and the amino acid sequence of the L chain V region of the single chain antibody comprises the amino acid sequences of SEQ ID NOS: 8, 9 and 10 as CDRs.

32. The single chain antibody according to claim 28, wherein the amino acid sequence of the H chain V region of the single chain antibody comprises the amino acid sequences of SEQ ID NOS: 11.12 and 13 as CDRs, and the amino acid sequence of the L chain V region of the single chain antibody comprises the amino acid sequences of SEQ ID NOS: 14, 15 and 16 as CDRs.

33. The single chain antibody according to claim 28, wherein the amino acid sequence of the H chain V region of the single chain antibody comprises the amino acid sequence of SEQ ID NO:90, and the amino acid sequence of the L chain V region of the single chain antibody comprises the amino acid sequence of SEQ ID NO:92.

34. The single chain antibody according to claim 28, wherein the amino acid sequence of the H chain V region of the single chain antibody comprises the amino acid sequence of SEQ ID NO:91 or 95, and the amino acid sequence of the L chain V region of the single chain antibody comprises the amino acid sequence of SEQ ID NO:93, 94 or 96.

35. The disulfide stabilized Fv according to claim 27, wherein the disulfide stabilized Fv comprises an H chain and L chain V antibody region.

36. The disulfide stabilized Fv according to claim 35, wherein the amino acid sequences of the H chain V region and L chain V region of the disulfide stabitized Fv are SEQ ID NOS:86 and 87, respectively.

37. The disulfide stabilized Fv according to claim 35, wherein the amino acid sequences of the H chain V region and L chain V region of the disulfide stabilized Fv are SEQ ID NOS:88 and 89, respectively.

38. The disulfide stabilized Fv according to claim 35, wherein the amino acid sequence of the H chain V region of the disulfide stabilized Fv comprises the amino acid sequences of SEQ ID NOS:5, 6 and 7 as CDRs, and the amino acid sequence of the L chain V region of the disulfide stabilized Fv comprises the amino acid sequences of SEQ ID NOS:8, 9 and 10 as CDRs.

39. The disulfide stabilized Fv according to claim 35, wherein the amino acid sequence of the H chain V region of the disulfide stabilized Fv comprises the amino acid sequences of SEQ ID NOS:11, 12 and 13 as CDRs, and the amino acid sequence of the L chain V region of the disulfide stabilized Fv comprises the amino acid sequences of SEQ ID NOS:14, 15 and 16 as CDRs.

40. The disulfide stabilized Fv according to claim 35, wherein the amino acid sequences of the H chain V region of the disulfide stabilized Fv comprises the amino acid sequence of SEQ ID NO:90, and the amino acid sequence of the L chain V region of the disulfide stabilized Fv comprises the amino acid sequence of SEQ ID NO:92.

41. The disulfide stabilized Fv according to claim 35, wherein the amino acid sequence of the H chain V region of the disulfide stabilized Fv comprises the amino acid sequences of SEQ ID NO:91 or 95, and the amino acid sequence of the L chain V region of the disulfide stabilized Fv comprises the amino acid sequence of SEQ ID NO:93, 94 or 96.

42. An antibody which is a fusion antibody, wherein the antibody of any one of claims 1 to 41 is chemically or gene-engineeringly bound with a radioisotope, protein or low molecular weight agent selected from the group consisting of an antitumor agent, an antimetabolite, an antibiotic, a plant alkaloid, a hormone agent, an anti-inflammatory agent, an immunomodulator and an antihistaminic agent.

43. An in vitro method for immunologically detecting human VEGF receptor Flt-1, using the antibody of any one of claims 1 to 12, 17 to 22 and 27 to 42.

44. An in vitro method for immunologically determining human VEGF receptor Flt-1, using the antibody of any one of claims 1 to 12, 17 to 22 and 27 to 42.

45. An in vitro method for immunologically detecting soluble human VEGF receptor Flt-1, using the antibody of any one of claims 1 to 12, 17 to 22 and 27 to 42.

46. An in vitro method for immunologically determining soluble human VEGF receptor Flt-1, using the antibody of any one of claims 1 to 12, 17 to 22 and 27 to 42.

47. An in vitro method for immunologically detecting cells in which human VEGF receptor Flt-1 is expressed on the surface thereof, using the antibody of any one of claims 1 to 12, 17 to 22 and 27 to 42.

48. An in vitro method for immunologically determining cells in which human VEGF receptor Flt-1 is expressed on the surface thereof, using the antibody of any one of claims 1 to 12, 17 to 22 and 27 to 42.

49. An in vitro method for inhibiting binding of human VEGF to human VEGF receptor Flt-1, using the antibody of any one of claims 1 to 12, 17 to 22 and 27 to 42.

50. An in vitro method for neutralizing the function of human VEGF receptor Flt-1, using the antibody of any one of claims 1 to 12, 17 to 22 and 27 to 42.

51. An in vitro method for inhibiting migration of vascular endothelial cells, using the antibody of any one of claims 1 to 12, 17 to 22 and 27 to 42.

52. An in vitro method for detecting a disease which is selected from the group consisting of proliferation or metastasis of solid tumors, rheumatoid arthritis, diabetic retinopathy, retinopathy of prematurity and psoriasis, using the antibody of any one of claims 1 to 12, 17 to 22 and 27 to 42.

53. The antibody of any one of claims 1 to 12, 17 to 22 and 27 to 42 for use in therapy or diagnosis.

54. Pharmaceutical composition comprising at least one antibody of any one of claims 1 to 12, 17 to 22 and 27 to 42.

55. The use of the antibody of any one of claims 1 to 12, 17 to 22 and 27 to 42 in the manufacture of an agent for detecting regions of angiogenesis or for inhibiting angiogenesis.

56. The antibody of any one of claims 1 to 12, 17 to 22 and 27 to 42 for use in detecting regions of angiogenesis or for inhibiting angiogenesis.

57. The use of the antibody of any one of claims 1 to 12, 17 to 22 and 27 to 42 for the manufacture of an agent for diagnosing a disease selected from the group consisting of proliferation or metastasis of solid tumors, rheumatoid arthritis, diabetic retinopathy, retinopathy of prematurity and psoriasis.

58. The antibody of any one of claims 1 to 12, 17 to 22 and 27 to 42 for use in diagnosing a disease selected from the group consisting of proliferation or metastasis of solid tumors, rheumatoid arthritis, diabetic retinopathy, retinopathy of prematurity and psoriasis.

59. The use of the antibody of any one of claims 1 to 12, 17 to 22 and 27 to 42 for the manufacture of an agent for treating a disease selected from the group consisting of proliferation or metastasis of solid tumors, rheumatoid arthritis, diabetic retinopathy, retinopathy of prematurity and psoriasis.

60. The antibody of any one of claims 1 to 12, 17 to 22 and 27 to 42 for use in treating a disease selected from the group consisting of proliferation or metastasis of solid tumors, rheumatoid arthritis, diabetic retinopathy, retinopathy of prematurity and psoriasis.

## Patentansprüche

1. Genrekombinanter Antikörper, der (i) mit humanem VEGF-Rezeptor Flt-1 spezifisch reagiert, (ii) ein Epitop erkennt, das in einer Region vorkommt, die definiert ist durch die N-terminale Aminosäuresequenz bestehend aus den Aminosäureresten 1 bis 338 des humanen VEGF-Rezeptors Flt-1 einschließlich dessen Signalsequenz, und (iii) die Bindung von humanem VEGF an humanen VEGF-Rezeptor Flt-1 inhibiert, wobei die Aminosäuresequenz der H-Ketten-V-Region des genrekombinanten Antikörpers die Aminosäuresequenzen der SEQ ID NO:5, 6, und 7 oder die Aminosäureseqenzen der SEQ ID NO:11, 12 und 13 als CDRs umfasst, und wobei die Aminosäuresequenz der L-Ketten-V-Region des genrekombinanten Antikörpers die Aminosäuresequenzen der SEQ ID NO:8, 9 und 10 oder die Aminosäuresequenzen der SEQ ID NO:14, 15 und 16 als CDRs umfasst, mit der Maßgabe, dass der unter der Zugangsnummer FERM BP-5698 hinterlegte monoklonale Antikörper KM1732 und der unter der Zugangsnummer FERM-BP-5700 hinterlegte monoklonale Antikörper KM1750 ausgenommen sind.

2. Genrekombinanter Antikörper nach Anspruch 1, wobei die Epitopregion definiert ist durch die N-terminale Aminosäuresequenz, bestehend aus den Aminosäureresten 100 bis 204 von humanem VEGF-Rezeptor Flt-1 einschließlich dessen Signalsequenz.

3. Genrekombinanter Antikörper nach Anspruch 1 oder 2, wobei der genrekombinante Antikörper ausgewählt ist unter einem humanisierten Antikörper und einem Antikörperfragment.

4. Genrekombinanter Antikörper nach Anspruch 3, wobei der humanisierte Antikörper zur humanen IgG-Immunglobulin-Klasse gehört.

5. Genrekombinanter Antikörper nach Anspruch 3, wobei der humanisierte Antikörper ein humaner chimärer Antikörper oder ein humaner Graft-CDR-Antikörper ist.

6. Humaner chimärer Antikörper nach Anspruch 5, der weiterhin eine H-Ketten-C-Region und eine L-Ketten-C-Region eines humanen Antikörpers umfasst.

7. Humaner chimärer Antikörper nach Anspruch 6, wobei die Aminosäuresequenzen der H-Ketten-V-Region und der L-Ketten-V-Region des humanen chimären Antiköpers die Aminosäuresequenzen der H-Ketten-V-Region beziehungsweise der L-Ketten-V-Region eines monoklonalen Antikörpers umfassen, der ausgewählt ist unter dem unter der Zugangsnummer FERM BP-5698 hinterlegten monoklonalen Antikörper KM1732 und dem unter der Zugangsnummer FERM BP-5700 hinterlegten monoklonalen Antikörper KM1750.

8. Humaner chimärer Antikörper nach Anspruch 6, wobei die Aminosäuresequenz der H-Ketten-V-Region des humanen chimären Antikörpers die Aminosäuresequenz der SEQ ID NO:86 oder 88 ist.

9. Humaner chimärer Antikörper nach Anspruch 6, wobei die Aminosäuresequenz der L-Ketten-V-Region des humanen chimären Antikörpers die Aminosäuresequenz der SEQ ID NO:87 oder 89 ist.

10. Humaner chimärer Antikörper nach Anspruch 6, wobei die Aminosäuresequenz der H-Ketten-V-Region des humanen chimären Antikörpers die Aminosäuresequenz der SEQ ID NO:86 umfasst, und die Aminosäuresequenz der L-Ketten-V-Region die Aminosäuresequenz der SEQ ID NO:87 umfasst.

11. Humaner chimärer Antikörper nach Anspruch 6, wobei die Aminosäuresequenz der H-Ketten-V-Region des humanen chimären Antikörpers die Aminosäuresequenz der SEQ ID NO:88 umfasst, und die Aminosäuresequenz der L-Ketten-V-Region die Aminosäuresequenz der SEQ ID NO:89 umfasst.

12. Humaner chimärer Antikörper nach Anspruch 6, ausgewählt unter dem unter den Zugangsnummern FERM BP-6352 und -6354 hinterlegten KM2532 und dem unter den Zugangsnummern FERM BP-6353 und - 6355 hinterlegten KM2550.

13. DNA, die den humanen chimären Antikörper nach einem der Ansprüche 6 bis 12 codiert.

14. Rekombinanter Vektor, umfassend die DNA nach Anspruch 13 und den Tandem-Kassetten-Vektor pKANTEX93.

15. Transformantenzelle, erhalten durch Einführen des rekombinanten Vektors nach Anspruch 14 in eine Wirtszelle.

16. Verfahren zur Herstellung eines Antikörpers, wobei man die Transformantenzelle nach Anspruch 15 in einem Medium kultiviert, um den humanen chimären Antikörper der Ansprüche 6 bis 12 in einer Kultur herzustellen und anzureichern; und den Antikörper aus der erhaltenen Kultur gewinnt.

17. Humaner Graft-CDR-Antikörper nach Anspruch 5, der weiterhin C-Regionen und V-Region-Gerüstregionen einer H-Kette und einer L-Kette eines humanen Antikörpers umfasst.

18. Humaner Graft-CDR-Antikörper nach Anspruch 17, wobei die Aminosäuresequenz der H-Ketten-V-Region des humanen Graft-CDR-Antikörpers die Aminosäuresequenzen der SEQ ID NO:5, 6 und 7 als CDRs umfasst, und die Aminosäuresequenz der L-Ketten-V-Region des humanen Graft-CDR-Antikörpers die Aminosäuresequenzen der SEQ ID NO:8, 9 und 10 als CDRs umfasst.

19. Humaner Graft-CDR-Antikörper nach Anspruch 17, wobei die Aminosäuresequenz der H-Ketten-V-Region des humanen Graft-CDR-Antikörpers die Aminosäuresequenzen der SEQ ID NO:11, 12 und 13 als CDRs umfasst, und die Aminosäuresequenz der L-Ketten-V-Region des humanen Graft-CDR-Antikörpers die Aminosäuresequenzen der SEQ ID NO:14,15 und 16 als CDRs umfasst.

20. Humaner Graft-CDR-Antikörper nach Anspruch 17, wobei die Aminosäuresequenz der H-Ketten-V-Region des humanen Graft-CDR-Antikörpers die Aminosäuresequenz der SEQ ID NO:90 umfasst, und die Aminosäuresequenz der L-Ketten-V-Region des humanen Graft-CDR-Antikörpers die Aminosäuresequenz der SEQ ID NO:92 umfasst.

21. Humaner Graft-CDR-Antikörper nach Anspruch 17, wobei die Aminosäuresequenz der H-Ketten-V-Region des humanen Graft-CDR-Antikörpers die Aminosäuresequenzen der SEQ ID NO:91 oder 95 umfasst, und die Aminosäuresequenz der L-Ketten-V-Region des humanen Graft-CDR-Antikörpers die Aminosäuresequenzen der SEQ ID NO:93, 94 oder 96 umfasst.

22. Humaner Graft-CDR-Antikörper nach Anspruch 17, ausgewählt unter dem unter den Zugangsnummern FERM BP-6719 und -6716 hinterlegten KM8550, dem unter den Zugangsnummern FERM BP-6719 und -6717 hinterlegten KM8551, dem unter den Zugangsnummern FERM BP-6720 und -6716 hinterlegten KM8552, dem unter den Zugangsnummern FERM BP-6720 und -6717 hinterlegten KM8553, dem unter den Zugangsnummern FERM BP-6719 und -6718 hinterlegten KM8554, und dem unter den Zugangsnummern FERM BP-6720 und -6718 hinterlegten KM 85555.

23. DNA, die den humanen Graft-CDR-Antikörper nach einem der Ansprüche 17-22 codiert.

24. Rekombinanter Vektor, umfassend die DNA nach Anspruch 23 und den Tandem-Kassetten-Vektor pKANTEX93.

25. Transformantenzelle, erhalten durch Einführen des rekombinanten Vektors nach Anspruch 24 in eine Wirtszelle.

26. Verfahren zur Herstellung eines Antikörpers, wobei man die Transformante nach Anspruch 25 in einem Medium kultiviert, um den humanen Graft-CDR-Antikörper der Ansprüche 17 bis 22 in einer Kultur herzustellen und anzureichern; und den Antikörper aus der erhaltenen Kultur gewinnt.

27. Genrekombinanter Antikörper nach Anspruch 3, wobei das Antikörperfragment ein beliebiges unter Fab, Fab', F(ab')₂, einem Einzelkettenantikörper und einem Disulfid-stabilisierten Fv ausgewähltes Antikörperfragment ist.

28. Einzelkettenantikörper nach Anspruch 27, wobei der Einzelkettenantikörper eine H-Ketten- und eine L-Ketten-V-Antikörperregion umfasst.

29. Einzelkettenantikörper nach Anspruch 28, wobei die Aminosäuresequenzen der H-Ketten-V-Region und der L-Ketten-V-Region des Einzelkettenantikörpers die SEQ ID NO:86 beziehungsweise 87 sind.

30. Einzelkettenantikörper nach Anspruch 28, wobei die Aminosäuresequenzen der H-Ketten-V-Region und der L-Ketten-V-Region des Einzelkettenantikörpers die SEQ ID NO:88 beziehungsweise 89 sind.

31. Einzelkettenantikörper nach Anspruch 28, wobei die Aminosäuresequenz der H-Ketten-V-Region des Einzelkettenantikörpers die Aminosäuresequenzen der SEQ ID NO:5, 6 und 7 als CDRs umfasst, und die Aminosäuresequenz der L-Ketten-V-Region des Einzelkettenantikörpers die Aminosäuresequenzen der SEQ ID NO:8, 9 und 10 als CDRs umfasst.

32. Einzelkettenantikörper nach Anspruch 28, wobei die Aminosäuresequenz der H-Ketten-V-Region des Einzelkettenantikörpers die Aminosäuresequenzen der SEQ ID NO:11, 12 und 13 als CDRs umfasst, und die Aminosäuresequenz der L-Ketten-V-Region des Einzelkettenantikörpers die Aminosäuresequenzen der SEQ ID NO:14, 15 und 16 als CDRs umfasst.

33. Einzelkettenantikörper nach Anspruch 28, wobei die Aminosäuresequenz der H-Ketten-V-Region des Einzelkettenantikörpers die Aminosäuresequenz der SEQ ID NO:90 umfasst, und die Aminosäuresequenz der L-Ketten-V-Region des Einzelkettenantikörpers die Aminosäuresequenz der SEQ ID NO:92 umfasst.

34. Einzelkettenantikörper nach Anspruch 28, wobei die Aminosäuresequenz der H-Ketten-V-Region des Einzelkettenantikörpers die Aminosäuresequenz der SEQ ID NO:91 oder 95 umfasst, und die Aminosäuresequenz der L-Ketten-V-Region des Einzelkettenantikörpers die Aminosäuresequenzen der SEQ ID NO:93, 94 oder 96 umfasst.

35. Disulfid-stabilisiertes Fv nach Anspruch 27, wobei das Disulfidstabilisierte Fv eine H-Ketten- und eine L-Ketten-V-Antikörper-Region umfasst.

36. Disulfid-stabilisiertes Fv nach Anspruch 35, wobei die Aminosäuresequenzen der H-Ketten-V-Region und der L-Ketten-V-Region des Disulfid-stabilisierten Fv die SEQ ID NO:86 beziehungsweise 87 sind.

37. Disulfid-stabilisiertes Fv nach Anspruch 35, wobei die Aminosäuresequenzen der H-Ketten-V-Region und der L-Ketten-V-Region des Disulfid-stabilisierten Fv die SEQ ID NO:88 beziehungsweise 89 sind.

38. Disulfid-stabilisiertes Fv nach Anspruch 35, wobei die Aminosäuresequenz der H-Ketten-V-Region des Disulfid-stabilisierten Fv die Aminosäuresequenzen der SEQ ID NO:5,6 und 7 als CDRs umfasst, und die Aminosäuresequenz der L-Ketten-V-Region des Disulfid-stabilisierten Fv die Aminosäuresequenzen der SEQ ID NO:8, 9 und 10 als CDRs umfasst.

39. Disulfid-stabilisiertes Fv nach Anspruch 35, wobei die Aminosäuresequenz der H-Ketten-V-Region des Disulfid-stabilisierten Fv die Aminosäuresequenzen der SEQ ID NO:11, 12 und 13 als CDRs umfasst, und die Aminosäuresequenz der L-Ketten-V-Region des Disulfid-stabilisierten Fv die Aminosäuresequenzen der SEQ ID NO:14, 15 und 16 als CDRs umfasst.

40. Disulfid-stabilisiertes Fv nach Anspruch 35, wobei die Aminosäuresequenz der H-Ketten-V-Region des Disulfid-stabilisierten Fv die Aminosäuresequenz der SEQ ID NO:90 umfasst, und die Aminosäuresequenz der L-Ketten-V-Region des Disulfid-stabilisierten Fv die Aminosäuresequenz der SEQ ID NO:92 umfasst.

41. Disulfid-stabilisiertes Fv nach Anspruch 35, wobei die Aminosäuresequenz der H-Ketten-V-Region des Disulfid-stabilisierten Fv die Aminosäuresequenzen der SEQ ID NO:91 oder 95 umfasst, und die Aminosäuresequenz der L-Ketten-V-Region des Disulfid-stabilisierten Fv die Aminosäuresequenzen der SEQ ID NO:93, 94 oder 96 umfasst.

42. Antikörper, der ein Fusionsantikörper ist, wobei der Antikörper nach einem der Ansprüche 1 bis 41 chemisch oder gentechnisch mit einem Radioisotop, einem Protein oder einem Agens mit niedrigem Molekulargewicht verbunden ist, das ausgewählt ist unter einem Antitumor-Agens, einem Antimetaboliten, einem Antibiotikum, einem Pflanzenalkaloid, einem Hormon-Agens, eine antiinflammatorischen Agens, einem Immunmodulator und einem Antihistamin-Agens.

43. In vitro-Verfahren zum immunologischen Nachweis von humanem VEGF-Rezeptor Flt-1, wobei man den Antikörper nach einem der Ansprüche 1 bis 12, 17 bis 22 und 27 bis 42 verwendet.

44. In vitro-Verfahren zur immunologischen Bestimmung von humanem VEGF-Rezeptor Flt-1, wobei man den Antikörper nach einem der Ansprüche 1 bis 12, 17 bis 22 und 27 bis 42 verwendet.

45. In vitro-Verfahren zum immunologischen Nachweis von löslichem humanem VEGF-Rezeptor Flt-1, wobei man den Antikörper nach einem der Ansprüche 1 bis 12, 17 bis 22 und 27 bis 42 verwendet.

46. In vitro-Verfahren zur immunologischen Bestimmung von löslichem humanem VEGF-Rezeptor Flt-1, wobei man den Antikörper nach einem der Ansprüche 1 bis 12, 17 bis 22 und 27 bis 42 verwendet.

47. In vitro-Verfahren zum immunologischen Nachweis von Zellen, auf deren Oberfläche humaner VEGF-Rezeptor Flt-1 exprimiert wird, wobei man den Antikörper nach einem der Ansprüche 1 bis 12, 17 bis 22 und 27 bis 42 verwendet.

48. In vitro-Verfahren zur immunologischen Bestimmung von Zellen, auf deren Oberfläche humaner VEGF-Rezeptor Flt-1 exprimiert wird, wobei man den Antikörper nach einem der Ansprüche 1 bis 12, 17 bis 22 und 27 bis 42 verwendet.

49. In vitro-Verfahren zur Inhibierung der Bindung von humanem VEGF an humanen VEGF-Rezeptor Flt-1, wobei man den Antikörper nach einem der Ansprüche 1 bis 12, 17 bis 22 und 27 bis 42 verwendet.

50. In vitro-Verfahren zur Neutralisierung der Funktion von humanen VEGF-Rezeptor Flt-1, wobei man den Antikörper nach einem der Ansprüche 1 bis 12, 17 bis 22 und 27 bis 42 verwendet.

51. In vitro-Verfahren zur Inhibierung der Migration von Gefäßendothelzellen, wobei man den Antikörper nach einem der Ansprüche 1 bis 12, 17 bis 22 und 27 bis 42 verwendet.

52. In vitro-Verfahren zum Nachweis einer Erkrankung, die ausgewählt ist unter der Proliferation oder Metastase solider Tumoren, rheumatoider Arthritis, diabetischer Retinopathie, Retinopathie Frühgeborener und Psoriasis, wobei man den Antikörper nach einem der Ansprüche 1 bis 12, 17 bis 22 und 27 bis 42 verwendet.

53. Antikörper nach einem der Ansprüche 1 bis 12, 17 bis 22 und 27 bis 42 zur Verwendung bei der Therapie oder Diagnose.

54. Pharmazeutische Zusammensetzung, umfassend wenigstens einen Antikörper nach einem der Ansprüche 1 bis 12, 17 bis 22 und 27 bis 42.

55. Verwendung des Antikörpers nach einem der Ansprüche 1 bis 12, 17 bis 22 und 27 bis 42 bei der Herstellung eines Agens zum Nachweis von Regionen der Angiogenese oder zur Inhibierung von Angiogenese.

56. Antikörper nach einem der Ansprüche 1 bis 12, 17 bis 22 und 27 bis 42 zur Verwendung beim Nachweis von Regionen der Angiogenese oder zur Inhibierung von Angiogenese.

57. Verwendung des Antikörpers nach einem der Ansprüche 1 bis 12, 17 bis 22 und 27 bis 42 bei der Herstellung eines Agens zur Diagnose einer Erkrankung, die ausgewählt ist unter der Proliferation oder Metastase solider Tumoren, rheumatoider Arthritis, diabetischer Retinopathie, Retinopathie Frühgeborener und Psoriasis.

58. Antikörper nach einem der Ansprüche 1 bis 12, 17 bis 22 und 27 bis 42 zur Verwendung bei der Diagnose einer Erkrankung, die ausgewählt ist unter der Proliferation oder Metastase solider Tumoren, rheumatoider Arthritis, diabetischer Retinopathie, Retinopathie Frühgeborener und Psoriasis.

59. Verwendung des Antikörpers nach einem der Ansprüche 1 bis 12, 17 bis 22 und 27 bis 42 zur Herstellung eines Agens zur Behandlung einer Erkrankung, die ausgewählt ist unter der Proliferation oder Metastase solider Tumoren, rheumatoider Arthritis, diabetischer Retinopathie, Retinopathie Frühgeborener und Psoriasis.

60. Antikörper nach einem der Ansprüche 1 bis 12, 17 bis 22 und 27 bis 42 zur Verwendung bei der Behandlung einer Erkrankung, die ausgewählt ist unter der Proliferation oder Metastase solider Tumoren, rheumatoider Arthritis, diabetischer Retinopathie, Retinopathie Frühgeborener und Psoriasis.

## Revendications

1. Anticorps recombiné génique qui (i) réagit spécifiquement avec le récepteur Flt-1 du VEGF humain, (ii) reconnaît un épitope présent dans une région définie par la séquence d'acides aminés N-terminale constituée des résidus acides aminés 1 à 338 du récepteur Flt-1 du VEGF humain, y compris sa séquence signal et (iii) inhibe la liaison du VEGF humain au récepteur Flt-1 du VEGF humain, où la séquence d'acides aminés de la région V de la chaîne H de l'anticorps recombiné génique comprend les séquences d'acides aminés de SEQ ID NO:5, 6 et 7 ou les séquences d'acides aminés de SEQ ID NO: 11, 12 et 13 en tant que CDR et la séquence d'acides aminés de la région V de la chaîne L de l'anticorps recombiné génique comprend les séquences d'acides aminés de SEQ ID NO:8, 9 et 10 ou les séquences d'acides aminés de SEQ ID NO:14, 15 et 16 en tant que CDR, pour autant que l'anticorps monoclonal KM1732 tel que déposé sous le n° d'entrée FERM BP-5698 et l'anticorps monoclonal KM1750 tel que déposé sous le n° d'entrée FERM BP-5700 sont exclus.

2. Anticorps recombiné génique selon la revendication 1, où la région épitopique est définie par la séquence d'acides aminés N-terminale constituée des résidus acides aminés 100 à 200 du récepteur Flt-1 du VEGF humain y compris sa séquence signal.

3. Anticorps recombiné génique selon la revendication 1 ou 2, où l'anticorps recombiné génique est sélectionné parmi un anticorps humanisé et un fragment d'anticorps.

4. Anticorps recombiné génique selon la revendication 3, où l'anticorps humanisé appartient à la classe des immunoglobulines IgG humaines.

5. Anticorps recombiné génique selon la revendication 3, où l'anticorps humanisé est un anticorps chimère humain ou un anticorps humain à CDR greffée.

6. Anticorps chimère humain selon la revendication 5, lequel comprend en outre une région C de chaîne H et une région C de chaîne L d'un anticorps humain.

7. Anticorps chimère humain selon la revendication 6, où les séquences d'acides aminés de la région V de la chaîne H et de la région V de la chaîne L de l'anticorps chimère humain comprennent respectivement les séquences d'acides aminés de la région V de la chaîne H et de la région V de la chaîne L d'un anticorps monoclonal sélectionné parmi un anticorps monoclonal KM1732 tel que déposé sous le n° d'entrée FERM BP-5698 et un anticorps monoclonal KM1750 tel que déposé sous le n° d'entrée FERM BP-5700.

8. Anticorps chimère humain selon la revendication 6, où la séquence d'acides aminés de la région V de la chaîne H de l'anticorps chimère humain est la séquence d'acides aminés de SEQ ID NO:86 ou 88.

9. Anticorps chimère humain selon la revendication 6, où la séquence d'acides aminés de la région V de la chaîne L de l'anticorps chimère humain est la séquence d'acides aminés de SEQ ID NO:87 ou 89.

10. Anticorps chimère humain selon la revendication 6, où la séquence d'acides aminés de la région V de la chaîne H de l'anticorps chimère humain comprend la séquence d'acides aminés de SEQ ID NO:86 et la séquence d'acides aminés de la région V de la chaîne L comprend la séquence d'acides aminés de SEQ ID NO:87.

11. Anticorps chimère humain selon la revendication 6, où la séquence d'acides aminés de la région V de la chaîne H de l'anticorps chimère humain comprend la séquence d'acides aminés de SEQ ID NO:88 et la séquence d'acides aminés de la région V de la chaîne L comprend la séquence d'acides aminés de SEQ ID NO:89.

12. Anticorps chimère humain selon la revendication 6, lequel est sélectionné parmi KM2532 tel que déposé sous les n° d'entrée FERM BP-6352 et 6354 et KM2550 tel que déposé sous les n° d'entrée FERM BP-6353 et 6355.

13. ADN qui code l'anticorps chimère humain selon l'une quelconque des revendication 6 à 12.

14. Vecteur recombiné comprenant l'ADN selon la revendication 13 et un vecteur à cassette tandem pKANTEX93.

15. Cellule transformée obtenue par introduction du vecteur recombiné selon la revendication 14 dans une cellule hôte.

16. Procédé de production d'un anticorps, comprenant la culture de la cellule transformée selon la revendication 15 dans un milieu pour produire et accumuler l'anticorps chimère humain selon les revendications 6 à 12 dans une culture ; et la récupération de l'anticorps dans la culture obtenue.

17. Anticorps humain à CDR greffée selon la revendication 5, lequel comprend en outre les régions C et les régions cadres de la région V d'une chaîne H et d'une chaîne L d'un anticorps humain.

18. Anticorps humain à CDR greffée selon la revendication 17, où la séquence d'acides aminés de la région V de la chaîne H de l'anticorps humain à CDR greffée comprend les séquences d'acides aminés de SEQ ID NO:5, 6 et 7 en tant que CDR et la séquence d'acides aminés de la région V de la chaîne L de l'anticorps humain à CDR greffée comprend les séquences d'acides aminés de SEQ ID NO:8, 9 et 10 en tant que CDR.

19. Anticorps humain à CDR greffée selon la revendication 17, où la séquence d'acides aminés de la région V de la chaîne H de l'anticorps humain à CDR greffée comprend les séquences d'acides aminés de SEQ ID NO:11, 12 et 13 en tant que CDR et la séquence d'acides aminés de la région V de la chaîne L de l'anticorps humain à CDR greffée comprend les séquences d'acides aminés de SEQ ID NO:14, 15 et 16 en tant que CDR.

20. Anticorps humain à CDR greffée selon la revendication 17, où la séquence d'acides aminés de la région V de la chaîne H de l'anticorps humain à CDR greffée comprend la séquence d'acides aminés de SEQ ID NO:90 et la séquence d'acides aminés de la région V de la chaîne L de l'anticorps humain à CDR greffée comprend la séquence d'acides aminés de SEQ ID NO:92.

21. Anticorps humain à CDR greffée selon la revendication 17, où la séquence d'acides aminés de la région V de la chaîne H de l'anticorps humain à CDR greffée comprend la séquence d'acides aminés de SEQ ID NO:91 ou 95 et la séquence d'acides aminés de la région V de la chaîne L de l'anticorps humain à CDR greffée comprend la séquence d'acides aminés de SEQ ID NO:93, 94 ou 96.

22. Anticorps humain à CDR greffée selon la revendication 17, lequel est sélectionné parmi KM8550 tel que déposé sous les n° d'entrée FERM BP-6719 et 6716, KM8551 tel que déposé sous les n° d'entrée FERM BP-6719 et 6717, KM8552 tel que déposé sous les n° d'entrée FERM BP-6720 et 6716, KM8553 tel que déposé sous les n° d'entrée FERM BP-6720 et 6717, KM8554 tel que déposé sous les n° d'entrée FERM BP-6719 et 6718 et KM85555 tel que déposé sous les n° d'entrée FERM BP-6720 et 6718.

23. ADN qui code l'anticorps humain à CDR greffée selon l'une quelconque des revendication 17 à 22.

24. Vecteur recombiné comprenant l'ADN selon la revendication 23 et le vecteur à cassette tandem pKANTEX93.

25. Cellule transformée obtenue par l'introduction du vecteur recombiné selon la revendication 24 dans une cellule hôte.

26. Procédé de production d'un anticorps, comprenant la culture du transformant selon la revendication 25 dans un milieu pour produire et accumuler l'anticorps humain à CDR greffée selon les revendications 17 à 22 dans une culture; et la récupération de l'anticorps dans la culture obtenue.

27. Anticorps recombiné génique selon la revendication 3, où le fragment d'anticorps est l'un quelconque sélectionné dans le groupe constitué de Fab, Fab', F(ab')₂, un anticorps monocaténaire et un Fv stabilisé par le disulfure.

28. Anticorps monocaténaire selon la revendication 27, où l'anticorps monocaténaire comprend une région d'anticorps V de la chaîne H et de la chaîne L.

29. Anticorps monocaténaire selon la revendication 28, où les séquences d'acides aminés de la région V de la chaîne H et de la région V de la chaîne L de l'anticorps monocaténaire sont respectivement SEQ ID NO:86 et 87.

30. Anticorps monocaténaire selon la revendication 28, où les séquences d'acides aminés de la région V de la chaîne H et de la région V de la chaîne L de l'anticorps monocaténaire sont respectivement SEQ ID NO:88 et 89.

31. Anticorps monocaténaire selon la revendication 28, où la séquence d'acides aminés de la région V de la chaîne H de l'anticorps monocaténaire comprend les séquences d'acides aminés de SEQ ID NO. 5, 6 et 7 en tant que CDR et la séquence d'acides aminés de la région V de la chaîne L de l'anticorps monocaténaire comprend les séquences d'acides aminés de SEQ ID NO.8, 9 et 10 en tant que CDR.

32. Anticorps monocaténaire selon la revendication 28, où la séquence d'acides aminés de la région V de la chaîne H de l'anticorps monocaténaire comprend les séquences d'acides aminés de SEQ ID NO.11, 12 et 13 en tant que CDR et la séquence d'acides aminés de la région V de la chaîne L de l'anticorps monocaténaire comprend les séquences d'acides aminés de SEQ ID NO. 14, 15 et 16 en tant que CDR.

33. Anticorps monocaténaire selon la revendication 28, où la séquence d'acides aminés de la région V de la chaîne H de l'anticorps monocaténaire comprend la séquence d'acides aminés de SEQ ID NO.90 et la séquence d'acides aminés de la région V de la chaîne L de l'anticorps monocaténaire comprend la séquence d'acides aminés de SEQ ID NO.92.

34. Anticorps monocaténaire selon la revendication 28, où la séquence d'acides aminés de la région V de la chaîne H de l'anticorps monocaténaire comprend la séquence d'acides aminés de SEQ ID NO.91 ou 95 et la séquence d'acides aminés de la région V de la chaîne L de l'anticorps monocaténaire comprend la séquence d'acides aminés de SEQ ID NO.93, 94 ou 96.

35. Fv stabilisé par le disulfure selon la revendication 27, où le Fv stabilisé par le disulfure comprend une région d'anticorps V de chaîne H et de chaîne L.

36. Fv stabilisé par le disulfure selon la revendication 35, où les séquences d'acides aminés de la région V de la chaîne H et de la région V de la chaîne L du Fv stabilisé par le disulfure sont respectivement SEQ ID NO. 86 et 87.

37. Fv stabilisé par le disulfure selon la revendication 35, où les séquences d'acides aminés de la région V de la chaîne H et de la région V de la chaîne L du Fv stabilisé par le disulfure sont respectivement SEQ ID NO. 88 et 89.

38. Fv stabilisé par le disulfure selon la revendication 35, où la séquence d'acides aminés de la région V de la chaîne H du Fv stabilisé par le disulfure comprend les séquences d'acides aminés de SEQ ID NO. 5, 6 et 7 en tant que CDR et la séquence d'acides aminés de la région V de la chaîne L du Fv stabilisé par le disulfure comprend les séquences d'acides aminés de SEQ ID NO. 8, 9 et 10 en tant que CDR.

39. Fv stabilité par le disulfure selon la revendication 35, où la séquence d'acides aminés de la région V de la chaîne H du Fv stabilisé par le disulfure comprend les séquences d'acides aminés de SEQ ID NO.11, 12 et 13 en tant que CDR et la séquence d'acides aminés de la région V de la chaîne L du Fv stabilisé par le disulfure comprend les séquences acides aminés de SEQ ID NO.14, 15 et 16 en tant que CDR.

40. Fv stabilisé par le disulfure selon la revendication 35, où les séquences d'acides aminés de la région V de la chaîne H du Fv stabilisé par le disulfure comprend la séquence d'acides aminés de SEQ ID NO.90 et la séquence d'acides aminés de la région V de la chaîne L du Fv stabilisé par le disulfure comprend la séquence d'acides aminés de SEQ ID NO.92.

41. Fv stabilisé par le disulfure selon la revendication 35, où la séquence d'acides aminés de la région V de la chaîne H du Fv stabilisé par le disulfure comprend les séquences d'acides aminés de SEQ ID NO.91 ou 95 et la séquence d'acides aminés de la région V de la chaîne L du Fv stabilisé par le disulfure comprend la séquence d'acides aminés de SEQ ID NO.93, 94 ou 96.

42. Anticorps, lequel est un anticorps de fusion où l'anticorps selon l'une quelconque des revendications 1 à 41 est lié chimiquement ou par génie génétique à un radioisotope, une protéine ou un agent à masse moléculaire faible sélectionné dans le groupe constitué d'un agent antitumoral, un antimétabolite, un antibiotique, un alcaloïde végétal, un agent hormonal, un anti-inflammatoire, un immunomodulateur et un antihistaminique.

43. Procédé *in vitro* de détection immunologique du récepteur Flt-1 du VEGF humain utilisant l'anticorps selon l'une quelconque des revendications 1 à 12, 17 à 22 et 27 à 42.

44. Procédé *in vitro* de détermination immunologique du récepteur Flt-1 du VEGF humain, utilisant l'anticorps selon l'une quelconque des revendications 1 à 12, 17 à 22 et 27 à 42.

45. Procédé *in vitro* de détection immunologique du récepteur Flt-1 du VEGF humain soluble, utilisant l'anticorps selon l'une quelconque des revendications 1 à 12, 17 à 22 et 27 à 42.

46. Procédé *in vitro* de détermination immunologique du récepteur Flt-1 du VEGF humain soluble, utilisant l'anticorps selon l'une quelconque des revendications 1 à 12, 17 à 22 et 27 à 42.

47. Procédé *in vitro* de détection immunologique de cellules à la surface desquelles est exprimé le récepteur Flt-1 du VEGF humain, utilisant l'anticorps selon l'une quelconque des revendications 1 à 12, 17 à 22 et 27 à 42.

48. Procédé *in vitro* de détermination immunologique de cellules à la surface desquelles est exprimé le récepteur Flt-1 du VEGF humain, utilisant l'anticorps selon l'une quelconque des revendications 1 à 12, 17 à 22 et 27 à 42.

49. Procédé *in vitro* d'inhibition de la liaison du VEGF humain au récepteur Flt-1 du VEGF humain, utilisant l'anticorps selon l'une quelconque des revendications 1 à 12, 17 à 22 et 27 à 42.

50. Procédé *in vitro* de neutralisation de la fonction du récepteur Flt-1 du VEGF humain, utilisant l'anticorps selon l'une quelconque des revendications 1 à 12, 17 à 22 et 27 à 42.

51. Procédé *in vitro* d'inhibition de la migration des cellules endothéliales vasculaires, utilisant l'anticorps selon l'une quelconque des revendications 1 à 12, 17 à 22 et 27 à 42.

52. Procédé *in vitro* de détection d'une pathologie sélectionnée dans le groupe constitué de la prolifération ou de la métastase de tumeurs solides, de la polyarthrite rhumatoïde, de la rétinopathie diabétique, de la rétinopathie de la prématurité et du psoriasis, utilisant l'anticorps selon l'une quelconque des revendications 1 à 12, 17 à 22 et 27 à 42.

53. Anticorps selon l'une quelconque des revendications 1 à 12, 17 à 22 et 27 à 42 pour utilisation dans un traitement ou un diagnostic.

54. Composition pharmaceutique comprenant au moins un anticorps selon l'une quelconque des revendications 1 à 12, 17 à 22 et 27 à 42.

55. Utilisation de l'anticorps selon l'une quelconque des revendications 1 à 12, 17 à 22 et 27 à 42 dans la préparation d'un agent destiné à détecter les régions d'angiogenèse ou à inhiber l'angiogenèse.

56. Anticorps selon l'une quelconque des revendications 1 à 12, 17 à 22 et 27 à 42 pour utilisation dans la détection de régions d'angiogenèse ou l'inhibition de l'angiogenèse.

57. Utilisation de l'anticorps selon l'une quelconque des revendications 1 à 12, 17 à 22 et 27 à 42 pour la préparation d'un agent destiné au diagnostic d'une pathologie sélectionnée dans le groupe constitué de la prolifération ou de la métastase de tumeurs solides, de la polyarthrite rhumatoïde, de la rétinopathie diabétique, de la rétinopathie de la prématurité et du psoriasis.

58. Anticorps selon l'une quelconque des revendications 1 à 12, 17 à 22 et 27 à 42 pour utilisation dans le diagnostic d'une pathologie sélectionnée dans le groupe constitué de la prolifération ou de la métastase de tumeurs solides, de la polyarthrite rhumatoïde, de la rétinopathie diabétique, de la rétinopathie de la prématurité et du psoriasis.

59. Utilisation de l'anticorps selon l'une quelconque des revendications 1 à 12, 17 à 22 et 27 à 42 pour la préparation d'un agent destiné à traiter une pathologie sélectionnée dans le groupe constitué de la prolifération ou de la métastase de tumeurs solides, de la polyarthrite rhumatoïde, de la rétinopathie diabétique, de la rétinopathie de la prématurité et du psoriasis.

60. Anticorps selon l'une quelconque des revendications 1 à 12, 17 à 22 et 27 à 42 pour utilisation dans le traitement d'une pathologie sélectionnée dans le groupe constitué de la prolifération ou de la métastase de tumeurs solides, de la polyarthrite rhumatoïde, de la rétinopathie diabétique, de la rétinopathie de la prématurité et du psoriasis.


### SEQUENCE LISTING

<110> KYOWA HAKKO KOGYO CO., LTD
<120> Recombinant antibody
<130> 11141WO
<140>
   <141>
<150> H10-139000
   <151> 1998-05-20
<160> 96
<170> PatentIn Ver. 2.0
<210> 1
   <211> 415
   <212> DNA
   <213> Mus musculus
<220>
   <223>
<220>
   <221> CDS
   <222> (1)..(414)
<400> 1
   atg gaa tgg aac tgg gtc gtt ctc ttc ctc ctg tca tta act gca ggt 48
   Met Glu Trp Asn Trp Val Val Leu Phe Leu Leu Ser Leu Thr Ala Gly
   -19             -15                 -10                 -5
   gtc tat gcc cag ggt cag atg cag cag tct gga gct gag ctg gtg aag 96
   Val Tyr Ala Gln Gly Gln Met Gln Gln Ser Gly Ala Glu Leu Val Lys
               -1   1                   5                       10
   cct ggg gct tca gtg aag ctg tcc tgc aag cct tct ggc ttc acc ttc 144
   Pro Gly Ala Ser Val Lys Leu Ser Cys Lys Pro Ser Gly Phe Thr Phe
            15                  20                      25
   agt agt aac tat ata agt tgg ttg aag cag aag cct gga cag agt ctt 192
   Ser Ser Asn Tyr Ile Ser Trp Leu Lys Gln Lys Pro Gly Gln Ser Leu
        30                  35                      40                  45
   gag tgg att gct tgg att tat gct gga act ggt gat gcc agc tat aat 240
   Glu Trp Ile Ala Trp Ile Tyr Ala Gly Thr Gly Asp Ala Ser Tyr Asn
                          50                      55                      60
   cag aag ttc aca gcc aag gcc cac gtg act gta gac aca tcc tcc agc 288
   Gln Lys Phe Thr Ala Lys Ala His Val Thr Val Asp Thr Ser Ser Ser
                    65                      70                      75
   aca gcc tac atg cag ttg agt agc ctg aca act gag gac tct gcc atc 336
   Thr Ala Tyr Met Gln Leu Ser Ser Leu Thr Thr Glu Asp Ser Ala Ile
                80                      85                      90
   tat tac tgt gca cga cac ggg ggg gac ggc tac tgg ttt gct tac tgg 384
   Tyr Tyr Cys Ala Arg His Gly Gly Asp Gly Tyr Trp Phe Ala Tyr Trp
            95                     100                     105
   ggc caa ggg act ctg gtc act gtc tct gca g                       415
   Gly Gln Gly Thr Leu Val Thr Val Ser Ala
   110                     115                 119
<210> 2
   <211> 385
   <212> DNA
   <213> Mus musculus
<220>
   <223>
<220>
   <221> CDS
   <222> (1)..(384)
<400> 2
   atg gat ttt caa gtg cag att ttc agc ttc ctg cta atc agt gcc tca 48
   Met Asp Phe Gln Val Gln Ile Phe Ser Phe Leu Leu Ile Ser Ala Ser
   -22 -20 -15 -10
   gtc ata ata tcc aga gga caa ctt gtt ctc acc cag tct cca gca atc 96
   Val Ile Ile Ser Arg Gly Gln Leu Val Leu Thr Gln Ser Pro Ala Ile
   -5 -1 1 5 10
   atg tct gca tct caa ggg gag aag gtc acc atg acc tgc agt gcc agc 144
   Met Ser Ala Ser Gln Gly Glu Lys Val Thr Met Thr Cys Ser Ala Ser
   15 20 25
   tca agt gtc agt tac atg cac tgg tac cag cag aag tca ggc acc tcc 192
   Ser Ser Val Ser Tyr Met His Trp Tyr Gln Gln Lys Ser Gly Thr Ser
   30 35 40
   ccc aaa aga tgg att tat gac aca tcc aaa ctg cct tct ggt gtc cct 240
   Pro Lys Arg Trp Ile Tyr Asp Thr Ser Lys Leu Pro Ser Gly Val Pro
   45 50 55
   gct cgc ttc agt ggc agt ggg tct ggg acc tct tac tct ctc aca atc 288
   Ala Arg Phe Ser Gly Ser Gly Ser Gly Thr Ser Tyr Ser Leu Thr Ile
   60 65 70
   agc agc atg gag gct gaa gat gct gcc act tat tat tgc cag cag tgg 336
   Ser Ser Met Glu Ala Glu Asp Ala Ala Thr Tyr Tyr Cys Gln Gln Trp
   75 80 85 90
   agt agt aac cca ccc acg ttc ggt gct ggg acc aag ctg gaa ctg aaa 384
   Ser Ser Asn Pro Pro Thr Phe Gly Ala Gly Thr Lys Leu Glu Leu Lys
   95 100 105 106
   c 385
<210> 3
   <211> 409
   <212> DNA
   <213> Mus musculus
<220>
   <223>
<220>
   <221> CDS
   <222> (1)..(408)
<400> 3
   atg gga ttc agc agg atc ttt ctc ttc ctc ctg tca gtg act aca ggt 48
   Met Gly Phe Ser Arg Ile Phe Leu Phe Leu Leu Ser Val Thr Thr Gly
   -19 -15 -10 -5
   gtc cac tcc cag gct ttt cta cag cag tct ggg gct gag ctg gtg agg 96
   Val His Ser Gln Ala Phe Leu Gln Gln Ser Gly Ala Glu Leu Val Arg
   -1 1 5 10
   cct ggg gcc tca gtg aag atg tcc tgc aag gct tct ggc tac aca ttt 144
   Pro Gly Ala Ser Val Lys Met Ser Cys Lys Ala Ser Gly Tyr Thr Phe
   15 20 25
   atc aat tac aat atg cac tgg gta aag cag aca cct aga cag ggc ctg 192
   Ile Asn Tyr Asn Met His Trp Val Lys Gln Thr Pro Arg Gln Gly Leu
   30 35 40 45
   gaa tgg att gga gct att ttt cca gga aat ggt ttt act tcc tac aat 240
   Glu Trp Ile Gly Ala Ile Phe Pro Gly Asn Gly Phe Thr Ser Tyr Asn
   50 55 60
   cag aag ttc aag ggc aag gcc aca ctg act gta gac aaa tcc tcc agc 288
   Gln Lys Phe Lys Gly Lys Ala Thr Leu Thr Val Asp Lys Ser Ser Ser
   65 70 75
   aca gtc tac atg cag ctc cgc agc ctg aca tct gaa gac tct gcg gtc 336
   Thr Val Tyr Met Gln Leu Arg Ser Leu Thr Ser Glu Asp Ser Ala Val
   80 85 90
   tat ttc tgt gca aga gat ggt gac tat tac ttt gac tac tgg ggc caa 384
   Tyr Phe Cys Ala Arg Asp Gly Asp Tyr Tyr Phe Asp Tyr Trp Gly Gln
   95 100 105
   ggc acc act ctc aca gtc tcc tca g 409
   Gly Thr Thr Leu Thr Val Ser Ser
   110 115 117
<210> 4
   <211> 379
   <212> DNA
   <213> Mus musculus
<220>
   <223>
<220>
   <221> CDS
   <222> (1)..(378)
<400> 4
   atg gat ttt cag gtg cag att ttc agc ttc ctg cta atc agt gcc tca 48
   Met Asp Phe Gln Val Gln Ile Phe Ser Phe Leu Leu Ile Ser Ala Ser
   -22 -20 -15 -10
   gtc ata atg tcc aga gga caa att gtt ctc acc cag tcg cca gca atc 96
   Val Ile Met Ser Arg Gly Gln Ile Val Leu Thr Gln Ser Pro Ala Ile
   -5 -1 1 5 10
   atg tct gca tct cta ggg gag gag atc acc cta acc tgc agt gcc agc 144
   Met Ser Ala Ser Leu Gly Glu Glu Ile Thr Leu Thr Cys Ser Ala Ser
   15 20 25
   tcg agt gta agt tac atg cac tgg tac cag cag aag tca ggc act tct 192
   Ser Ser Val Ser Tyr Met His Trp Tyr Gln Gln Lys Ser Gly Thr Ser
   30 35 40
   ccc aaa ctc ttg att tat aga aca tcc aac ctg gct tct gga gtc cct 240
   Pro Lys Leu Leu Ile Tyr Arg Thr Ser Asn Leu Ala Ser Gly Val Pro
   45 50 55
   ttt cgc ttc agt ggc agt ggg tct ggg acc ttt tat tct ctc aca atc 288
   Phe Arg Phe Ser Gly Ser Gly Ser Gly Thr Phe Tyr Ser Leu Thr Ile
   60 65 70
   agc agt gtg gag gct gaa gat gct gcc gat tat tac tgc cat cag tgg 336
   Ser Ser Val Glu Ala Glu Asp Ala Ala Asp Tyr Tyr Cys His Gln Trp
   75 80 85 90
   agt atg tac acg ttc gga ggg ggg acc aag ctg gaa ata aaa c 379
   Ser Met Tyr Thr Phe Gly Gly Gly Thr Lys Leu Glu Ile Lys
   95 100 104
<210> 5
   <Z11> 5
   <212> PRT
   <213> Mus musculus
<220>
   <223>
<400> 5
<210> 6
   <211> 17
   <212> PRT
   < 213 > Mus musculus
<220>
   <2Z3>
<400> 6
   Trp Ile Tyr Ala Gly Thr Gly Asp Ala Ser Tyr Asn Gln Lys Phe Thr
   1 5 10 15
   Ala
   17
<210> 7
   <211> 10
   <212> PRT
   <213> Mus musculus
<220>
   <223>
<400> 7
   His Gly Gly Asp Gly Tyr Trp Phe Ala Tyr
   1 5 10
<210> 8
   <211> 10
   <212> PRT
   <213> Mus musculus
<220>
   <223>
<400> 8
   Ser Ala Ser Ser Ser Val Ser Tyr Met His
   1 5 10
<210> 9
   <211> 7
   <212> PRT
   <213> Mus musculus
<220>
   <223>
<400> 9
   Asp Thr Ser Lys Leu Pro Ser
   1 5 7
<210> 10
   <211> 9
   <212> PRT
   <213> Mus musculus
<220>
   <223>
<400> 10
   Gln Gln Trp Ser Ser Asn Pro Pro Thr
   1 5 9
<210> 11
   <211> 5
   <212> PRT
   <213> Mus musculus
<220>
   <223>
<400> 11
   Asn Tyr Asn Met His
   1 5
<210> 12
   <211> 17
   <212> PRT
   <213> Mus musculus
<220>
   <223>
<400> 12
   Ala Ile Phe Pro Gly Asn Gly Phe Thr Ser Tyr Asn Gln Lys Phe Lys
   1 5 10 15
   Gly
   17
<210> 13
   <211> 8
   <212> PRT
   <213> Mus musculus
<220>
   <223>
<400> 13
   Asp Gly Asp Tyr Tyr Phe Asp Tyr
   1 5 8
<210> 14
   <211> 10
   <212> PRT
   <213> Mus musculus
<220>
   <223>
<400> 14
   Ser Ala Ser Ser Ser Val Ser Tyr Met His
   1 5 10
<210> 15
   <211> 7
   <212> PRT
   <213> Mus musculus
<220>
   <223>
<400> 15
   Arg Thr Ser Asn Leu Ala Ser
   1 5 7
<210> 16
   <211> 7
   <212> PRT
   <213> Mus musculus
<220>
   <223>
<400> 16
   His Gln Trp Ser Met Tyr Thr
   1 5 7
<210> 17
   <211> 45
   <212> DNA
   <213> Artificial Sequence
<220>
   <223> Description of Artificial Sequence: Synthetic DNA
<400> 17
   gccaagggac tctggtcact gtctctgcag cctccaccaa gggcc 45
<210> 18
   <211> 41
   <212> DNA
   <213> Artificial Sequence
<220>
   <223> Description of Artificial Sequence: Synthetic DNA
<400> 18
   cttggtggag gctgcagaga cagtgaccag agtcccttgg c 41
<210> 19
   <211> 69
   <212> DNA
   <213> Artificial Sequence
<220>
   <223> Description of Artificial Sequence: Synthetic DNA
<400> 19
   cttattattg ccagcagtgg agtagtaacc cacccacgtt cggtgctggg accaagctgg 60
   aactgaaac 69
<210> 20
   <211> 74
   <212> DNA
   <213> Artificial Sequence
<220>
   <223> Description of Artificial Sequence: Synthetic DNA
<400> 20
   gtacgtttca gttccagctt ggtcccagca ccgaacgtgg gtgggttact actccactgc 60
   tggcaataat aagt 74
<210> 21
   <211> 19
   <212> DNA
   <213> Artificial Sequence
<220>
   <223> Description of Artificial Sequence: Synthetic DNA
<400> 21
   tcagcctcca ccaagggcc 19
<210> 22
   <211> 11
   <212> DNA
   <213> Artificial Sequence
<220>
   <223> Description of Artificial Sequence: Synthetic DNA
<400> 22
   cttggtggag g 11
<210> 22
   <211> 33
   <212> DNA
   <213> Artificial Sequence
<220>
   <223> Description of Artificial Sequence: Synthetic DNA
<400> 23
   cgttcggagg ggggaccaag ctggaaataa aac 33
<210> 24
   <211> 35
   <212> DNA
   <213> Artificial Sequence
<220>
   <223> Description of Artificial Sequence: Synthetic DNA
<400> 24
   gtacgtttta tttccagctt ggtcccccct ccgaa 35
<210> 25
   <211> 94
   <212> DNA
   <213> Artificial Sequence
<220>
   <223> Description of Artificial Sequence: Synthetic DNA
<400> 25
   caggaaacag ctatgacgcg gccgccacca tggaatggaa ctgggtcgtt ctcttcctcc 60
   tgtcattaac tgcaggtgtc tatgcccagg tgca 94
<210> 26
   <211> 95
   <212> DNA
   <213> Artificial Sequence
<220>
   <223> Description of Artificial Sequence: Synthetic DNA
<400> 26
   gtgtatccag aagccttgca ggagaccttc actgaggcgc caggcttctt cacctcagcc 60
   ccagactgca ccagctgcac ctgggcatag acacc 95
<210> 27
   <211> 94
   <212> DNA
   <213> Artificial Sequence
<220>
   <223> Description of Artificial Sequence: Synthetic DNA
<400> 27
   tgcaaggctt ctggatacac cttcagcagt aactatataa gttgggtgcg acaggcccct 60
   ggacaagggc ttgagtggat gggatggatt tatg 94
<210> 28
   <211> 101
   <212> DNA
   <213> Artificial Sequence
<220>
   <223> Description of Artificial Sequence: Synthetic DNA
<400> 28
   aggctgtgct cgtggatgtg tcgacggtaa tggtgactct ggctgtgaac ttctgattat 60
   agctggcatc accagttcca gcataaatcc atcccatcca c 101
<210> 29
   <211> 100
   <212> DNA
   <213> Artificial Sequence
<220>
   <223> Description of Artificial Sequence: Synthetic DNA
<400> 29
   cacatccacg agcacagcct acatggagct gagcagcctg agatctgagg acacggccgt 60
   gtattactgt gcgagacacg ggggggacgg ctactggttt 100
<210> 30
   <211> 93
   <212> DNA
   <213> Artificial Sequence
<220>
   <223> Description of Artificial Sequence: Synthetic DNA
<400> 30
   gttttcccag tcacgacggg cccttggtgg aggctgagga gacggtgacc agggttccct 60
   ggccccagta agcaaaccag tagccgtccc ccc 93
<210> 31
   <211> 421
   <212> DNA
   <213> Artificial Sequence
<220>
   <223> Description of Artificial Sequence:Synthetic DNA
<220>
   <221> CDS
   <222> (1)..(420)
<400> 31
<210> 32
   <211> 96
   <212> DNA
   <213> Artificial Sequence
<220>
   <223> Description of Artificial Sequence: Synthetic DNA
<400> 32
   caggaaacag ctatgacgcg gccgccacca tgggattcag caggatcttt ctcttcctcc 60
   tgtcagtgac tacaggtgtc cactcccagg tgcagc 96
<210> 33
   <211> 94
   <212> DNA
   <213> Artificial Sequence
<220>
   <223> Description of Artificial Sequence: Synthetic DNA
<400> 33
   ggtgtatcca gaagccttgc aggagacctt cactgaggcc ccaggcttct tcacctcagc 60
   tccggactgc accagctgca cctgggagtg gaca 94
<210> 34
   <211> 90
   <212> DNA
   <213> Artificial Sequence
<220>
   <223> Description of Artificial Sequence: Synthetic DNA
<400> 34
   gcaaggcttc tggatacacc ttcattaatt acaatatgca ctgggtgcga caggcccctg 60
   gacaagggct tgagtggatg ggagctattt 90
<210> 35
   <211> 90
   <212> DNA
   <213> Artificial Sequence
<220>
   <223> Description of Artificial Sequence: Synthetic DNA
<400> 35
   tgtgctcgtg gacttgtcga cggtaatggt gactctgccc ttgaacttct gattgtagga 60
   agtaaaacca tttcctggaa aaatagctcc 90
<210> 36
   <211> 95
   <212> DNA
   <213> Artificial Sequence
<220>
   <223> Description of Artificial Sequence: Synthetic DNA
<400> 36
   tcgacaagtc cacgagcaca gcctacatgg agctgagcag cctgagatct gaggacacgg 60
   ccgtgtatta ctgtgcgaga gatggtgact attac 95
<210> 37
   <211> 96
   <212> DNA
   <213> Artificial Sequence
<220>
   <223> Description of Artificial Sequence: Synthetic DNA
<400> 37
   gttttcccag tcacgacggg cccttggtgg aggctgagga gacggtgacc agggttccct 60
   ggccccagta gtcaaagtaa tagtcaccat ctctcg 96
<210> 38
   <211> 409
   <212> DNA
   <213> Artificial Sequence
<220>
   <223> Description of Artificial Sequence:Synthetic DNA
<220>
   <221> CDS
   <222> (1)..(408)
<400> 38
   atg gga ttc agc agg atc ttt ctc ttc ctc ctg tca gtg act aca ggt 48
   Met Gly Phe Ser Arg Ile Phe Leu Phe Leu Leu Ser Val Thr Thr Gly
   -19             -15                 -10                  -5
   gtc cac tcc cag gtg cag ctg gtg cag tcc gga gct gag gtg aag aag 96
   Val His Ser Gln Val Gln Leu Val Gln Ser Gly Ala Glu Val Lys Lys
           -1   1               5                  10
   cct ggg gcc tca gtg aag gtc tcc tgc aag gct tct gga tac acc ttc 144
   Pro Gly Ala Ser Val Lys Val Ser Cys Lys Ala Ser Gly Tyr Thr Phe
        15                 20                  25
   att aat tac aat atg cac tgg gtg cga cag gcc cct gga caa ggg ctt 192
   Ile Asn Tyr Asn Met His Trp Val Arg Gln Ala Pro Gly Gln Gly Leu
    30                 35                  40                  45
   gag tgg atg gga gct att ttt cca gga aat ggt ttt act tcc tac aat 240
   Glu Trp Met Gly Ala Ile Phe Pro Gly Asn Gly Phe Thr Ser Tyr Asn
                   50                  55                  60
   cag aag ttc aag ggc aga gtc acc att acc gtc gac aag tcc acg agc 288
   Gln Lys Phe Lys Gly Arg Val Thr Ile Thr Val Asp Lys Ser Thr Ser
               65                  70                  75
   aca gcc tac atg gag ctg agc agc ctg aga tct gag gac acg gcc gtg 336
   Thr Ala Tyr Met Glu Leu Ser Ser Leu Arg Ser Glu Asp Thr Ala Val
           80                  85                  90
   tat tac tgt gcg aga gat ggt gac tat tac ttt gac tac tgg ggc cag 384
   Tyr Tyr Cys Ala Arg Asp Gly Asp Tyr Tyr Phe Asp Tyr Trp Gly Gln
        95                 100                 105
   gga acc ctg gtc acc gtc tcc tca g                               409
   Gly Thr Leu Val Thr Val Ser Ser
   110                 115     117
<210> 39
   <211> 87
   <212> DNA
   <213> Artificial Sequence
<220>
   <223> Description of Artificial Sequence: Synthetic DNA
<400> 39
   caggaaacag ctatgacgaa ttccaccatg gattttcaag tgcagatttt cagcttcctg 60
   ctaatcagtg cctcagtcat aatatcc                                     87
<210> 40
   <211> 93
   <212> DNA
   <213> Artificial Sequence
<220>
   <223> Description of Artificial Sequence: Synthetic DNA
<400> 40
   aagtgatggt gactctgtct cctacagatg cagacaggga ggatggagac tgggtcatct 60
   ggatatctcc tctggatatt atgactgagg cac 93
<210> 41
   <211> 85
   <212> DNA
   <213> Artificial Sequence
<220>
   <223> Description of Artificial Sequence: Synthetic DNA
<400> 41
   agacagagtc accatcactt gtagtgctag ctcaagtgtc agttacatgc actggtatca 60
   gcagaaacca gggaaagccc ctaag 85
<210> 42
   <211> 84
   <212> DNA
   <213> Artificial Sequence
<220>
   <223> Description of Artificial Sequence: Synthetic DNA
<400> 42
   atccactgcc gctgaacctt gatgggaccc cagaaggcag tttggatgtg tcatagatca 60
   gaagcttagg ggctttccct ggtt 84
<210> 43
   <211> 94
   <212> DNA
   <213> Artificial Sequence
<220>
   <223> Description of Artificial Sequence: Synthetic DNA
<400> 43
   aaggttcagc ggcagtggat ctgggacaga tttcactctc accatcagca gcctgcagcc 60
   tgaagatttt gcaacttatt actgtcagca gtgg 94
<210> 44
   <211> 91
   <212> DNA
   <213> Artificial Sequence
<220>
   <223> Description of Artificial Sequence: Synthetic DNA
<400> 44
   gttttcccag tcacgaccgt acgtttgatc tctaccttgg tcccttggcc gaacgtgggt 60
   gggttactac tccactgctg acagtaataa g 91
<210> 45
   <211> 385
   <212> DNA
   <213> Artificial Sequence
<220>
   <223> Description of Artificial Sequence:Synthetic DNA
<220>
   <221> CDS
   <222> (1)..(384)
<400> 45
   atg gat ttt caa gtg cag att ttc agc ttc ctg cta atc agt gcc tca 48
   Met Asp Phe Gln Val Gln Ile Phe Ser Phe Leu Leu Ile Ser Ala Ser
   -22 -20 -15 -10
   gtc ata ata tcc aga gga gat atc cag atg acc cag tct cca tcc tcc 96
   Val Ile Ile Ser Arg Gly Asp Ile Gln Met Thr Gln Ser Pro Ser Ser
        -5                 -1   1                5                10
   ctg tct gca tct gta gga gac aga gtc acc atc act tgt agt gct agc 144
   Leu Ser Ala Ser Val Gly Asp Arg Val Thr Ile Thr Cys Ser Ala Ser
                       15                   20                  25
   tca agt gtc agt tac atg cac tgg tat cag cag aaa cca ggg aaa gcc 192
   Ser Ser Val Ser Tyr Met His Trp Tyr Gln Gln Lys Pro Gly Lys Ala
               30                   35                  40
   cct aag ctt ctg atc tat gac aca tcc aaa ctg cct tct ggg gtc cca 240
   Pro Lys Leu Leu Ile Tyr Asp Thr Ser Lys Leu Pro Ser Gly Val Pro
           45                   50                  55
   tca agg ttc agc ggc agt gga tct ggg aca gat ttc act ctc acc atc 288
   Ser Arg Phe Ser Gly Ser Gly Ser Gly Thr Asp Phe Thr Leu Thr Ile
       60                   65                  70
   agc agc ctg cag cct gaa gat ttt gca act tat tac tgt cag cag tgg 336
   Ser Ser Leu Gln Pro Glu Asp Phe Ala Thr Tyr Tyr Cys Gln Gln Trp
   75                   80                  85                  90
   agt agt aac cca ccc acg ttc ggc caa ggg acc aag gta gag atc aaa 384
   Ser Ser Asn Pro Pro Thr Phe Gly Gln Gly Thr Lys Val Glu Ile Lys
                       95                  100                 105 106
   c                                                                 385
<210> 46
   <211> 84
   <212> DNA
   <213> Artificial Sequence
<220>
   <223> Description of Artificial Sequence: Synthetic DNA
<400> 46
   caggaaacag ctatgacgaa ttccaccatg gattttcagg tgcagatttt cagcttcctg   60
   ctaatcagtg cctcagtcat aatg                                           84
<210> 47
   <211> 96
   <212> DNA
   <213> Artificial Sequence
<220>
   <223> Description of Artificial Sequence: Synthetic DNA
<400> 47
   agttgatggt ggccctctcg cccagagaca cagccaggga gtctggagac tgggtcatca 60
   cgatgtctcc tctggacatt atgactgagg cactga 96
<210> 48
   <211> 85
   <212> DNA
   <213> Artificial Sequence
<220>
   <223> Description of Artificial Sequence: Synthetic DNA
<400> 48
   cgagagggcc accatcaact gcagtgccag ctcgagtgta agttacatgc actggtacca 60
   gcagaaacca ggacagcctc ctaag 85
<210> 49
   <211> 87
   <212> DNA
   <213> Artificial Sequence
<220>
   <223> Description of Artificial Sequence: Synthetic DNA
<400> 49
   cagacccgct gccactgaat cggtcaggga ccccagaagc caggttggat gttctgtaaa 60
   tgagcagctt aggaggctgt cctggtt 87
<210> 50
   <211> 86
   <212> DNA
   <213> Artificial Sequence
<220>
   <223> Description of Artificial Sequence: Synthetic DNA
<400> 50
   attcagtggc agcgggtctg ggacagattt cactctcacc atcagcagcc tgcaggctga 60
   agacgtcgca gtttattact gtcatc 86
<210> 51
   <211> 90
   <212> DNA
   <213> Artificial Sequence
<220>
   <223> Description of Artificial Sequence: Synthetic DNA
<400> 51 <210> 52
   <211> 379
   <212> DNA
   <213> Artificial Sequence
<220>
   <223> Description of Artificial Sequence:Synthetic DNA
<220>
   <221> CDS
   <222> (1)..(378)
<400> 52
<210> 53
   <211> 23
   <212> DNA
   <213> Artificial Sequence
<220>
   <223> Description of Artificial Sequence: Synthetic DNA
<400> 53
   cgacaaacca atataatcta agc 23
<210> 54
   <211> 25
   <212> DNA
   <213> Artificial Sequence
<220>
   <223> Description of Artificial Sequence: Synthetic DNA
<400> 54
   ggccgcttag attatattgg tttgt 25
<210> 55
   <211> 21
   <212> DNA
   <213> Artificial Sequence
<220>
   <223> Description of Artificial Sequence: Synthetic DNA
<400> 55
   ggaatctaca tttgcatagc t 21
<210> 56
   <211> 33
   <212> DNA
   <213> Artificial Sequence
<220>
   <223> Description of Artificial Sequence: Synthetic DNA
<400> 56
   ttatgcggcc gcttatcctt gaacagtgag gta 33
<210> 57
   <211> 8
   <212> DNA
   <213> Artificial Sequence
<220>
   <223> Description of Artificial Sequence: Synthetic DNA
<400> 57
   ctctagag 8
<210> 58
   <211> 60
   <212> DNA
   <213> Artificial Sequence
<220>
   <223> Description of Artificial Sequence: Synthetic DNA
<400> 58
   cagtgttctt ggctgtgcaa aaagtggagg catttttcat aatagaaggt gcctacgtag 60
<210> 59
   <211> 67
   <212> DNA
   <213> Artificial Sequence
<220>
   <223> Description of Artificial Sequence: Synthetic DNA
<400> 59
   gatcctacgt aggcaccttc tattatgaaa aatgcctcca cttttgcaca gccaagaaca 60
   ctgcatg 67
<210> 60
   <211> 18
   <212> DNA
   <213> Artificial Sequence
<220>
   <223> Description of Artificial Sequence: Synthetic DNA
<400> 60
   GTATAATGAG CGGCCGCG 18
<210> 61
   <211> 22
   <212> DNA
   <213> Artificial Sequence
<220>
   <223> Description of Artificial Sequence: Synthetic DNA
<400> 61
   gatccgcggc cgctcattat ac 22
<210> 62
   <211> 56
   <212> DNA
   <213> Artificial Sequence
<220>
   <223> Description of Artificial Sequence: Synthetic DNA
<400> 62
   gaaggaaaca gaaggcgcca tctatatatt tattcgaggt accaatacaa tcatag 56
<210> 63
   <211> 30
   <212> DNA
   <213> Artificial Sequence
<220>
   <223> Description of Artificial Sequence: Synthetic DNA
<400> 63
   aaactgactt ggccggcgcc atttatgtct 30
<210> 64
   <211> 30
   <212> DNA
   <213> Artificial Sequence
<220>
   <223> Description of Artificial Sequence: Synthetic DNA
<400> 64
   cataaatcct ataggtacca acgacaacta 30
<210> 65
   <211> 87
   <212> DNA
   <213> Artificial Sequence
<220>
   <223> Description of Artificial Sequence: Synthetic DNA
<400> 65
   caggaaacag ctatgacgaa ttccaccatg gattttcaag tgcagatttt cagcttcctg 60
   ctaatcagtg cctcagtcat aatatcc 87
<210> 66
   <211> 93
   <212> DNA
   <213> Artificial Sequence
<220>
   <223> Description of Artificial Sequence: Synthetic DNA
<400> 66
   aagtgatggt gactctgtct cctacagatg cagacaggga ggatggagac tgggtcatct 60
   ggatatctcc tctggatatt atgactgagg cac 93
<210> 67
   <211> 85
   <212> DNA
   <213> Artificial Sequence
<220>
   <223> Description of Artificial Sequence: Synthetic DNA
<400> 67
   agacagagtc accatcactt gtagtgccag ctcgagtgta agttacatgc actggtatca 60
   gcagaaacca gggaaagccc ctaag 85
<210> 68
   <211> 84
   <212> DNA
   <213> Artificial Sequence
<220>
   <223> Description of Artificial Sequence: Synthetic DNA
<400> 68
   atccactgcc gctgaacctt gatgggaccc cagaagccag gttggatgtt ctatagatca 60
   gaagcttagg ggctttccct ggtt 84
<210> 69
   <211> 94
   <212> DNA
   <213> Artificial Sequence
<220>
   <223> Description of Artificial Sequence: Synthetic DNA
<400> 69
   aaggttcagc ggcagtggat ctgggacaga tttcactctc accatcagca gcctgcagcc 60
   tgaagatttt gcaacttatt actgtcatca gtgg 94
<210> 70
   <211> 85
   <212> DNA
   <213> Artificial Sequence
<220>
   <223> Description of Artificial Sequence: Synthetic DNA
<400> 70
   gttttcccag tcacgaccgt acgtttgatc tctaccttgg tcccttggcc gaacgtgtac 60
   atactccact gatgacagta ataag 85
<210> 71
   <211> 379
   <212> DNA
   <213> Artificial Sequence
<220>
   <223> Description of Artificial Sequence:Synthetic DNA
<220>
   <221> CDS
   <222> (1)..(378)
<400> 71
   atg gat ttt caa gtg cag att ttc agc ttc ctg cta atc agt gcc tca 48
   Met Asp Phe Gln Val Gln Ile Phe Ser Phe Leu Leu Ile Ser Ala Ser
   -22     -20                 -15                 -10
   gtc ata ata tcc aga gga gat atc cag atg acc cag tct cca tcc tcc 96
   Val Ile Ile Ser Arg Gly Asp Ile Gln Met Thr Gln Ser Pro Ser Ser
       -5                  -1   1               5                  10
   ctg tct gca tct gta gga gac aga gtc acc atc act tgt agt gcc agc 144
   Leu Ser Ala Ser Val Gly Asp Arg Val Thr Ile Thr Cys Ser Ala Ser
                       15                  20                  25
   tcg agt gta agt tac atg cac tgg tat cag cag aaa cca ggg aaa gcc 192
   Ser Ser Val Ser Tyr Met His Trp Tyr Gln Gln Lys Pro Gly Lys Ala
                   30                  35                  40
   cct aag ctt ctg atc tat aga aca tcc aac ctg gct tct ggg gtc cca 240
   Pro Lys Leu Leu Ile Tyr Arg Thr Ser Asn Leu Ala Ser Gly Val Pro
           45                  50                  55
   tca agg ttc agc ggc agt gga tct ggg aca gat ttc act ctc acc atc 288
   Ser Arg Phe Ser Gly Ser Gly Ser Gly Thr Asp Phe Thr Leu Thr Ile
       60                  35                  70
   agc agc ctg cag cct gaa gat ttt gca act tat tac tgt cat cag tgg 336
   Ser Ser Leu Gln Pro Glu Asp Phe Ala Thr Tyr Tyr Cys His Gln Trp
   75                  80                  85                  90
   agt atg tac acg ttc ggc caa ggg acc aag gta gag atc aaa c       379
   Ser Met Tyr Thr Phe Gly Gln Gly Thr Lys Val Glu Ile Lys
                       95                  100             104
<210> 72
   <211> 80
   <212> DNA
   <213> Artificial Sequence
<220>
   <223> Description of Artificial Sequence: Synthetic DNA
<400> 72
   caggaaacag ctatgactcc ggagctgagg tgaagaagcc tggggcctca gtgaaggtct 60
   cctgcaaggc ttctggatac                                              80
<210> 73
   <211> 80
   <212> DNA
   <213> Artificial Sequence
<220>
   <223> Description of Artificial Sequence: Synthetic DNA
<400> 73
   ccactcaagc ccttgtccag gggcctgtcg cacccagtgc atattgtaat taatgaaggt 60
   gtatccagaa gccttgcagg 80
<210> 74
   <211> 81
   <212> DNA
   <213> Artificial Sequence
<220>
   <223> Description of Artificial Sequence: Synthetic DNA
<400> 74
   ctggacaagg gcttgagtgg atgggagcta tttttccagg aaatggtttt acttcctaca 60
   atcagaagtt caagggcaga g 81
<210> 75
   <211> 79
   <212> DNA
   <213> Artificial Sequence
<220>
   <223> Description of Artificial Sequence: Synthetic DNA
<400> 75
   tctcaggctg cgcagctgca tgtaggctgt gctcgtggac ttgtcgacgg taatggtgac 60
   tctgcccttg aacttctga 79
<210> 76
   <211> 83
   <212> DNA
   <213> Artificial Sequence
<220>
   <223> Description of Artificial Sequence: Synthetic DNA
<400> 76
   tgcagctgcg cagcctgaga tctgaggaca cggccgtgta tttctgtgcg agagatggtg 60
   actattactt tgactactgg ggc 83
<210> 77
   <211> 81
   <212> DNA
   <213> Artificial Sequence
<220>
   <223> Description of Artificial Sequence: Synthetic DNA
<400> 77
   gttttcccag tcacgacggg cccttggtgg aggctgagga gacggtgacc agggttccct 60
   ggccccagta gtcaaagtaa t 81
<210> 78
   <211> 409
   <212> DNA
   <213> Artificial Sequence
<220>
   <223> Description of Artificial Sequence:Synthetic DNA
<220>
   <221> CDS
   <222> (1)..(408)
<400> 78
   atg gga ttc agc agg atc ttt ctc ttc ctc ctg tca gtg act aca ggt  48
   Met Gly Phe Ser Arg Ile Phe Leu Phe Leu Leu Ser Val Thr Thr Gly
   -19             -15                 -10                 -5
   gtc cac tcc cag gtg cag ctg gtg cag tcc gga gct gag gtg aag aag  96
   Val His Ser Gln Val Gln Leu Val Gln Ser Gly Ala Glu Val Lys Lys
           -1   1               5                  10
   cct ggg gcc tca gtg aag gtc tcc tgc aag gct tct gga tac acc ttc 144
   Pro Gly Ala Ser Val Lys Val Ser Cys Lys Ala Ser Gly Tyr Thr Phe
        15                  20                  25
   att aat tac aat atg cac tgg gtg cga cag gcc cct gga caa ggg ctt 192
   Ile Asn Tyr Asn Met His Trp Val Arg Gln Ala Pro Gly Gln Gly Leu
    30                  35                  40                  45
   gag tgg atg gga gct att ttt cca gga aat ggt ttt act tcc tac aat 240
   Glu Trp Met Gly Ala Ile Phe Pro Gly Asn Gly Phe Thr Ser Tyr Asn
   50 55 60
   cag aag ttc aag ggc aga gtc acc att acc gtc gac aag tcc acg agc 288
   Gln Lys Phe Lys Gly Arg Val Thr Ile Thr Val Asp Lys Ser Thr Ser
   65 70 75
   aca gcc tac atg cag ctg cgc agc ctg aga tct gag gac acg gcc gtg 336
   Thr Ala Tyr Met Gln Leu Arg Ser Leu Arg Ser Glu Asp Thr Ala Val
   80 85 90
   tat ttc tgt gcg aga gat ggt gac tat tac ttt gac tac tgg ggc cag 384
   Tyr Phe Cys Ala Arg Asp Gly Asp Tyr Tyr Phe Asp Tyr Trp Gly Gln
   95 100 105
   gga acc ctg gtc acc gtc tcc tca g 409
   Gly Thr Leu Val Thr Val Ser Ser
   110 115 117
<210> 79
   <211> 87
   <212> DNA
   <213> Artificial Sequence
<220>
   <223> Description of Artificial Sequence: Synthetic DNA
<400> 79
   caggaaacag ctatgacgaa ttccaccatg gattttcaag tgcagatttt cagcttcctg 60
   ctaatcagtg cctcagtcat aatatcc 87
<210> 80
   <211> 93
   <212> DNA
   <213> Artificial Sequence
<220>
   <223> Description of Artificial Sequence: Synthetic DNA
<400> 80
   aagtgatggt gacctcctct cctacagatg cagacaggga ggatggagac tgggtcatct 60
   ggatatctcc tctggatatt atgactgagg cac 93
<210> 81
   <211> 85
   <212> DNA
   <213> Artificial Sequence
<220>
   <223> Description of Artificial Sequence: Synthetic DNA
<400> 81
   agaggaggtc accatcactt gtagtgccag ctcgagtgta agttacatgc actggtatca 60
   gcagaaacca gggaaagccc ctaag 85
<210> 82
   <211> 84
   <212> DNA
   <213> Artificial Sequence
<220>
   <223> Description of Artificial Sequence: Synthetic DNA
<400> 82
   atccactgcc gctgaacctt gatgggaccc cagaagccag gttggatgtt ctatagatca 60
   gaagcttagg ggctttccct ggtt 84
<210> 83
   <211> 94
   <212> DNA
   <213> Artificial Sequence
<220>
   <223> Description of Artificial Sequence: Synthetic DNA
<400> 83
   aaggttcagc ggcagtggat ctgggacatt ttatactctc accatcagca gcctgcagcc 60
   tgaagatttt gcaacttatt actgtcatca gtgg 94
<210> 84
   <211> 85
   <212> DNA
   <213> Artificial Sequence
<220>
   <223> Description of Artificial Sequence: Synthetic DNA
<400> 84
   gttttcccag tcacgaccgt acgtttgatc tctaccttgg tcccttggcc gaacgtgtac 60
   atactccact gatgacagta ataag 85
<210> 85
   <211> 379
   <212> DNA
   <213> Artificial Sequence
<220>
   <223> Description of Artificial Sequence:Synthetic DNA
<220>
   <221> CDS
   <222> (1)..(378)
<400> 85
   atg gat ttt caa gtg cag att ttc agc ttc ctg cta atc agt gcc tca  48
   Met Asp Phe Gln Val Gln Ile Phe Ser Phe Leu Leu Ile Ser Ala Ser
   -22     -20                 -15                 -10
   gtc ata ata tcc aga gga gat atc cag atg acc cag tct cca tcc tcc  96
   Val Ile Ile Ser Arg Gly Asp Ile Gln Met Thr Gln Ser Pro Ser Ser
        -5                  -1   1               5                  10
   ctg tct gca tct gta gga gag gag gtc acc atc act tgt agt gcc agc 144
   Leu Ser Ala Ser Val Gly Glu Glu Val Thr Ile Thr Cys Ser Ala Ser
                        15                  20                  25
   tcg agt gta agt tac atg cac tgg tat cag cag aaa cca ggg aaa gcc 192
   Ser Ser Val Ser Tyr Met His Trp Tyr Gln Gln Lys Pro Gly Lys Ala
                   30                  35                  40
   cct aag ctt ctg atc tat aga aca tcc aac ctg gct tct ggg gtc cca 240
   Pro Lys Leu Leu Ile Tyr Arg Thr Ser Asn Leu Ala Ser Gly Val Pro
               45                  50                  55
   tca agg ttc agc ggc agt gga tct ggg aca ttt tat act ctc acc atc 288
   Ser Arg Phe Ser Gly Ser Gly Ser Gly Thr Phe Tyr Thr Leu Thr Ile
        60                  65                  70
   agc agc ctg cag cct gaa gat ttt gca act tat tac tgt cat cag tgg 336
   Ser Ser Leu Gln Pro Glu Asp Phe Ala Thr Tyr Tyr Cys His Gln Trp
    75                  80                  85                  90
   agt atg tac acg ttc ggc caa ggg acc aag gta gag atc aaa c       379
   Ser Met Tyr Thr Phe Gly Gln Gly Thr Lys Val Glu Ile Lys
                        95                 100
<210> 86
   <211> 38
   <212> PRT
   <213> Mus musculus
<220>
   <223>
<400> 86
<210> 87
   <211> 128
   <212> PRT
   <213> Mus musculus
<220>
   <223>
<400> 87
   Met Asp Phe Gln Val Gln Ile Phe Ser Phe Leu Leu Ile Ser Ala Ser
   -22 -20 -15 -10
   Val Ile Ile Ser Arg Gly Gln Leu Val Leu Thr Gln Ser Pro Ala Ile
   -5 -1 1 5 10
   Met Ser Ala Ser Gln Gly Glu Lys Val Thr Met Thr Cys Ser Ala Ser
   15 20 25
   Ser Ser Val Ser Tyr Met His Trp Tyr Gln Gln Lys Ser Gly Thr Ser
   30 35 40
   Pro Lys Arg Trp Ile Tyr Asp Thr Ser Lys Leu Pro Ser Gly Val Pro
   45 50 55
   Ala Arg Phe Ser Gly Ser Gly Ser Gly Thr Ser Tyr Ser Leu Thr Ile
   60 65 70
   Ser Ser Met Glu Ala Glu Asp Ala Ala Thr Tyr Tyr Cys Gln Gln Trp
   75 80 85 90
   Ser Ser Asn Pro Pro Thr Phe Gly Ala Gly Thr Lys Leu Glu Leu Lys
   95 100 105 106
<210> 88
   <211> 136
   <212> PRT
   <213> Mus musculus
<220>
   <223>
<400> 88
   Met Gly Phe Ser Arg Ile Phe Leu Phe Leu Leu Ser Val Thr Thr Gly
   -19 -15 -10 -5
   Val His Ser Gln Ala Phe Leu Gln Gln Ser Gly Ala Glu Leu Val Arg
   -1 1 5 10
   Pro Gly Ala Ser Val Lys Met Ser Cys Lys Ala Ser Gly Tyr Thr Phe
   15 20 25
   Ile Asn Tyr Asn Met His Trp Val Lys Gln Thr Pro Arg Gln Gly Leu
   30 35 40 45
   Glu Trp Ile Gly Ala Ile Phe Pro Gly Asn Gly Phe Thr Ser Tyr Asn
   50 55 60
   Gln Lys Phe Lys Gly Lys Ala Thr Leu Thr Val Asp Lys Ser Ser Ser
   65 70 75
   Thr Val Tyr Met Gln Leu Arg Ser Leu Thr Ser Glu Asp Ser Ala Val
   80 85 90
   Tyr Phe Cys Ala Arg Asp Gly Asp Tyr Tyr Phe Asp Tyr Trp Gly Gln
   95 100 105
   Gly Thr Thr Leu Thr Val Ser Ser
   110 115 117
<210> 89
   <211> 126
   <212> PRT
   <213> Mus musculus
<220>
   <223>
<400> 89
   Met Asp Phe Gln Val Gln Ile Phe Ser Phe Leu Leu Ile Ser Ala Ser
   -22 -20 -15 -10
   Val Ile Met Ser Arg Gly Gln Ile Val Leu Thr Gln Ser Pro Ala Ile
   -5 -1 1 5 10
   Met Ser Ala Ser Leu Gly Glu Glu Ile Thr Leu Thr Cys Ser Ala Ser
   15 20 25
   Ser Ser Val Ser Tyr Met His Trp Tyr Gln Gln Lys Ser Gly Thr Ser
   30 35 40
   Pro Lys Leu Leu Ile Tyr Arg Thr Ser Asn Leu Ala Ser Gly Val Pro
   45 50 55
   Phe Arg Phe Ser Gly Ser Gly Ser Gly Thr Phe Tyr Ser Leu Thr Ile
   60 65 70
   Ser Ser Val Glu Ala Glu Asp Ala Ala Asp Tyr Tyr Cys His Gln Trp
   75 80 85 90
   Ser Met Tyr Thr Phe Gly Gly Gly Thr Lys Leu Glu Ile Lys
   95 100 104
<210> 90
   <211> 140
   <212> PRT
   <213> Artificial Sequence
<220>
   <223> Description of Artificial Sequence: Humanized antibody
<400> 90
<210> 91
   <211> 136
   <212> PRT
   <213> Artificial Sequence
<220>
   <223> Description of Artificial Sequence: Humanized antibody
<400> 91
   Met Gly Phe Ser Arg Ile Phe Leu Phe Leu Leu Ser Val Thr Thr Gly
   -19 -15 -10 -5
   Val His Ser Gln Val Gln Leu Val Gln Ser Gly Ala Glu Val Lys Lys
   -1 1 5 10
   Pro Gly Ala Ser Val Lys Val Ser Cys Lys Ala Ser Gly Tyr Thr Phe
   15 20 25
   Ile Asn Tyr Asn Met His Trp Val Arg Gln Ala Pro Gly Gln Gly Leu
   30 35 40 45
   Glu Trp Met Gly Ala Ile Phe Pro Gly Asn Gly Phe Thr Ser Tyr Asn
   50 55 60
   Gln Lys Phe Lys Gly Arg Val Thr Ile Thr Val Asp Lys Ser Thr Ser
   65 70 75
   Thr Ala Tyr Met Glu Leu Ser Ser Leu Arg Ser Glu Asp Thr Ala Val
   80 85 90
   Tyr Tyr Cys Ala Arg Asp Gly Asp Tyr Tyr Phe Asp Tyr Trp Gly Gln
   95 100 105
   Gly Thr Leu Val Thr Val Ser Ser
   110 115 117
<210> 92
   <211> 128
   <212> PRT
   <213> Artificial Sequence
<220>
   <223> Description of Artificial Sequence: Humanized antibody
<400> 92
   Met Asp Phe Gln Val Gln Ile Phe Ser Phe Leu Leu Ile Ser Ala Ser
   -22 -20 -15 -10
   Val Ile Ile Ser Arg Gly Asp Ile Gln Met Thr Gln Ser Pro Ser Ser
   -5 -1 1 5 10
   Leu Ser Ala Ser Val Gly Asp Arg Val Thr Ile Thr Cys Ser Ala Ser
   15 20 25
   Ser Ser Val Ser Tyr Met His Trp Tyr Gln Gln Lys Pro Gly Lys Ala
   30 35 40
   Pro Lys Leu Leu Ile Tyr Asp Thr Ser Lys Leu Pro Ser Gly Val Pro
   45 50 55
   Ser Arg Phe Ser Gly Ser Gly Ser Gly Thr Asp Phe Thr Leu Thr Ile
   60 65 70
   Ser Ser Leu Gln Pro Glu Asp Phe Ala Thr Tyr Tyr Cys Gln Gln Trp
   75 80 85 90
   Ser Ser Asn Pro Pro Thr Phe Gly Gln Gly Thr Lys Val Glu Ile Lys
   95 100 105 106
<210> 93
   <211> 126
   <212> PRT
   <213> Artificial Sequence
<220>
   <223> Description of Artificial Sequence: Humanized antibody
<400> 93
   Met Asp Phe Gln Val Gln Ile Phe Ser Phe Leu Leu Ile Ser Ala Ser
   -22 -20 -15 -10
   Val Ile Met Ser Arg Gly Asp Ile Val Met Thr Gln Ser Pro Asp Ser
   -5 -1 1 5 10
   Leu Ala Val Ser Leu Gly Glu Arg Ala Thr Ile Asn Cys Ser Ala Ser
   15 20 25
   Ser Ser Val Ser Tyr Met His Trp Tyr Gln Gln Lys Pro Gly Gln Pro
   30 35 40
   Pro Lys Leu Leu Ile Tyr Arg Thr Ser Asn Leu Ala Ser Gly Val Pro
   45 50 55
   Asp Arg Phe Ser Gly Ser Gly Ser Gly Thr Asp Phe Thr Leu Thr Ile
   60 65 70
   Ser Ser Leu Gln Ala Glu Asp Val Ala Val Tyr Tyr Cys His Gln Trp
   75 80 85 90
   Ser Met Tyr Thr Phe Gly Gln Gly Thr Lys Val Glu Ile Lys
   95 100 104
<210> 94
   <211> 126
   <212> PRT
   <213> Artificial Sequence
<220>
   <223> Description of Artificial Sequence: Humanized antibody
<400> 94
   Met Asp Phe Gln Val Gln Ile Phe Ser Phe Leu Leu Ile Ser Ala Ser
   -22 -20 -15 -10
   Val Ile Ile Ser Arg Gly Asp Ile Gln Met Thr Gln Ser Pro Ser Ser
   -5 -1 1 5 10
   Leu Ser Ala Ser Val Gly Asp Arg Val Thr Ile Thr Cys Ser Ala Ser
   15 20 25
   Ser Ser Val Ser Tyr Met His Trp Tyr Gln Gln Lys Pro Gly Lys Ala
   30 35 40
   Pro Lys Leu Leu Ile Tyr Arg Thr Ser Asn Leu Ala Ser Gly Val Pro
   45 50 55
   Ser Arg Phe Ser Gly Ser Gly Ser Gly Thr Asp Phe Thr Leu Thr Ile
   60 65 70
   Ser Ser Leu Gln Pro Glu Asp Phe Ala Thr Tyr Tyr Cys His Gln Trp
   75 80 85 90
   Ser Met Tyr Thr Phe Gly Gln Gly Thr Lys Val Glu Ile Lys
   95 100 104
<210> 95
   <211> 136
   <212> PRT
   <213> Artificial Sequence
<220>
   <223> Description of Artificial Sequence: Humanized antibody
<400> 95
   Met Gly Phe Ser Arg Ile Phe Leu Phe Leu Leu Ser Val Thr Thr Gly
   -19 -15 -10 -5
   Val His Ser Gln Val Gln Leu Val Gln Ser Gly Ala Glu Val Lys Lys
   -1 1 5 10
   Pro Gly Ala Ser Val Lys Val Ser Cys Lys Ala Ser Gly Tyr Thr Phe
   15 20 25
   Ile Asn Tyr Asn Met His Trp Val Arg Gln Ala Pro Gly Gln Gly Leu
   30 35 40 45
   Glu Trp Met Gly Ala Ile Phe Pro Gly Asn Gly Phe Thr Ser Tyr Asn
   50 55 60
   Gln Lys Phe Lys Gly Arg Val Thr Ile Thr Val Asp Lys Ser Thr Ser
   65 70 75
   Thr Ala Tyr Met Gln Leu Arg Ser Leu Arg Ser Glu Asp Thr Ala Val
   80 85 90
   Tyr Phe Cys Ala Arg Asp Gly Asp Tyr Tyr Phe Asp Tyr Trp Gly Gln
   95 100 105
   Gly Thr Leu Val Thr Val Ser Ser
   110 115 117
<210> 96
   <211> 126
   <212> PRT
   <213> Artificial Sequence
<220>
   <223> Description of Artificial Sequence: Humanized antibody
<400> 96
   Met Asp Phe Gln Val Gln Ile Phe Ser Phe Leu Leu Ile Ser Ala Ser
   -22 -20 -15 -10
   Val Ile Ile Ser Arg Gly Asp Ile Gln Met Thr Gln Ser Pro Ser Ser
   -5 -1 1 5 10
   Leu Ser Ala Ser Val Gly Glu Glu Val Thr Ile Thr Cys Ser Ala Ser
   15 20 25
   Ser Ser Val Ser Tyr Met His Trp Tyr Gln Gln Lys Pro Gly Lys Ala
               30                  35                  40
   Pro Lys Leu Leu Ile Tyr Arg Thr Ser Asn Leu Ala Ser Gly Val Pro
           45                  50                  55
   Ser Arg Phe Ser Gly Ser Gly Ser Gly Thr Phe Tyr Thr Leu Thr Ile
       60                  65                  70
   Ser Ser Leu Gln Pro Glu Asp Phe Ala Thr Tyr Tyr Cys His Gln Trp
    75                  80                  85                  90
   Ser Met Tyr Thr Phe Gly Gln Gly Thr Lys Val Glu Ile Lys
                   95                 100             104